(19) Europäisches Patentamt
European Patent Office
Office européen des brevets

(11) Veröffentlichungsnummer: **0 347 766 B1**

(12) # EUROPÄISCHE PATENTSCHRIFT

(45) Veröffentlichungstag der Patentschrift: **25.05.94**

(51) Int. Cl.$^5$: **C07D 513/04**, A61K 31/55, //(C07D513/04,277:00,223:00)

(21) Anmeldenummer: **89110950.6**

(22) Anmeldetag: **16.06.89**

(54) **4,5,7,8-Tetrahydro-6H-thiazolo[5,4-d]azepine, ihre Herstellung und ihre Verwendung als Arzneimittel.**

(30) Priorität: **20.06.88 DE 3820775**

(43) Veröffentlichungstag der Anmeldung:
**27.12.89 Patentblatt 89/52**

(45) Bekanntmachung des Hinweises auf die Patenterteilung:
**25.05.94 Patentblatt 94/21**

(84) Benannte Vertragsstaaten:
**AT BE CH DE ES FR GB GR IT LI LU NL SE**

(56) Entgegenhaltungen:
**GB-A- 1 321 509**
**GB-A- 1 412 377**
**GB-A- 2 173 187**

(73) Patentinhaber: **Dr. Karl Thomae GmbH**

**D-88397 Biberach(DE)**

(72) Erfinder: **Grell, Wolfgang, Dr. Dipl.-Chem.**
**Geschwister-Scholl-Strasse 18**
**D-7950 Biberach 1(DE)**
Erfinder: **Hurnaus, Rudolf, Dr. Dipl.-Chem.**
**Silcherstrasse 19**
**D-7950 Biberach 1(DE)**
Erfinder: **Reiffen, Manfred, Dr. Dipl.-Chem.**
**Matthias-Erzberger-Strasse 40**
**D-7950 Biberach 1(DE)**
Erfinder: **Sauter, Robert, Dr. Dipl.-Chem.**
**Albert-Schweitzer-Weg 9**
**D-7958 Laupheim(DE)**
Erfinder: **Pichler, Ludwig, Dr.**
**Gusshausstrasse 24/11**
**A-1040 Wien(AT)**
Erfinder: **Kobinger, Walter, Dr. Prof.**
**Belghofergasse 27**
**A-1121 Wien(AT)**
Erfinder: **Entzeroth, Michael, Dr. Dipl-Chem.**
**Sebastian-Sailer-Strasse 20**
**D-7951 Warthausen 1(DE)**
Erfinder: **Schingnitz, Günter, Dr.**
**Unter den Gärten 18**
**D-6550 Bad Kreuznach 14(DE)**
Erfinder: **Mierau, Joachim, Dr.**
**An den Weiden 3**
**D-6500 Mainz 33(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

In der britischen Patentschrift 1.321.509 werden u.a. Verbindungen der allgemeinen Formel I

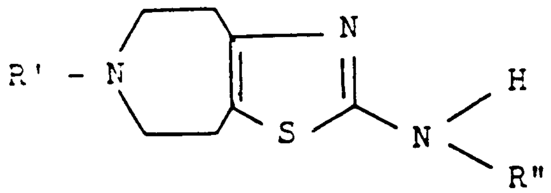

,(I)

in der
R′ ein Wasserstoffatom, einen gegebenenfalls durch eine Hydroxylgruppe substituierten geradkettigen oder verzweigten Alkylrest mit 1 bis 4 Kohlenstoffatomen, einen Allyl-, Cycloalkyl-, Hexahydrobenzyl-, Phenyl-, Phenyläthyl- oder Benzyl-rest, wobei der Benzylrest im Kern durch ein oder zwei Halogenatome, durch ein bis drei Methoxygruppen, durch eine Trifluormethyl- oder Alkylgruppe mit 1 bis 3 Kohlenstoffatomen substituiert sein kann, und
R″ ein Wasserstoffatom, einen geradkettigen oder verzweigten Alkylrest mit 1 bis 5 Kohlenstoffatomen, einen Allyl-, Cycloalkyl-, Phenyl-, Benzyl- oder Phenyläthylrest bedeuten, beschrieben. Diese Verbindungen weisen wertvolle pharmakologische Eigenschaften auf, insbesondere eine blutdrucksenkende, sedierende, hustenstillende und/oder antiphlogistische Wirksamkeit.

Von den in der britischen Patentschrift 1.321.509 beschriebenen Verbindungen wurden die Verbindungen 2-Amino-6-allyl-4,5,7,8-tetrahydro-6H-thiazolo[5,4-d]azepin (Verbindung A) und 2-Amino-6-(4-chlor-benzyl)-4,5,7,8-tetrahydro-6H-thiazolo[5,4-d]azepin (Verbindung B) in der Folgezeit näher untersucht.

So wurde für die Verbindung A (= B-HT 920) ihre Affinität zu α2-Adrenozeptoren [R. Hammer, W. Kobinger und L. Pichler, Europ. J. Pharmacol. 62, 277 (1980)]; ihre Verwendung zur Bekämpfung der Angina pectoris [DE-A-2.820.808, L. Benedikter et al.], ihre agonistische Wirkung auf Dopamin-Autorezeptoren [N.-E. Andén et al., Naunyn-Schmiedeberg's Arch. Pharmacol. 321, 100 (1982) sowie J. Neural Transmission 57, 129 (1983)]; ihre in vivo-Hemmung der endogenen Dopamin-Synthese im Gehirn [N.-E. Andén et al., Acta pharmacol. et toxicol. 52, 51 (1983)]; ihre Hemmung der Prolaktinspiegel im Blut [V. Brantl et al., EP-A-0.195.888]; ihre Wirkung als post-synaptischer Dopamin-Agonist am denervierten Dopamin-Rezeptor im Striatum und ihre Verwendung als Mittel zur Behandlung des Parkinsonismus [O. Hornykiewicz, DE-A-3.503.963; sowie D. Hinzen, O. Hornykiewicz, et al., Europ. J. Pharmacol. 131, 75 (1986)] und ihre Affinität zu Dopamin-D2-Rezeptoren [G.Griss, R. Hurnaus et al.; EFMC-IXth International Symposium on Medicinal Chemistry, Berlin (West), September 14-18, 1986, Short Communication Nr. 64, Abstract Seite 114] sowie
für die Verbindung B (= B-HT 958) ihre α2-agonistische Aktivität mit einem hohen prae/postsynaptischen Aktivitäts-Verhältnis [L. Lichler, H. Hörtnagl und W. Kobinger, Naunyn-Schmiedeberg's Arch. Pharmacol. 320, 110 (1982)]; ihre Verwendung zur Bekämpfung der Angina pectoris [DE-A-2.820.808, L. Benedikter et al.]; ihre kardiovasculären Effekte [W. Kobinger und und L. Pichler, Europ. J. Pharmacol. 97, 67 (1984)]; ihre zentrale α2-antagonistische Wirkung und ihr agonistischer Effekt auf Dopamin-Autorezeptoren im Gehirn [H. Hörtnagl, L. Pichler, U. Holzer-Petsche, O. Hornykiewicz und W. Kobinger, Europ. J. Pharmacol. 106, 335 (1985); ihre blutdruck- und herzfrequenzserkende Wirkung durch Stimulation von (wahrscheinlich im ZNS-lokalisierten) Dopamin-Rezeptoren [M.J. Brown and D. Harland, Brit. J. Pharmacol. 87, 361 (1986)] und ihre Affinität zu α2- und D2-Rezeptoren [G.Griss, R. Hurnaus et al.; EFMC-IXth Intern. Symp. on Medicinal Chemistry, Berlin (West), September 14-18, 1986 Short Communication Nr. 64, Abstract Seite 114] veröffentlicht.

Der vorliegenden Erfindung lag die Aufgabe zugrunde, neue Dopaminergika mit günstigeren Eigenschaften aufzufinden, die im Unterschied zu den vorbekannten Verbindungen der allgemeinen Formel I eine wesentlich verminderte Affinität zu α2-Adrenozeptoren besitzen, um so die Gefahr von α2-vermittelten Nebenwirkungen (Sedation, Ataxie, Hypotonie) zu verringern.

Gegenstand der vorliegenden Erfindung sind somit neue 4,5,7,8-Tetrahydro-6H-thiazolo[5,4-d]azepine der allgemeinen Formel II

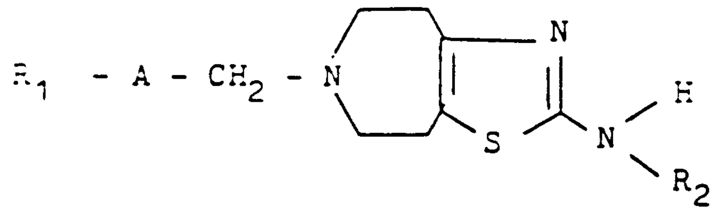

,(II)

deren Säureadditionssalze, insbesondere für die pharmazeutische Anwendung deren physiologisch verträgliche Additionssalze mit anorganischen oder organischen Säuren, ihre Herstellung und ihre Verwendung als Arzneimittel.

Die neuen Verbindungen weisen wertvolle pharmakologische Eigenschaften auf, nämlich selektive Wirkungen auf das dopaminerge System, die durch Stimulation von (vorrangig D2) Dopamin-Rezeptoren vermittelt werden. Daneben werden auch analgetische und antiinflammatorische Wirkungen sowie Serotonin-2-antagonistische Wirkungen beobachtet. Die erfindungsgemäßen Verbindungen sind auf Grund ihrer pharmakologischen Eigenschaften insbesondere zur Behandlung von Krankheiten des Zentralnervensystems wie Parkinsonismus, Hyperprolactinämie und Schizophrenie, ferner zur Behandlung von cardiovasculären Erkrankungen verwendbar.

In der obigen allgemeinen Formel II bedeuten
A eine Gruppe der Formeln

$-\underset{*}{C}(R_3)=CH-$, $-\underset{*}{C}H=C(R_4)-$, 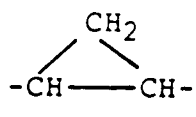, $-C{\equiv}C-$, $-\underset{*}{C}H=CH-CH_2-$,

$-\underset{*}{C}H(OR_5)-CH_2-$ oder $-(CH_2)_n-$

in welchen
n die Zahlen 2, 3 oder 4,
$R_3$ ein Wasserstoffatom oder eine Methylgruppe,
$R_4$ eine Alkylgruppe mit 1 bis 3 Kohlenstoffatomen oder eine Phenylgruppe und
$R_5$ ein Wasserstoffatom, eine Methyl- oder Äthylgruppe bedeuten und das mit * gekennzeichnete Konlenstoffatom mit dem Resp $R_1$ verknüpft ist, und
$R_1$ einen gegebenenfals durch ein Halogenatom, eine Alkoxygruppe mit 1 bis 4 Kohlenstoffatomen, eine Methyl-, Trifluormethyl-, Phenyl-, Nitro-, Amino-, Dimethylamino-, Piperidino-, Acetylamino-, Methylthio-, Methylsulfinyl-, Methylsulfonyl-, Cyano-, Aminocarbonyl-, Carboxy-, Methoxycarbonyl-, Äthoxycarbonyl-, Benzyloxy-, Pyridylmethoxy- oder Hydroxygruppe monosubstituierten Phenylrest, einen durch Methoxy-, Benzyloxy-, Hydroxy- oder Methylgruppen disubstituierten Phenylrest, wobei die Substituenten gleich oder verschieden sein können, oder einen durch drei Methoxygruppen, durch drei Hydroxygruppen oder durch eine Hydroxy- oder Aminogruppe und durch zwei Chlor- oder Bromatome trisubstituierten Phenylrest,
einen gegebenenfalls durch ein Chloratom, eine Methyl-, Methoxy-, Benzyloxy- oder Hydroxygruppe substituierten Pyridylrest,
einen Naphthyl-, Chinolyl-, Isochinolyl-, Indolyl-, Furyl-, Thienyl-, (2-Indolinon)yl-, Carbostyril- oder 3,4-Dihydrocarbostyrilrest,
einen gegebenenfalls in 2-Position durch eine Methyl- oder Aminogruppe substituierten Thiazolylrest,
einen Benzothiophenyl- oder Benzofuranylrest,
einen gegebenenfalls in 2-Position durch eine Methyl-, Phenyl- oder Aminogruppe substituierten Benzothiazolyl-, Benzoxazolyl- oder Benzimidazolylrest oder
A eine Kohlenstoff-Kohlenstoff-Bindung und
$R_1$ eine 1H-Inden-2-yl- oder 1,2-Dihydronaphthalin-3-yl-gruppe oder eine gegebenenfalls durch eine oder zwei Methyl-gruppen substituierte 2H-1-Benzopyran-3-yl- oder 2H-1-Benzothiopyran-3-yl-gruppe und
$R_2$ ein Wasserstoffatom oder eine gegebenenfalls in omega-Stellung durch einen Phenyl- oder 4-Methoxyphenylrest substituierte Acetyl- oder Propionylgruppe.

Für die bei der Definition der Reste eingangs erwähnten Bedeutungen kommen beispielsweise

für A die der Vinylen-, 2-Methyl-vinylen-, 1-Methyl-vinylen-, 1-Äthyl-vinylen-, 1-n-Propyl-vinylen-, 1-Isopropyl-vinylen-, 1-Phenyl-vinylen-, Cyclopropylen-, Äthinylen-, n-2-Propenylen-, 2-Hydroxy-äthylen-, 2-Methoxy-äthylen-, 2-Äthoxy-äthylen-, Äthylen-, n-Propylen- oder n-Butylengruppe und

für $R_1$ die der Phenyl-, 2-Chlor-phenyl-, 3-Chlor-phenyl-, 4-Chlor-phenyl-, 2-Methoxy-phenyl-, 3-Methoxy-phenyl-, 4-Methoxy-phenyl-, 2-Äthoxy-phenyl-, 3-Äthoxy-phenyl-, 4-Äthoxy-phenyl-, 2-n-Propoxy-phenyl-, 3-n-Propoxy-phenyl-, 4-n-Propoxy-phenyl-,2-Isopropoxy-phenyl-, 3-Isopropoxy-phenyl-, 4-Isopropoxy-phenyl-, 2-n-Butoxy-phenyl-, 3-n-Butoxy-phenyl-, 4-n-Butoxy-phenyl-, 2-sec.Butoxy-phenyl-, 3-sec.Butoxy-phenyl-, 4-sec.Butoxy-phenyl-, 2-Isobutoxy-phenyl-, 3-Isobutoxy-phenyl-, 4-Isobutoxy-phenyl-, 2-tert.Butoxy-phenyl-, 3-tert.Butoxy-phenyl-, 4-tert.Butoxy-phenyl-, 2-Methyl-phenyl-, 3-Methyl-phenyl-, 4-Methyl-phenyl-, 2-Trifluormethyl-phenyl-, 3-Trifluormethyl-phenyl-, 4-Trifluormethyl-phenyl-, 2-Biphenyl-, 3-Biphenyl-, 4-Biphenyl-, 2-Nitro-phenyl-, 3-Nitro-phenyl-, 4-Nitro-phenyl-, 2-Amino-phenyl-, 3-Amino-phenyl-, 4-Amino- phenyl-, 2-Dimethylamino-phenyl-, 3-Dimethylamino-phenyl-, 4-Dimethylamino-phenyl-, 2-Piperidino-phenyl-, 3-Piperidino-phenyl-, 4-Piperidino-phenyl-, 2-Acetylamino-phenyl-, 3-Acetylamino-phenyl-, 4-Acetylamino-phenyl-, 2-Methylthio-phenyl-,3-Methylthio-phenyl-, 4-Methylthio-phenyl-, 2-Methylsulfinyl-phenyl-, 3-Methylsulfinyl-phenyl-, 4-Methylsulfinyl-phenyl-, 2-Methylsulfonyl-phenyl-, 3-Methylsulfonyl-phenyl-, 4-Methylsulfonyl-phenyl-, 2-Cyano-phenyl-, 3-Cyano-phenyl-, 4-Cyano-phenyl-, 2-Aminocarbonyl-phenyl-, 3-Aminocarbonyl-phenyl-, 4-Aminocarbonyl-phenyl-, 2-Carboxy-phenyl-, 3-Carboxy-phenyl-, 4-Carboxy-phenyl-, 2-Methoxycarbonyl-phenyl-, 3-Methoxycarbonyl-phenyl-, 4-Methoxycarbonyl-phenyl-, 2-Äthoxycarbonyl-phenyl-, 3-Äthoxycarbonyl-phenyl-, 4-Äthoxycarbonyl-phenyl-, 2-Benzyloxy-phenyl-, 3-Benzyloxy-phenyl-,4-Benzyloxy-phenyl-, 2-(2-Pyridylmethoxy)phenyl-, 3-(2-Pyridylmethoxy)phenyl-, 4-(2-Pyridylmethoxy)phenyl-, 2-(3-Pyridylmethoxy)phenyl-, 3-(3-Pyridylmethoxy)phenyl-, 4-(3-Pyridylmethoxy)phenyl-, 2-(4-Pyridylmethoxy)phenyl-, 3-(4-Pyridylmethoxy)phenyl-, 4-(4-Pyridylmethoxy)phenyl-, 2-Hydroxy-phenyl-, 3-Hydroxy-phenyl-, 4-Hydroxy-phenyl-, 2,3-Dihydroxy-phenyl-, 2,4-Dihydroxy-phenyl-, 2,5-Dihydroxy-phenyl-,2,6-Dihydroxy-phenyl-, 3,4-Dihydroxy-phenyl-, 3,5-Dihydroxy-phenyl-, 2,3-Dimethoxy-phenyl-, 2,4-Dimethoxy-phenyl-,2,5-Dimethoxy-phenyl-, 2,6-Dimethoxy-phenyl-, 3,4-Dimethoxy-phenyl-, 3,5-Dimethoxy-phenyl-, 2,3-Dimethyl-phenyl-,2,4-Dimethyl-phenyl-, 2,5-Dimethyl-phenyl-, 2,6-Dimethyl-phenyl-, 3,4-Dimethyl-phenyl-, 3,5-Dimethyl-phenyl-, 2,3-Di(benzyloxy)phenyl-, 2,4-Di(benzyloxy)phenyl-, 2,5-Di(benzyloxy)phenyl-, 3,4-Di(benzyloxy)phenyl-, 3,5-Di(benzyloxy)phenyl-, 2-Hydroxy-3-methoxy-phenyl-, 2-Hydroxy-4-methoxy-phenyl-, 2-Hydroxy-5-methoxy-phenyl-, 2-Hydroxy-6-methoxy-phenyl-, 3-Hydroxy-2-methoxy-phenyl-, 3-Hydroxy-4-methoxy-phenyl-, 3-Hydroxy-5-methoxy-phenyl-, 5-Hydroxy-2-methoxy-phenyl-, 4-Hydroxy-2-methoxy-phenyl-, 4-Hydroxy-3-methoxy-phenyl-, 3-Benzyloxy-2-hydroxy-phenyl-, 2-Benzyloxy-3-methoxy-phenyl-, 2-Benzyloxy-4-methoxy-phenyl-, 2-Benzyloxy-5-methoxy-phenyl-, 2-Benzyloxy-6-methoxy-phenyl-, 3-Benzyloxy-2-methoxy-phenyl-, 3-Benzyloxy-4-methoxy-phenyl-, 3-Benzyloxy-5-methoxy-phenyl-, 5-Benzyloxy-2-methoxy-phenyl-, 4-Benzyloxy-2-methoxy-phenyl-, 4-Benzyloxy-3-methoxy-phenyl-, 2-Hydroxy-3-methyl-phenyl-, 2-Hydroxy-4-methyl-phenyl-, 2-Hydroxy-5-methyl-phenyl-, 2-Hydroxy-6-methyl-phenyl-, 3-Hydroxy-2-methyl-phenyl-, 3-Hydroxy-4-methyl-phenyl-, 3-Hydroxy-5-methyl-phenyl-, 5-Hydroxy-2-methyl-phenyl-, 4-Hydroxy-2-methyl-phenyl-, 4-Hydroxy-3-methyl-phenyl-, 2-Benzyloxy-3-methyl-phenyl-, 2-Benzyloxy-4-methylphenyl-, 2-Benzyloxy-5-methyl-phenyl-, 2-Benzyloxy-6-methyl-phenyl-, 3-Benzyloxy-2-methyl-phenyl-, 3-Benzyloxy-4-methyl-phenyl-, 3-Benzyloxy-5-methyl-phenyl-, 5-Benzyloxy-2-methyl-phenyl-, 4-Benzyloxy-2-methyl-phenyl-, 4-Benzyloxy-3-methyl-phenyl-, 2-Methoxy-3-methyl-phenyl-, 2-Methoxy-4-methyl-phenyl-, 2-Methoxy-5-methyl-phenyl-, 2-Methoxy-6-methyl-phenyl-, 3-Methoxy-2-methyl-phenyl-, 3-Methoxy-4-methyl-phenyl-, 3-Methoxy-5-methyl-phenyl-, 5-Methoxy-2-methyl-phenyl-, 4-Methoxy-2-methyl-phenyl-, 4-Methoxy-3-methyl-phenyl-, 2,3,4-Trimethoxy-phenyl-, 3,4,5-Trimethoxy-phenyl-, 2,4,5-Trimethoxy-phenyl-, 2,4,6-Trimethoxy-phenyl-, 2,3,4-Trihydroxy-phenyl-, 3,4,5-Trihydroxy-phenyl-, 2,4,5-Trihydroxy-phenyl-, 2,4,6-Trihydroxy-phenyl-, 3,5-Dichlor-4-hydroxy-phenyl-, 3,5-Dichlor-2-hydroxy-phenyl-, 3,5-Dibrom-4-hydroxy-phenyl-, 3,5-Dibrom-2-hydroxy-phenyl-, 2-Amino-3,5-dichlor-phenyl-, 4-Amino-3,5-dichlor-phenyl-, 2-Amino-3,5-dibrom-phenyl-, 4-Amino-3,5-dibrom-phenyl-, 2-Pyridyl-, 3-Pyridyl-, 4-Pyridyl, 3-Methyl-2-pyridyl-, 4-Methyl-2-pyridyl-, 5-Methyl-2-pyridyl-, 6-Methyl-2-pyridyl-,3-Methoxy-2-pyridyl-, 4-Methoxy-2-pyridyl-, 5-Methoxy-2-pyridyl-, 6-Methoxy-2-pyridyl-, 3-Benzyloxy-2-pyridyl-, 4-Benzyloxy-2-pyridyl-, 5-Benzyloxy-2-pyridyl-, 6-Benzyloxy-2-pyridyl-, 3-Chlor-2-pyridyl-, 4-Chlor-2-pyridyl-,5-Chlor-2-pyridyl-, 6-Chlor-2-pyridyl-, 3-Hydroxy-2-pyridyl-,4-Hydroxy-2-pyridyl-, 5-Hydroxy-2-pyridyl-, 6-Hydroxy-2-pyridyl-, 2-Methyl-3-pyridyl-, 4-Methyl-3-pyridyl-, 5-Methyl-3-pyridyl-, 6-Methyl-3-pyridyl-, 2-Methyl-4-pyridyl-,3-Methyl-4-pyridyl-, 1-Naphthyl-, 2-Naphthyl-, Chinolin-2-yl-, Chinolin-3-yl-, Chinolin-4-yl-, Chinolin-5-yl-, Chinolin-6-yl-, Chinolin-7-yl-, Chinolin-8-yl-, Isochinolin-1-yl-, Isochinolin-3-yl-, Isochinolin-4-yl-, Isochinolin-5-yl-, Isochinolin-6-yl-, Isochinolin-7-yl-, Isochinolin-8-yl-, 3-Indolyl-, 5-Indolyl-, 2-Furyl-, 3-Furyl-, 2-Thienyl-, 3-Thienyl-, Indolin-2-on-4-yl-, Indolin-2-on-5-yl-, Indolin-2-on-6-yl-, Indolin-2-on-7-yl-, 5-Carbostyril, 6-Carbostyril-, 7-Carbostyril-, 8-Carbostyril-, 3,4-Dihydro-5-carbostyril-, 3,4-Dihydro-6-carbostyril-, 3,4-Dihydro-7-carbostyril-, 3,4-Dihydro-8-carbostyril-, 2-Thiazolyl-, 4-Thiazolyl-, 5-Thiazolyl-, 2-Methyl-4-thiazolyl-, 2-Methyl-5-thiazolyl-, 2-Amino-4-thiazolyl-, 2-Amino-5-thiazolyl-, 2-Benzothiophenyl-, 3-Benzothiophenyl-, 4-Benzothiophenyl-, 5-Benzothiophenyl-, 6-Benzothiophenyl-, 7-Benzothiophenyl-, 2-Benzofuranyl-, 3-Benzofuranyl-, 4-Benzofuranyl-, 5-Benzofuranyl-, 6-Benzofuranyl-, 7-Benzofuranyl-, 2-Benzothiazolyl-, 4-Benzothiazolyl-, 5-Benzothiazolyl-, 6-Benzothiazolyl-, 7-Benzothiazolyl-, 2-Methyl-4-benzothiazolyl-, 2-Methyl-5-benzothiazolyl-, 2-Methyl-6-benzothiazolyl-, 2-Methyl-7-benzothiazolyl-, 2-Phenyl-4-benzothiazolyl-, 2-Phenyl-5-benzothiazolyl-2-Phenyl-6-benzothiazolyl 2-Phenyl-7-benzothiazo-lyl-, 2-Amino-4-benzothiazolyl-, 2-Amino-5-benzothiazolyl-, 2-Amino-6-benzothiazolyl-, 2-Amino-7-benzothiazolyl-, 2-Benzoxazolyl-, 4-Benzoxazolyl-, 5-Benzoxazolyl-, 6-Benzoxazolyl-, 7-Benzoxazolyl-, 2-Methyl-4-benzoxazolyl-, 2-Methyl-5-benzoxazolyl-,2-Methyl-6-benzoxazolyl-, 2-Methyl-7-benzoxazolyl-, 2-Phenyl-4-benzoxazolyl-, 2-Phenyl-5-benzoxazolyl-, 2-Phenyl-6-benzoxazolyl-, 2-Phenyl-7-benzoxazolyl-, 2-Amino-4-benzoxazolyl-, 2-Amino-5-benzoxazolyl-, 2-Amino-6-benzoxazolyl-, 2-Amino-7-benzoxazolyl-, 2-Benzimidazolyl-, 4-Benzimidazolyl-, 5-Benzimidazolyl-, 2-Methyl-4-benzimidazolyl-, 2-Methyl-5-benzimidazolyl-, 2-Phenyl-4-benzimidazolyl-, 2-Phenyl-5-benzimidazolyl-, 2-Amino-4-benzimidazolyl- oder 2-Amino-5-benzimidazolylgruppe oder

für A die einer Kohlenstoff-Kohlenstoff-Bindung und

für $R_1$ die der 1H-Inden-2-yl-, 1,2-Dihydro-naphthalin-3-yl-, 2H-1-Benzopyran-3-yl-, 2-Methyl-2H-1-benzopyran-3-yl-, 2,2-Dimethyl-2H-1-benzopyran-3-yl-, 2H-1-Benzothiopyran-3-yl-,2-Methyl-2H-1-benzothiopyran-3-yl- oder 2,2-Dimethyl-2H-1-benzothiopyran-3-yl-gruppe

sowie für $R_2$ die des Wasserstoffatoms, der Acetyl-, Phenylacetyl-, (4-Methoxy-phenyl)acetyl-, Propionyl-, 3-Phenylpropionyl- oder 3-(4-Methoxy-phenyl)propionylgruppe in Betracht.

Bevorzugte Verbindungen der obigen allgemeinen Formel II sind jedoch diejenigen, in denen

$R_2$ wie vorstehend definiert ist,

A eine Gruppe der Formeln

$-\underset{*}{C}(R_3)=CH-$, $-\underset{*}{C}H=C(R_4)-$, 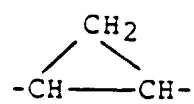, $-C\equiv C-$, $-\underset{*}{C}H=CH-CH_2-$,

$-\underset{*}{C}H(OR_5)-CH_2-$ oder $-(CH_2)_n-$

in welchen

n die Zahlen 2, 3 oder 4,

$R_3$ ein Wasserstoffatom oder eine Methylgruppe,

$R_4$ eine Alkylgruppe mit 1 bis 3 Kohlenstoffatomen oder eine Phenylgruppe und

$R_5$ ein Wasserstoffatom, eine Methyl- oder Äthylgruppe bedeuten und das mit * gekennzeichnete Kohlenstoffatom mit dem Rest $R_1$ verknüpft ist, und

$R_1$ eine gegebenenfalls durch ein Fluor-, Chlor- oder Bromatom, eine Alkoxygruppe mit 1 bis 4 Kohlenstoffatomen, eine Methyl-, Trifluormethyl-, Phenyl-, Hydroxy-, Benzyloxy-, Nitro-, Amino-, Dimethylamino-, Piperidino-, Cyano-, Aminocarbonyl-, Methoxycarbonyl-, Ethoxycarbonyl-, Methylmercapto-, Methylsulfinyl-, Methylsulfonyl- oder Pyridylmethoxygruppe substituierten Phenylrest, eine Dimethoxyphenyl-, Dihydroxyphenyl-, 4-Hydroxy-3,5-dichlor-phenyl-, 4-Hydroxy-3,5-dibrom-phenyl-, 4-Amino-3,5-dichlor-phenyl-, 4-Amino-3,5-dibrom-phenyl-, 3,4,5-Trimethoxy-phenyl-, Naphthyl-, 6-Chlor-2-pyridyl-, Thienyl-, Furyl-, Chinolyl-, Isochinolyl-, Benzothio-phenyl-, Indolyl- oder Indolin-2-on-4-yl-gruppe oder eine gegebenenfalls durch eine Methylgruppe substituierten Pyridylgruppe oder

A eine Kohlenstoff-Kohlenstoff-Bindung und

$R_1$ eine 1H-Inden-2-yl-, 1,2-Dihydronaphthalin-3-yl- oder 2H-1-Benzopyran-3-yl-gruppe bedeuten, insbesondere diejenigen Verbindungen der allgemeinen Formel II, in denen $R_1$ und A wie vorstehend definiert sind und $R_2$ ein Wasserstoffatom darstellt, und deren Säureadditionssalze, insbesondere deren physiologisch verträglichen Säureadditionssalze mit anorganischen oder organischen Säuren.

Besonders bevorzugte Verbindungen der allgemeinen Formel II sind diejenigen, in denen

A eine Vinylen-, Äthinylen-, Cyclopropylen- oder Äthylengruppe,

$R_1$ eine gegebenenfalls durch ein Chloratom, eine Hydroxy-, Methoxy-, Benzyloxy-, Isobutoxy-, Phenyl-,

Nitro-, Amino-, Cyano- oder Piperidinogruppe substituierte Phenylgruppe, eine gegebenenfalls durch eine Methylgruppe substituierte Pyridylgruppe, eine Dimethoxyphenyl-, Naphthyl-, Isochinolyl-, 2-Methyl-thiazolyl-, Furyl- oder Thienylgruppe und

$R_2$ ein Wasserstoffatom bedeuten, und deren Säureadditionssalze, insbesondere deren physiologisch verträglichen Säureadditionssalze mit anorganischen oder organischen Säuren.

Erfindungsgemäß erhält man die neuen Verbindungen nach folgenden Verfahren:

a) Umsetzung einer Verbindung der allgemeinen Formel III

$$R_1 - A - CH_2 - X \quad ,(III)$$

in der

A und $R_1$ wie eingangs definiert sind und

X eine nukleophile Austrittsgruppe wie ein Chlor- oder Bromatom, einen Methansulfonyloxy-, Trifluormethansulfonyloxy-oder Tosyloxyrest bedeutet,

mit einer Verbindung der allgemeinen Formel IV

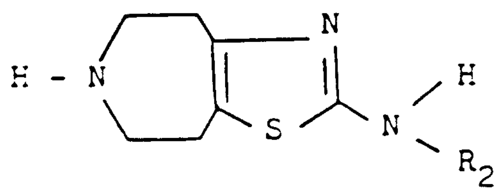

, (IV)

in der

$R_2$ wie eingangs definiert ist.

Die Umsetzung wird vorzugsweise in einem Lösungsmittel wie Aceton, Dioxan, Tetrahydrofuran, Methylenchlorid, Chloroform, Acetonitril, Dimethylformamid oder Dimethylsulfoxid zweckmäßigerweise in Gegenwart eines säurebindenden Mittels wie Kaliumcarbonat, Triethylamin, Pyridin oder auch in Gegenwart eines Überschusses der eingesetzten Verbindung der Formel IV von ein bis drei Äquivalenten bei Temperaturen zwischen -10 und 100 °C, vorzugsweise jedoch bei Temperaturen zwischen 0 und 80 °C, durchgeführt. Ferner kann es von Vorteil sein, wenn die Umsetzung unter Schutzgas, z.B. unter Stickstoff, durchgeführt wird.

b) Zur Herstellung von Verbindungen der allgemeinen Formel II, in der $R_1$ mit Ausnahme eines gegebenenfalls durch ein Chloratom, eine Methyl-, Methoxy-, Benzyloxy- oder Hydroxygruppe substituierten 2-Pyridylrestes, eines 2-Chinolyl-, 1-Isochinolyl-, 3-Isochinolyl-, 2-Thiazolyl-, 2-Benzoxazolyl-oder 2-Benzimidazolylrestes die für $R_1$ eingangs erwähnten Bedeutungen besitzt:

Reduktive Aminierung eines Aldehyds der allgemeinen Formel V

$$R_1' - A - \overset{H}{C} = O \quad , (V)$$

in der

A wie eingangs definiert ist und

$R_1'$ mit Ausnahme eines gegebenenfalls durch ein Chloratom, eine Methyl-, Methoxy-, Benzyloxy- oder Hydroxygruppe substituierten 2-Pyridylrestes, eines 2-Chinolyl-, 1-Isochinolyl-, 3-Isochinolyl-, 2-Thiazolyl-, 2-Benzoxazolyl- oder 2-Benzimidazolylrestes die für $R_1$ eingangs erwähnten Bedeutungen besitzt,

mit einer Verbindung der allgemeinen Formel IV

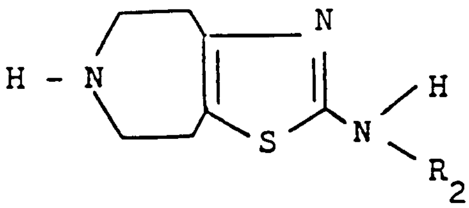

, (IV)

in der
$R_2$ wie eingangs definiert ist.

Die reduktive Aminierung, die über die entsprechende intermediär gebildete Immoniumverbindung läuft, wird in einem geeigenten Lösungsmittel wie Methanol, Äthanol, Tetrahydrofuran oder Dioxan in Gegenwart einer Säure, vorzugsweise eines Äquivalentes einer Säure wie Eisessig, und in Gegenwart eines geeigneten Reduktionsmittels wie einem komplexen Metallhydrid, vorzugsweise jedoch in Gegenwart von Natriumcyanoborhydrid, bei Temperaturen zwischen -10 und 50 ° C, vorzugsweise jedoch bei Temperaturen zwischen 0 und 20 ° C, durchgeführt.

c) Umsetzung eines 5-Halogen-azepin-4-ons der allgemeinen Formel VI

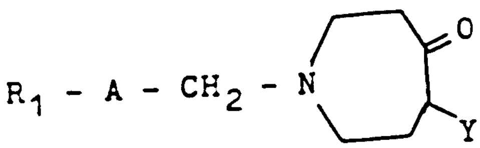

x HY , (VI)

in der
A und $R_1$ wie eingangs definiert sind und
Y ein Brom- oder Chloratom bedeutet,
mit einem Thioharnstoff der allgemeinen Formel VII

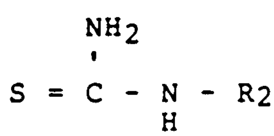

, (VII)

in der
$R_2$ wie eingangs definiert ist.

Die Umsetzung wird in der Schmelze oder in einem Lösungsmittel wie Ethanol, Chloroform, Dioxan, Pyridin, Tetrahydrofuran oder Dimethylformamid gegebenenfalls in Gegenwart eines säurebindenden Mittels wie Natriumacetat, Kaliumcarbonat, Triethylamin oder Pyridin bei Temperaturen zwischen 0 und 150 ° C, vorzugsweise jedoch bei Temperaturen zwischen 50 und 100 ° C, durchgeführt.

d) Zur Herstellung von Verbindungen der allgemeinen Formel II, in der $R_2$ ein Wasserstoffatom bedeutet: Umsetzung eines Azepin-4-ons der allgemeinen Formel VIII

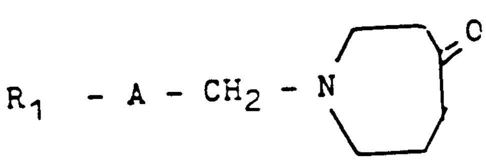

, (VIII)

in der
A und $R_1$ wie eingangs definiert sind,
mit einem Formamidin-disulfid-Salz der allgemeinen Formel IX

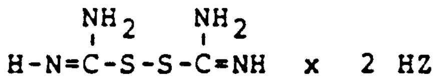

, (IX)

in der

Z einen Rest einer anorganischen oder organischen Säure darstellt.

Die Umsetzung wird in der Schmelze oder in einem Lösungsmittel wie Glykol, Dimethylformamid, Eisessig, Propionsäure oder Eisessig/Glykol bei Temperaturen zwischen 50 oder 150 °C, vorzugsweise bei Temperaturen zwischen 70 oder 120 °C, durchgeführt.

e) Zur Herstellung von Verbindungen der allgemeinen Formel II, in der $R_1$ einen Phenyl-, Naphthyl-, Pyridyl-, Chinolinyl-, Isochinolinyl-, Furyl-, Thienyl-, Benzofuryl-, Benzothienyl-, (2-Indolinon)yl-, Carbostyril- oder 3,4-Dihydrocarbostyrilrest und A eine Äthinylengruppe bedeuten:

Umsetzung einer Verbindung der allgemeinen Formel X

$R_1''$ - Hal ,(X)

in der

$R_1''$ einen Phenyl-, Naphthyl-, Pyridyl-, Chinolinyl-, Isochinolinyl-, Furyl-, Thienyl-, Benzofuryl-, Benzothienyl-, (2-Indolinon)yl-, Carbostyril- oder 3,4-Dihydro-carbostyrilrest und

Hal ein Brom- oder Jodatom bedeuten,

mit einer Propargyl-Verbindung der allgemeinen Formel XI

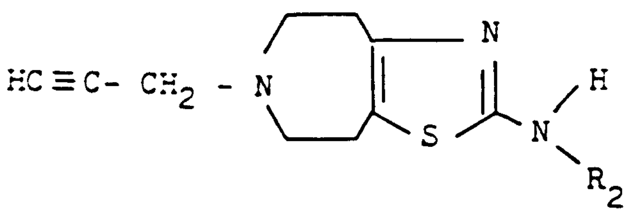

, (XI)

in der

$R_2$ wie eingangs definiert ist.

Die Umsetzung wird vorzugsweise in einem basischen Lösungsmittel wie Diäthylamin, Triäthylamin, Triäthylamin/Acetonitril oder Triäthylamin/N,N-Dimethylacetamid in Gegenwart katalytischer Mengen von Kupfer(I)jodid und eines Nickel- oder Palladium-triphenylphosphin-Komplexes, vorzugsweise Bis-(triphenylphosphin)-palladiumchlorid, bei Temperaturen zwischen 0 und 120 °C, vorzugsweise bei Temperaturen zwischen 20 und 100 °C, durchgeführt.

f) Zur Herstellung von Verbindungen der allgemeinen Formel II, in der A eine Vinylengruppe bedeutet: Reduktion einer Propargyl-Verbindung der allgemeinen Formel XII

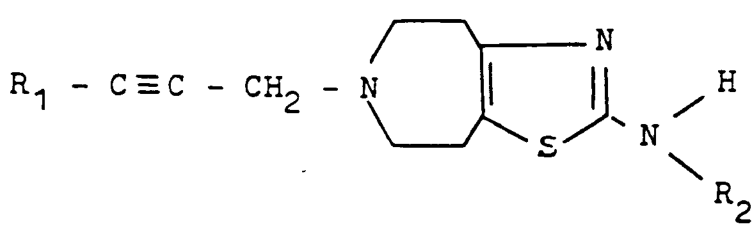

, (XII)

in der

$R_1$ und $R_2$ wie eingangs definiert sind.

Die Reduktion wird in einem geeigneten Lösungsmittel wie Äthanol, Essigester, Tetrahydrofuran, Eisessig oder Dioxan mit einem geeigneten Reduktionsmittel wie nascierendem Wasserstoff, z.B. in Gegenwart von Zink/Eisessig, Zinn/Salzsäure oder Zinn(II)chlorid/Salzsäure, oder vorzugsweise mit

Wasserstoff, z.B. bei einem Wasserstoffdruck von 1 bis 5 bar, in Gegenwart eines geeigneten Katalysators wie Palladium/Bariumsulfat, bei Temperaturen zwischen 0 und 50 ° C, vorzugsweise bei Raumtemperatur, durchgeführt.

Bei der katalytischen Hydrierung erhält man bevorzugt das entsprechende Z-Isomere.

g) Zur Herstellung von Verbindungen der allgemeinen Formel II, in der A eine Äthylengruppe bedeutet: Reduktion einer Verbindung der allgemeinen Formel XIII

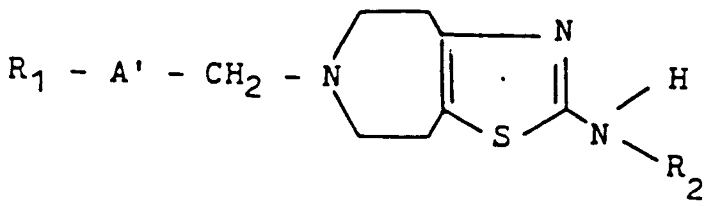

, (XIII)

in der
$R_1$ und $R_2$ wie eingangs definiert sind und
A' eine Vinylen- oder Äthinylengruppe bedeutet.

Die Reduktion wird ein einem geeigneten Lösungsmittel wie Äthanol, Essigester, Tetrahydrofuran, Eisessig oder Dioxan mit einem geeigneten Reduktionsmittel wie nascierendem Wasserstoff, z.B. in Gegenwart von Zink/Eisessig, Zinn/Salzsäure oder Zinn(II)chlorid/Salzsäure, oder mit Wasserstoff, z.B. bei einem Wasserstoffdruck von 1 bis 5 bar, in Gegenwart eines geeigneten Katalysators wie Palladium/Kohle, bei Temperaturen zwischen 0 und 50 ° C, vorzugsweise bei Raumtemperatur, durchgeführt.

h) Zur Herstellung von Verbindungen der allgemeinen Formel II, in der A eine Cyclopropylen- oder eine n-Alkylengruppe mit zwei bis vier Kohlenstoffatomen und $R_2$ ein Wasserstoffatom darstellen: Reduktion eines Amids der allgemeinen Formel XIV

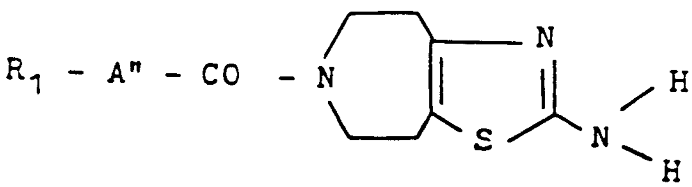

, (XIV)

in der
$R_1$ wie eingangs definiert ist und
A″ eine Cyclopropylen- oder eine n-Alkylengruppe mit zwei bis vier Kohlenstoffatomen bedeuten.

Die Reduktion wird in einem geeigneten Lösungsmittel wie Diäthyläther, Tetrahydrofuran, Dioxan, Eisessig, Trifluoressigsäure, Methanol oder Äthanol in Gegenwart eines geeigneten Reduktionsmittels wie einem komplexen Metallhydrid, beispielsweise Lithiumaluminiumhydrid, Natriumborhydrid/Bortrifluorid, Natriumborhydrid/Aluminiumchlorid, Diboran oder Boran-Dimethylsulfid-Komplex, vorzugsweise jedoch mit Lithiumaluminiumhydrid in Tetrahydrofuran, bei Temperaturen zwischen 0 und 80 ° C, vorzugsweise bei Temperaturen zwischen 20 und 40 ° C, durchgeführt.

i) Zur Herstellung von Verbindungen der allgemeinen Formel II, in der A eine Gruppe der Formel $-CH(OR_5)-CH_2-$ bedeutet:
Reduktion eines Ketons der allgemeinen Formel XV

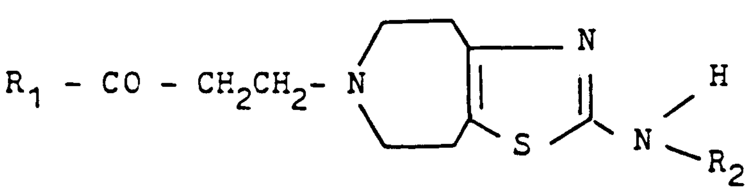

, (XV)

in der
$R_1$ und $R_2$ wie eingangs definiert sind.

Die Reduktion wird in einem geeigneten Lösungsmittel wie Methanol, Methanol/Wasser, Äthanol, Äthanol/Wasser, Tetrahydrofuran/Wasser oder Dioxan/Wasser in Gegenwart eines geeigneten komplexen Metallhydrids wie Natriumborhydrid bei Temperaturen zwischen 0 und 40 °C, vorzugsweise jedoch bei Raumtemperatur, durchgeführt.

k) Zur Herstellung von Verbindungen der allgemeinen Formel II, in der A eine Vinylengruppe bedeutet:
Dehydratisierung eines Alkohols der allgemeinen Formel XVI

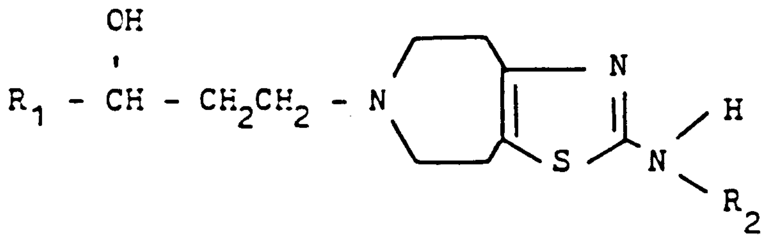

, (XVI)

in der
$R_1$ und $R_2$ wie eingangs definiert sind.

Die Dehydratisierung wird gegebenenfalls in einem Lösungsmittel wie Äthanol, Isopropanol, Methylenchlorid, Toluol oder Pyridin in Gegenwart eines wasserentziehenden Mittels wie Phosphorpentoxid, Schwefelsäure, p-Toluolsulfonsäure, p-Toluolsulfonsäurechlorid oder eines sauren Ionenaustauschers bei Temperaturen zwischen 20 und 100 °C, vorzugsweise bei Temperaturen zwischen 30 und 80 °C, durchgeführt.

l) Zur Herstellung von Verbindungen der allgemeinen Formel II, in der $R_2$ eine gegebenenfalls in omega-Stellung durch einen Phenyl- oder 4-Methoxyphenylrest substituierte Acetyl-oder Propionylgruppe bedeutet:
Acylierung eines Amines der allgemeinen Formel XVII

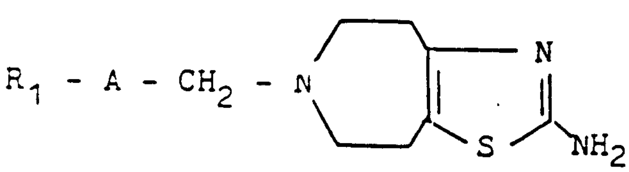

, (XVII)

in der
A und $R_1$ wie eingangs definiert sind,
mit einer Carbonsäure der allgemeinen Formel XVIII

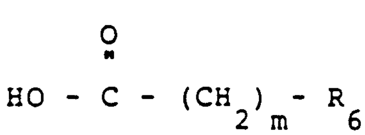

, (XVIII)

in der
m die Zahl 1 oder 2 und
$R_6$ ein Wasserstoffatom, einen Phenyl- oder 4-Methoxyphenylrest bedeuten,
oder mit deren gegebenenfalls im Reaktionsgemisch hergestellten reaktionsfähigen Derivaten.

Als reaktionsfähige Derivate einer Verbindung der allgemeinen Formel XVIII kommen beispielsweise deren Ester wie der Methyl-, Äthyl- oder Benzylester, deren Thioester wie der Methylthio- oder Äthylthioester, deren Halogenide wie das Säurechlorid, deren Anhydride oder Imidazolide in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid,

gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen -25 und 250 °C, vorzugsweise jedoch bei Temperaturen zwischen -10 °C und der Siedetemperatur des verwendeten Lösungsmittels, durchgeführt. Die Umsetzung kann auch ohne Lösungsmittel durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

m) Zur Herstellung von Verbindungen der allgemeinen Formel II, in der $R_2$ ein Wasserstoffatom bedeutet:

Desacylierung einer Verbindung der allgemeinen Formel XIX

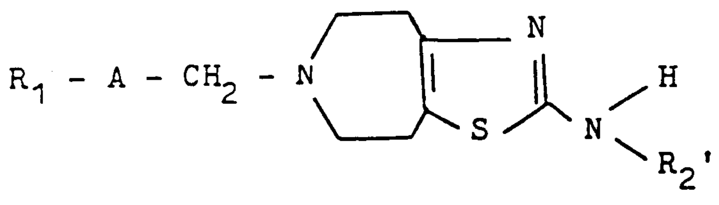

, (XIX)

in der

A und $R_1$ wie eingangs definiert sind und

$R_2'$ einen hydrolytisch abspaltbaren Rest wie einen Acyl-oder Kohlensäureesterrest, z.B. eine Acetyl-, Propionyl-, Benzoyl-, Methoxycarbonyl-, Äthoxycarbonyl- oder Benzyloxycarbonylgruppe, darstellt.

Die Desacylierung erfolgt vorzugsweise hydrolytisch, zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen -10 und 120 °C, z.B. bei Temperaturen zwischen Raumtemperatur und der Siedetemperatur des Reaktionsgemisches.

n) Zur Herstellung von Verbindungen der allgemeinen Formel II, in der A mit Ausnahme der $-CH(OR_5)-CH_2$-Gruppe die für A eingangs erwähnten Bedeutungen besitzt und $R_1$ einen durch eine Hydroxygruppe substituierten Phenyl-, Methylphenyl-, Methoxyphenyl- oder Pyridylrest oder einen durch zwei oder drei Hydroxygruppen substituierten Phenylrest und $R_2$ ein Wasserstoffatom bedeuten:

Ätherspaltung einer Verbindung der allgemeinen Formel XX

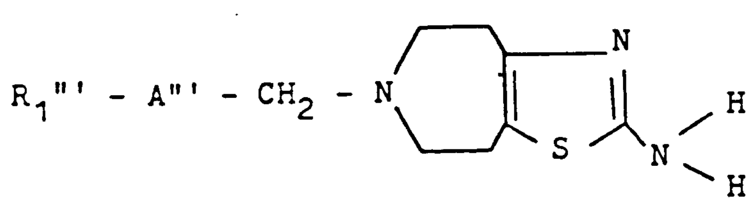

, (XX)

in der

$A'''$ mit Ausnahme der $-CH(OR_5)-CH_2$-Gruppe die für A eingangs erwähnten Bedeutungen besitzt und

$R_1'''$ einen durch eine Benzyloxy- oder Methoxygruppe substituierten Phenyl-, Methylphenyl-, Methoxyphenyl- oder Pyridylrest oder einen durch zwei oder drei Benzyloxy- oder Methoxygruppen substituierten Phenylrest bedeutet.

Die Ätherspaltung wird zweckmäßigerweise in Gegenwart einer Säure wie Chlorwasserstoff, Bromwasserstoff, Schwefelsäure, Bortribromid, Aluminiumtrichlorid oder Pyridinhydrochlorid und zweckmäßigerweise in einem geeigneten Lösungsmittel wie Methylenchlorid, Eisessig oder Wasser oder in deren Gemischen bei Temperaturen zwischen -78 und 250 °C durchgeführt. Hierbei wird die Ätherspaltung mit einer Protonensäure zweckmäßigerweise bei Temperaturen zwischen 0 und 150 °C, vorzugsweise bei Temperaturen zwischen 50 und 150 °C, oder mit einer Lewis-Säure vorzugsweise in einem Lösungsmittel wie Methylenchlorid bei Temperaturen zwischen -78 und 20 °C durchgeführt.

Erhält man erfindungsgemäß eine Verbindung der allgemeinen Formel II, in der $R_1$ einen Nitrophenylrest bedeutet, so kann diese mittels Reduktion in eine entsprechende Verbindung, in der $R_1$ einen Aminophenylrest bedeutet, übergeführt werden oder
eine Verbindung der allgemeinen Formel II, in der $R_1$ einen Cyanophenylrest bedeutet, so kann diese mittels Hydratisierung in eine entsprechende Verbindung, in der $R_1$ einen Aminocarbonylphenylrest bedeutet, übergeführt werden oder
eine Verbindung der allgemeinen Formel II, in der $R_1$ einen Cyanophenylrest bedeutet, so kann diese mittels Alkoholyse in eine Verbindung, in der $R_1$ einen Methoxycarbonylphenyl- oder Äthoxycarbonylphenylrest bedeutet, übergeführt werden oder
eine Verbindung der allgemeinen Formel II, in der $R_1$ einen Cyanophenylrest bedeutet, so kann diese mittels Hydrolyse in eine Verbindung, in der $R_1$ einen Carboxyphenylrest bedeutet, übergeführt werden oder
eine Verbindung der allgemeinen Formel II, in der $R_1$ einen Hydroxyphenylrest bedeutet, so kann diese mittels Benzylalkohol oder einem Pyridylmethanol und einem Azodicarbonsäurediester in eine Verbindung, in der $R_1$ einen Benzyloxyphenyl-oder Pyridylmethoxyphenylrest bedeutet, übergeführt werden.

Die nachträgliche Reduktion der Nitroverbindung wird vorzugsweise in einem Lösungsmittel wie Wasser, Wasser/Äthanol, Methanol, Eisessig, Essigsäureäthylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50 °C, vorzugsweise jedoch bei Raumtemperatur durchgeführt.

Die nachträgliche Hydratisierung zu einer Aminocarbonylverbindung wird vorzugsweise durch Erhitzen in Polyphosphorsäure auf Temperaturen zwischen 50 und 150 °C, vorzugsweise auf Temperaturen zwischen 80 und 100 °C, durchgeführt.

Die nachträgliche Alkoholyse zu einer Esterverbindung wird zweckmäßigerweise in Gegenwart von Halogenwasserstoff, vorzugsweise Chlorwasserstoff, und in Gegenwart eines entsprechenden Alkohols wie Methanol oder Äthanol bei erhöhten Temperaturen, z.B. bei der Siedetemperatur des Reaktionsgemisches, durchgeführt.

Die nachträgliche Hydrolyse zu einer Carboxyverbindung wird vorzugsweise in Gegenwart einer Säure wie Salzsäure, Schwefelsäure, Phosphorsäure oder Trifluoressigsäure oder in Gegenwart einer Base wie Natriumhydroxid oder Kaliumhydroxid in einem geeigneten Lösungsmittel wie Wasser, Äthanol, Wasser/Äthanol, Wasser/Isopropanol oder Wasser/Dioxan bei erhöhten Temperaturen, z.B. bei der Siedetemperatur des Reaktionsgemisches, durchgeführt.

Die nachträgliche Überführung in eine Benzyloxy- oder Pyridylmethoxyverbindung wird mittels der sogenannten Mitsunobu-Reaktion vorzugsweise in Gegenwart von Azodicarbonsäuredimethylester oder -diäthylester und in einem inerten Lösungsmittel wie Tetrahydrofuran, Methylenchlorid oder Acetonitril bei Temperaturen zwischen 0 und 40 °C, vorzugsweise jedoch bei Raumtemperatur, durchgeführt.

Desweiteren können die erhaltenen Verbindungen der Formel II in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formel III bis XX sind teilweise literaturbekannt oder nach literaturbekannten Methoden erhältlich.

So erhält man die Ausgangsverbindungen III aus den entsprechenden Alkoholen durch Umsetzung beispielsweise mit Thionylchlorid, Mesylchlorid, Phosphortribromid oder Tetrabromkohlenstoff/Triphenylphosphin. Die entsprechenden Alkohole sind ihrerseits aus den entsprechenden Aldehyden oder den entsprechenden Carbonsäureestern durch Reduktion herstellbar.

Die Allylalkohole der Formel $R_1$-CH = CH-$CH_2$OH erhält man auch aus den entsprechenden Aldehyden durch Wittig-Olefinierung mit den entsprechenden Derivaten des 2-Hydroxyäthylidentriphenylphosphorans, bei denen die Hydroxygruppe beispielsweise durch Ketalisierung geschützt ist, und anschließende Entfernung der verwendeten Schutzgruppe.

Die Ausgangsverbindungen V erhält man aus den entsprechenden Alkoholen durch Oxidation beispielsweise mit Mangandioxid, aus den entsprechenden Säurechloriden durch Reduktion beispielsweise mit Organo-zinnhydriden oder Amino-organo-siliciumhydriden, aus den entsprechenden Carbonsäuren beispielsweise mit Amino-organo-siliciumhydriden, oder, wenn A eine Vinylen-gruppe bedeutet, durch Wittig-Olefinierung mit Formylmethylen-triphenylphosphoran aus den entsprechenden Aldehyden der allgemeinen Formel $R_1$-CH=O.

Die Ausgangsverbindungen VIII erhält man beispielsweise aus Hexahydro-4H-4-azepinon-hydrochlorid durch Umsetzung mit Verbindungen der allgemeinen Formel III, bevorzugt in Dimethylformamid bei Raumtemperatur in Gegenwart von Kaliumcarbonat, oder durch entsprechende Umsetzung des Äthylenketals von Hexahydro-4H-4-azepinon mit anschließender Entketalisierung.

Die Ausgangsverbindungen VI erhält man aus den Verbindungen VIII beispielsweise durch Umsetzung mit Brom in Eisessig in Gegenwart von Bromwasserstoff.

Die Ausgangsverbindungen IV werden teilweise in der GB-A-1.321.509 beschrieben.

Die Ausgangsverbindungen der Formeln XI bis XV, XVII, XIX und XX erhält man aus den Verbindungen IV durch Umsetzung mit den entsprechenden Verbindungen unter Alkylierung bzw. reduktiver Aminierung oder Acylierung.

Die Ausgangsverbindungen XVI werden durch Reduktion der Verbindungen XV beispielsweise mittels Natriumborhydrid erhalten.

Wie bereits eingangs erwähnt, weisen die neuen Verbindungen wertvolle pharmakologische Eigenschaften auf, nämlich selektive Wirkungen auf das dopaminerge System, die durch Stimulation von (vorrangig D2) Dopamin-Rezeptoren vermittelt werden. Die erfindungsgemäßen Verbindungen eignen sich insbesondere zur Behandlung von Krankheiten des Zentralnervensystems wie Parkinsonismus, Hyperprolactinämie und Schizophrenie, ferner zur Behandlung von Cardiovasculären Erkrankungen, von Ischämien und des cardiogenen Schocks. Daneben werden auch analgetische und antiinflammatorische Wirkungen sowie Serotonin-2-antagonistische Wirkungen und eine inhibierende Wirkung auf Granulocyten-abhängige Prozesse, z.B. auf die Sauerstoffradikalbildung, beobachtet. - Die erfindungsgemäßen Verbindungen, bei denen $R_2$ einen der eingangs definierten Acylreste bedeutet, sind wahrscheinlich Prodrugs der entsprechenden Verbindungen, in denen $R_2$ ein Wasserstoffatom bedeutet.

Die biologischen Eigenschaften der nachstehend aufgeführten erfindungsgemäßen Verbindungen wurden mit Hilfe folgender Methoden geprüft:

1. Bestimmung der Affinität zu Dopamin $D_2$-Rezeptoren durch Verdrängungsexperimente mit [$^3$H]-Spiperon. [Modifizierte Methode von W. Billard et al., Life Sci. 35, 1885 (1984) sowie D.J. de Vries und P.M. Beart, Eur. J. Pharmacol. 109, 417 (1985)]

Membranpräparation

Männliche Ratten (Stamm Chbb:Thom, ca. 200 g) wurden durch Genickschlag getötet. Die Gehirne wurden entnommen und auf Eis seziert. Die Striata wurden herauspräpariert, gewogen und in 25 Volumina Tris-Puffer (50 mM Tris-HCl, 1 nM EDTA, 5 mM $MgCl_2$ und 1 mM Ascorbinsäure) 30 sec. am Ultra-Turrax bei max. Geschwindigkeit, gefolgt von 10 Hüben in einem Potter-Elvehjem bei 1400 rpm homogenisiert. Das nach Zentrifugation mit 50.000 x g bei 4°C für 15 min. erhaltene Pellet wurde erneut in 25 ml Tris-Puffer aufgenommen und unter obigen Bedingungen zentrifugiert. Der Überstand wurde verworfen und das erhaltene Pellet in 25 ml Tris-Puffer für 30 min. bei 37°C inkubiert und anschließend erneut mit 50.000 x g bei 4°C für 15 min. zentrifugiert. Schließlich wurde das erhaltene Pellet mit Tris-Puffer zu einer Homogenatverdünnung von 1:500 (bezogen auf das Gewicht der Striata) versetzt.

Bindungsassay

Aliquote von 1 ml der Membranpräparation wurden mit 1 ml einer Lösung von 0.25 nM [$^3$H]-Spiperon (0.75 GBq/mMol, Fa. DuPont NEN) und steigender Konzentration der Testsubstanz ($10^{-11}$ bis $10^{-4}$M) bei Raumtemperatur für eine Stunde inkubiert. Die Inkubation wurde durch Zugabe von 5 ml eiskaltem Tris-Puffer und Filtration durch Whatman GF/B-Filter beendet. Die Filter wurden zweimal mit je 5 ml eiskaltem Puffer gewaschen. Die Radioaktivität der Filter wurde durch Flüssigszintillationsmessung in Instagel (R) (Fa. Canberra Packard) bestimmt.
Die unspezifische Bindung wurde in Gegenwart von $10^{-5}$ M Haloperidol (Sigma Chemical Co.) ermittelt.

Datenanalyse

Verdrängungskurven wurden aus den ermittelten Daten mit Hilfe des TOPFIT-Programmpaketes (G. Heinzel in "Pharmacokinetics During Drug Development: Data Analysis and Evaluation Techniques", G. Bozler und J.M. van Rossum Eds., G. Fischer Verlag, Stuttgart 1982, 207) erhalten.
Die Substanzen verdrängen den Radioliganden in einer für Agonisten typischen biphasischen Weise; die angegebenen D2-Ki-Werte [Ki = $IC_{50}$: (1 + $C_L/K_L$), wobei $C_L$ und $K_L$ die Konzentration bzw. die Dissoziationskonstante des eingesetzten Radioliganden bedeuten (siehe Cheng und Prusoff in Biochem. Pharmacol. 22, 3099 (1973))] beziehen sich auf die hochaffine Form des Dopamin-Rezeptors.

**2. Bestimmung der Affinität zu Alpha$_2$-Rezeptoren durch Verdrängungsexperimente mit [$^3$H]-Clonidin [Modifizierte Methode von B. Jarrott, W.J. Louis und R.J. Summers, Biochem. Pharmacol. 27, 141 (1979)]**

Membranpräparation

Eine männliche Ratte (Stamm Chbb:Thom, ca. 200 g) wurde durch Genickschlag getötet. Das Gehirn wurde entnommen und der Cortex präpariert, gewogen und in 25 Volumina Tris-Puffer (50 mM Tris-HCl, pH 7.50) 30 Sekunden am Ultra-Turrax bei max. Geschwindigkeit, gefolgt von 10 Hüben in einem Potter-Elvehjem bei 1400 rpm homogenisiert. Das Homogenat wurde mit Tris-Puffer zu einer Homogenatverdünnung von 1:50 (bezogen auf das Gewicht des Cortex) versetzt.

Bindungsassay

Aliquots von 1 ml der Membranpräparation wurden mit 1 ml einer Lösung von 1 nM [$^3$H]-Clonidin (2.2 TBq/mMol, Fa. DuPont NEN) und steigender Konzentration der Testsubstanz ($10^{-11}$ bis $10^{-4}$ M) bei Raumtemperatur für 3 Stunden inkubiert. Die Inkubation wurde durch Zugabe von 5 ml eiskaltem Tris-Puffer und Filtration durch Whatman GF/B-Filter beendet. Die Filter wurden zweimal mit je 5 ml eiskaltem Puffer gewaschen. Die Radioaktivität der Filter wurde durch Flüssigszintillationsmessung in Instagel(R) (Fa. Canberra Packard) bestimmt.
Die unspezifische Bindung wurde in Gegenwart von $10^{-5}$ M Oxymetazolin (Sigma Chemical Co.) ermittelt.

Datenanalyse

Verdrängungskurven wurden aus den ermittelten Daten mit Hilfe des TOPFIT-Programmpaketes (G. Heinzel in "Pharmacokinetics During Drug Development: Data Analysis and Evaluation Techniques", G. Bozler und J.M. van Rossum Eds., G. Fischer Verlag, Stuttgart 1982, 207) erhalten.
In der nachstehenden Tabelle 1 sind die D2-Ki-Werte und $\alpha$2-$IC_{50}$-Werte von erfindungsgemäßen Verbindungen aufgeführt. Der Quotient $\alpha$2-$IC_{50}$/D2-Ki stellt eine Maßzahl für die relative Affinität einer Substanz zu Dopamin-D2-Rezeptoren im Vergleich zu $\alpha$2-Adrenozeptoren dar. Je größer diese Maßzahl ist, umso höher ist die D2/$\alpha$2-Selektivität.

Tabelle 1

| Verbindung (Beispiel Nr.) | D2-Ki [nM] | $\alpha2\text{-}IC_{50}$ [nM] | $\frac{\alpha2\text{-}IC_{50}}{D2\text{-}Ki}$ |
|---|---|---|---|
| 3a | 7 | 620 | 88 |
| 3b | 24 | 810 | 145 |
| 4d | 10 | 890 | 89 |
| 4e | 3 | 190 | 63 |
| 4g | 2 | 240 | 120 |
| 4i | 13 | 2.050 | 157 |
| 4o | 1,3 | 550 | 423 |
| 4p | 0,89 | 89 | 100 |
| 4t | 3,1 | 460 | 148 |
| 4u | 6,9 | 2.200 | 318 |
| 4v | 8 | 2.300 | 287 |
| 4w | 3 | 2.200 | 733 |
| 4y | 0,73 | 300 | 410 |
| 4z | 8,4 | 3.800 | 452 |
| 7a | 0,92 | 3.100 | 3.370 |
| 8n | 15 | 2.000 | 133 |
| 8o | 14 | 3.300 | 235 |
| 9 | 6,7 | 530 | 79 |

Fortsetzung Tabelle 1

| Verbindung (Beispiel Nr.) | D2-Ki [nM] | $\alpha$2-$IC_{50}$ [nM] | $\frac{\alpha 2\text{-}IC_{50}}{D2\text{-}Ki}$ |
|---|---|---|---|
| 10 | 17 | 15.000 | 882 |
| 11a | 4,3 | 760 | 176 |
| 11i | 2,6 | 1.500 | 576 |
| 11 | 3,2 | 820 | 256 |
| 11p | 9,9 | 3.000 | 303 |
| 12b | 4,4 | 2.000 | 450 |
| 14b | 2,6 | 500 | 192 |
| 15 | 0,95 | 590 | 621 |
| 16 | 1,4 | 400 | 285 |
| 24a | 1,7 | 900 | 529 |
| Verb. A | 5 | 24 | 4,8 |
| Verb. B | 13 | 410 | 31,5 |
| Verb. C* | 410 | 5.100 | 12,4 |

* Verbindung C = 2-Amino-6-(2-phenyläthyl)-4,5,7,8-tetrahydro-6H-thiazolo[5,4-d]azepin (siehe GB-A-1.321.509)

3. Bestimmung der Affinität zu Serotonin-1A(5-HT1A)-Rezeptoren durch Verdrängungsexperimente mit [$^3$H]-8-OH-DPAT. [Methode von S.J. Peroutka, J. Neurochem. 47, 529 (1986), modifiziert nach H. Gozlan et al., J. Receptor Research 7, 195 (1987)].

Membranpräparation:

Eine männliche Ratte (Stamm Chbb:Thom, ca. 200 g) wurde dekapitiert. Das Gehirn wurde entnommen und in eiskaltem Puffer (50 mMol/l Tris-HCl plus Salzsäure ad pH = 7,4 bei Raumtemperatur) gegeben. Der frontale Cortex wurde herauspräpariert; sein Feuchtgewicht wurde bestimmt. Es wurde im 40-fachen Volumen an Puffer homogenisiert (Polytron, Stellung 6, 10 Sekunden), dann 20 Minuten bei 45.000 x g in der Kühlzentrifuge zentrifugiert. Das Pellet wurde im 100-fachen Volumen Puffer gewaschen und erneut wie oben zentrifugiert. Das erhaltene Pellet wurde im 40-fachen Volumen Puffer resuspendiert und 10 Minuten bei 37°C vorinkubiert. Es wurde das 60-fache Volumen Puffer zugegeben und wie oben zentrifugiert. Das

erhaltene Pellet wurde mit dem 100-fachen Volumen Puffer gewaschen und wie oben zentrifugiert. Das Pellet wurde in Puffer (0,8 ml/10 mg resuspendiert und kurz polytroniert. Dieses fertige Gewebehomogenat wurde bis zur Inkubation in Eis gekühlt.

Bindungsassay:

0,8 ml Gewebehomogenat (= 10 mg Feuchtgewicht) wurden zusammen mit 0,1 ml einer Lösung von [$^3$H]-8-OH-DPAT (ca. 0,1 nMol/l Endkonzentration) sowie mit 0,1 ml einer Lösung der Testsubstanz (steigende Konzentration: $10^{-10}$ bis $10^{-4}$ Mol) 30 Minuten bei Raumtemperatur inkubiert (3-fach-Bestimmung). Die Inkubation wurde durch schnelles Filtrieren über Whatman GF/B-Filter gestoppt; es wurde 2mal mit je 5 ml eiskaltem Puffer gespült (Filter Prep, Firma Ismatec), Die Radioaktivität der Filter wurde durch Flüssigszintillationsmessung bestimmt.
Die unspezifische Bindung wurde in Gegenwart von $10^{-4}$ Mol Serotonin ermittelt.

Datenanalyse:

Die spezifische Bindung ergibt sich aus der Gesamtbindung abzüglich der unspezifischen Bindung. Die Mittelwerte der 3-fach-Bestimmungen werden in ein Koordinatenkreuz eingezeichnet (Abszisse (log.): Testsubstanzkonzentration (Mol/l), Ordinate (lin.): Radioaktivität der Proben (x dpm). - Der $IC_{50}$-Wert ist die Konzentration, die die spezifische Bindung von [$^3$H]-8-OH-DPAT [= [$^3$H]-8-Hydroxy-2-(di-n-propylamino)-tetralin] um 50 % hemmt.

**4. Bestimmung der Affinität zu Serotonin-2(5-HT2)-Rezeptoren durch Verdrängungsexperimente mit [$^3$H]-Spiperon [Modifizierte Methode von S.J. Peroutka et al., Mol. Pharmacol 16, 700 (1979)].**

Membranpräparation:

Eine männliche Ratte (Stamm Chbb:Thom, ca. 200 g) wurde dekapitiert. Das Gehirn wurde entnommen. Der frontale Cortex wurde herauspräpariert und in eiskalte 0,32 M Saccharose-Lösung gegeben; sein Feuchtgewicht wurde bestimmt. Es wurde im 10-fachen Volumen (0,32 M) Saccharose-Lösung 1 Minute bei 800 Upm in einem Potter S (Firma Braun, Melsungen) homogenisiert. Das Homogenat wurde 10 Minuten bei 1000 x g (= 3.000 Upm bei Rotor 8 x 38 ml) in einer Kühlzentrifuge zentrifugiert. Der Überstand wurde dekantiert und homogenisiert (Polytron, Stellung 5, 1 Minute); das Sediment wurde verworfen.

5 ml (= 0,5 g Feuchtgewicht) der so erhaltenen Gewebesuspension wurden mit Puffer (50 mM Tris-HCl; pH = 7,7 bei Raumtemperatur) auf 40 ml aufgefüllt.

Bindungsassay:

0,8 ml des gepufferten Gewebehomogenats (= 10 mg Feuchtgewicht) wurden zusammen mit 0,1 ml einer Lösung von [$^3$H]-Spiperon (ca. 0,2 nMol/l Endkonzentration) sowie mit 0,1 ml einer Lösung der Testsubstanz (steigende Konzentration: $10^{-10}$ bis $10^{-4}$ Mol) 15 Minuten bei 37 °C inkubiert (3-fach-Bestimmung). Die Inkubation wurde durch schnelles Filtrieren über Whatman GF/B-Filter gestoppt; es wurde 3mal mit je 5 ml eiskaltem Puffer innerhalb maximal 10 Sekunden gespült (Filter Prep Firma Ismatec). Die Radioaktivität der Filter wurde durch Flüssigszintillationsmessung bestimmt. Die unspezifische Bindung wurde in Gegenwart von $10^{-4}$ Mol/l Ketanserin ermittelt.

Datenanalyse:

Die spezifische Bindung ergibt sich aus der Gesamtbindung abzüglich der unspezifischen Bindung. Die Mittelwerte der 3-fach-Bestimmungen werden in ein Koordinatenkreuz wie bei 3 eingetragen. - Der $IC_{50}$-

Wert ist die Konzentration, die die spezifische Bindung von [$^3$H]-Spiperon um 50 % hemmt.

In der nachfolgenden Tabelle 2 sind die 5-HT1A-Ki-Werte und die 5-HT2-Ki-Werte von erfindungsgemäßen Verbindungen zusammengestellt:

Tabelle 2

| Verbindung (Beispiel Nr.) | 5-HT1A-Ki [nM] | 5-HT2-Ki [nM] |
|---|---|---|
| 1 | 4.340 | 356 |
| 4d | 14.160 | 1.800 |
| 4t | 350 | 26 |
| 4u | 14.000 | 350 |
| 4w | 36.280 | 1.030 |
| 4y | 23.000 | 470 |
| 7a | 1.100 | 120 |
| 11p | 1.770 | 558 |
| 12b | 53.000 | 860 |
| 14b | 7.800 | 15.000 |
| 17 | 1.060 | 339 |
| 24a | 3.500 | 560 |
| Verb. A | 4.690 | 20.172 |
| Verb. B | nicht geprüft | 18.430 |
| 8-OH-DPAT | 1,2 | 2.876 |
| Ketanserin | 2.830 | 7,7 |
| Serotonin | 1,1 | 2.833 |

5. Motilitätsauslösung an der 24 Stunden zuvor mit Reserpin behandelten Maus [Methode: D. Hinzen et al., Europ. J. Pharmacol. 131, 75 (1986)]

In diesem Test werden vorrangig agonistische Wirkungen am hypersensitiven Dopamin-Rezeptor erfaßt.

Versuchsbeschreibung:

Männliche Mäuse werden 24 Stunden vor dem Experiment mit 5 mg/kg i.p. Reserpin behandelt. Die Tiere werden bei 25-30 °C gehalten und dreimal mit je 2 ml einer 5%igen Traubenzucker-Lösung in Tyrode s.c. behandelt (1. zum Zeitpunkt der Reserpingabe, 2. am Abend des Vorbehandlungstages, 3. am Morgen des Versuchstages).

Gruppen zu je 6 Tieren erhalten die Testsubstanz mit 5 mg/kg s.c. injiziert. (Das Injektionsvolumen beträgt generell 0,1 ml/ 10 g KG). 30 Minuten später werden die Tiergruppen zur Aktivitätsmessung in die Beobachtungskäfige (Maße: 42 x 24 x 8 cm) gesetzt, die mit einer Infrarot-Lichtschranke ausgerüstet sind. Meßwert ist die Häufigkeit des Passierens des Infrarotstrahles durch eine Gruppe von 6 Mäusen innerhalb 5 Minuten ("Laufimpulse/5 Min."; mean bei n = 3; mean ± s.e.m. bei n = 6).

Pro Substanz werden 3 bis 6 Gruppen geprüft. Kontrolltiere erhalten isotone Kochsalzlösung s.c.; sie zeigen minimale Aktivität (< 5 Laufimpulse/5 Min.). Die Standardsubstanz A ergibt bei 3 mg/kg s.c. 50 Laufimpulse/5 Minuten. Die Substanz B ist schwächer wirksam als Verbindung A; sie bewirkt bei 5 mg/kg s.c. 22 Laufimpulse/5 Minuten. Werden komplette Dosis-Wirkungs-Kurven erstellt, so wird die DLi50 als diejenige Dosis abgelesen, die zu 50 Laufimpulsen/5 Minuten Anlaß gibt.

In der nachstehenden Tabelle 3 werden die DLi50-Werte von erfindungsgemäßen Verbindungen mitgeteilt.

Tabelle 3

| Verbindung (Beispiel Nr.) | DLi50 [mg/kg s.c.] |
|---|---|
| 4a | 1,85 |
| 8 | 0,34 |
| 11a | 0,35 |
| 11b | 0,29 |
| Verb. A | 3,00 |

6. Bestimmung der postsynaptischen dopaminergenen Wirkung am MPTP-Affen [Modifizierte Methode von R.S. Burns et al., Proc. Natl. Acad. Sci. 80,.4546 (1983)]

Versuchsbeschreibung:

Durch das Neurotoxin 1-Methyl-4-phenyl-1,2,3,6-tetrahydropyridin (MPTP) wird beim Menschen und beim Affen ein irreversibles Krankheitsbild ausgelöst, das in seiner klinischen, pathologischen, biochemischen und pharmakologischen Ausprägung weitgehend der idiopathischen Parkinson'schen Erkrankung ähnelt [Markey et al., Nature 311, 464 (1984)]. Ursache dieser Ähnlichkeit ist, daß durch MPTP selektiv jene dopaminergen Nervenzellen in der Substantia nigra des Gehirns zerstört werden, die auch bei der Parkinson'schen Erkrankung durch degenerative Prozesse zugrunde gehen. Es wird sogar diskutiert, daß MPTP oder eine MPTP-ähnliche Substanz im Organismus entstehen und die Parkinson'sche Erkrankung auslösen könnte [S.H. Snyder, Nature 311, 514 (1984)]. Möglicherweise bedingt durch den spezifischen MPTP-Metabolismus, ist die klinische Ausbildung des MPTP-induzierten Parkinsonbildes bisher außer am Menschen nur am Affen nachweisbar.

Das am Rhesusaffen realisierte MPTP-Modell ist daher hervorragend geeignet, die Wirkung von postsynaptisch angreifenden Dopamin-Agonisten zu prüfen. Dazu erhielten Rhesusaffen MPTP in Gesamtdosen bis etwa 6 mg/kg Körpergewicht, bis folgende Symptome auftraten: Die Tiere waren akinetisch und nicht in der Lage, Wasser und Futter aufzunehmen. Sie zeigten eine typische, gebeugte Haltung; gelegentlich traten kataleptische Zustände auf. Die Extremitäten wiesen einen Rigor auf, welcher bei passiver Bewegung von klonischen Krämpfen durchbrochen wurde. Dopamin-Agonisten, wie B-HT 920 (= Verbindung A), Levodopa oder Apomorphin führen zu einer vorübergehenden Aufhebung dieses Zustandsbildes.

In der nachfolgenden Tabelle 4 werden von erfindungsgemäßen Verbindungen die Mindest-Dosen (MED) zur Aufhebung der Parkinson-Symptomatik, die Wirkungsdauer sowie beobachtete Nebenwirkungen aufgeführt. Es ist ersichtlich, daß bei den erfindungsgemäßen Verbindungen bei einem Mehrfachen der MED keine Sedation oder Ataxie auftritt, was auf das Fehlen einer entsprechenden durch $\alpha$2-Rezeptoren vermittelten Wirkung zurückgeführt werden kann.

Tabelle 4

| Verbindung (Beispiel Nr.) | Dosis [mg/kg i.m.] | Wirkungsdauer [Stunden] | Nebenwirkungen |
|---|---|---|---|
| 4p | 0,05 (MED) | ~ 2 | keine |
| 4p | 0,30 | 2,0-5 | keine |
| 11a | 0,05 (MED) | 1,5-2 | keine |
| 11a | 3,00 | 5 | leichte Unruhe |
| 11i | 0,05 (MED) | ~ 1,5 | keine |
| 11i | 0,20 | 2,0-5 | keine |
| 14b | 0,05 (MED) | 2,0-5 | keine |
| 14b | 0,50 | >5 | leichte Unruhe |
| Verb. A | 0,03 (MED) | 0,5-1 | keine |
| Verb. A | 0,15 | 5 | Sedation, Ataxie |

7. Bestimmung der Affininät zu Dopamin $D_1$-Rezeptoren durch Verdrängungsexperimente mit [$^3$H]-Sch 23390

Die Membranpräparation und die Datenanalyse erfolgten wie dies bei der Bestimmung der Affininät zu Dopamin $D_2$-Rezeptoren beschrieben wurde.

Bindungsassay:

Aliquote von 1 ml der Membranpräparation wurden mit 1 ml einer Lösung von 0.25 nM [$^3$H]-Sch 23390 (2.44 TBq/mMol, Fa. DuPont NEN) und steigender Konzentration der Testsubstanz ($10^{-11}$ bis $10^{-4}$ M) bei Raumtemperatur für eine Stunde inkubiert. Die Inkubation wurde durch Zugabe von 5 ml eiskaltem Tris-Puffer und Filtration durch Whatman GF/B-Filter beendet. Die Filter wurden zweimal mit je 5 ml eiskaltem Puffer gewaschen. Die Radioaktivität der Filter wurde durch Flüssigszintillationsmessung in Instagel (R) (Fa. Canberra Packard) bestimmt.

Die unspezifische Bindung wurde in Gegenwart von $10^{-6}$ M (-)-Butaclamol (Research Biochemicals Inc.) ermittelt.

In der nachstehenden Tabelle 5 werden von erfindungsgemäßen Verbindungen die $D_1$-Ki-Werte aufgeführt:

Tabelle 5

| Verbindung (Beispiel Nr.) | $D_1$-Ki [nM] |
|---|---|
| 1b | 800 |
| 4t | 210 |
| 4x | 400 |
| 4y | 1.500 |
| 7a | 180 |
| 8k | 510 |
| 11p | 920 |
| 14b | 5.100 |
| 24a | 3.000 |
| Verb. A | 12.000 |
| Verb. B | 11.000 |
| Verb. C* | 11.000 |

* siehe Seite 30 (Tabelle 1)

Bei den untersuchten erfindungsgemäßen Verbindungen traten in den bisher bei den Untersuchungen angewandten Dosierungen keine toxischen Nebenwirkungen auf.

Die erfindungsgemäßen Verbindungen der allgemeinen Formel II und deren physiologisch verträglichen Säureadditionssalze mit anorganischen oder organischen Säuren lassen sich zur pharmazeutischen Anwendung, gegebenenfalls in Kombination mit anderen Wirksubstanzen, in die üblichen galenischen Zubereitungsformen wie Tabletten, Dragées, Kapseln, Pulver, Suppositorien, Lösungen, Emulsionen oder Suspensionen einarbeiten. Die Einzeldosis für Erwachsene beträgt bei oraler oder parenteraler Applikation 1 bis 150 mg, vorzugsweise, 2,5 bis 50 mg, 1 bis 3 mal täglich.

Die nachfolgenden Beispiele sollen die Erfindung näher erläutern:
Herstellung der Ausgangsprodukte der allgemeinen Formel IV, in denen $R_2$ ein Wasserstoffatom oder die Acetylgruppe darstellt:

Beispiel (i)

a) 5-Brom-hexahydro-4H-4-azepinon-hydrobromid

Zur Lösung von 130 g (0,87 Mol) Hexahydro-4H-azepin-4-on-hydrochlorid (Schmelzpunkt: 177-178 °C) in 975 ml Eisessig gibt man bei Raumtemperatur unter Rühren 260 ml einer 33%igen Bromwasserstoff/Eisessig-Lösung. Dazu tropft man unter Rühren bei Raumtemperatur binnen 1,5 Stunden die Lösung von 44,6 ml = 139 g (0,87 Mol) Brom in 260 ml Eisessig und rührt dann noch 1,5 Stunden bei Raumtemperatur nach. Man dampft im Vakuum zur Trockne ein, löst den Eindampfrückstand in ca. 1 l Aceton, versetzt mit ca. 0,2 l Äthylacetat und läßt auskristallisieren. Man filtriert durch eine Glasfritte und wäscht den Filterkuchen mit ca. 0, 2 l eiskaltem Aceton. Man trocknet bei 80 °C im Umluft-Trockenschrank.
Ausbeute: 214 g (90 % der Theorie),
Schmelzpunkt: 140-145 °C.

b) 2-Amino-4,5,7,8-tetrahydro-6H-thiazolo[5,4-d]azepin-dihydrobromid

Zu 59,7 g (0.78 Mol) Thioharnstoff in 1,6 l wasserfreiem Äthanol gibt man unter Rühren bei Raumtemperatur 214 g (0,78 Mol) 5-Brom-hexahydro-4H-azepin-4-on-hydrobromid und erhitzt dann 3,5 Stunden unter Rückfluß. Man läßt das Reaktionsgemisch über Nacht eisgekühlt stehen und filtriert durch eine Glasfritte ab. Man wäscht den Filterkuchen mit ca. 0,2 l eiskaltem Äthanol und mit ca. 0,2 l Äther und trocknet ihn bei 80 °C über Calciumchlorid im Umlufttrockenschrank.
Ausbeute: 194 g (75 % der Theorie),
Schmelzpunkt: 270-280 °C (Zers.).

c) 2-Amino-4,5,7,8-tetrahydro-6H-thiazolo[5,4-d]azepin

Man rührt die Lösung von 194 g (0,586 Mol) des unter (ib) beschriebenen Dihydrobromides in 200 ml Wasser. Dazu gibt man 240 ml 6N-Kalilauge und unmittelbar darauf 2 l Chloroform. Man rührt kräftig eine Stunde lang (nach 15 Minuten ist die Chloroformlösung dunkelrot gefärbt), trennt die Phasen und wiederholt die Extraktion der wäßrigen alkalischen Phase noch zweimal mit je 1 l Chloroform je eine Stunde lang. Die vereinigten Chloroform-Extrakte trocknet man über Natriumsulfat, filtriert durch eine mit Natriumsulfat bedeckte Glasfritte und dampft das Filtrat im Vakuum ein. Den halbkristallinen Eindampfrückstand verreibt man mit ca. 300 ml Äther. Man filtriert ab und trocknet den Filterkuchen bei 80 °C im Umluft-Trockenschrank.
Ausbeute: 75 g (76 % der Theorie),
Schmelzpunkt: 150-160 °C

| | | | | | | |
|---|---|---|---|---|---|---|
| Ber.:<br>Gef.: | C | 49,70<br>49,80 | H | 6,55<br>6,50 | N | 24,84<br>24,76 |

Beispiel (ii)

a) 2-Acetylamino-6-benzyl-4,5,7,8-tetrahydro-6H-thiazolo[5,4-d]azepin

Hergestellt aus 2-Amino-6-benzyl-4,5,7,8-tetrahydro-6H-thiazolo[5,4-d)azepin [siehe Beispiel 4 der GB-A-1.321.509, Schmelzpunkt des Dihydrochlorides: 232 °C] und 1,2 Äquivalenten Acetanhydrid während 2

Stunden bei Rückfluß.
Ausbeute: 62 % der Theorie;
Schmelzpunkt: 129-130 ° C.

b) 2-Acetylamino-6-benzyloxycarbonyl-4,5,7,8-tetrahydro-6H-thiazolo[5,4-d]azepin

Hergestellt aus der vorstehend beschriebenen Verbindung durch Lösen in Methylenchlorid, Zugabe von 0,3 Äquivalenten Äthyldiisopropylamin, Abkühlen auf 0 ° C und Zutropfen einer Lösung von 1,1 Äquivalenten Chlorameisensäurebenzylester in Methylenchlorid bei 0 ° C, Rühren über Nacht bei Raumtemperatur, Zugabe weiterer 0,55 Äquivalente Chlorameisensäurebenzylester und 2-stündiges Weiterrühren, Ausschütteln mit Wasser sowie Reinigen des Eindampfrückstandes der getrockneten und filtrierten organischen Phase durch Säulenchromatographie an Kieselgel (Toluol/Äthylacetat/Methanol = 6:3:0,5).
Ausbeute: 52 % der Theorie,
Schmelzpunkt: 126-128 ° C.

c) 2-Acetylamino-4,5,7,8-tetrahydro-6H-thiazolo[5,4-d]azepin-dihydrobromid

Hergestellt durch portionsweises Eintragen der vorstehend beschriebenen Verbindung in 4 Äquivalente einer 33%igen Bromwasserstoff/Eisessig-Lösung, Rühren bei Raumtemperatur während einer Stunde, Zugabe von Äthylacetat, Filtration, Waschen des Filterkuchens mit Äthylacetat und Äther sowie Trocknen bei 80 ° C/20 Torr.
Ausbeute: 96 % der Theorie,
Schmelzpunkt: 237-242 ° C.
(Durch Lösen in gesättigter Kaliumcarbonat-Lösung, Extraktion mit Chloroform und Kristallisation aus Aceton/Äther ist die freie Base erhältlich. Schmelzpunkt: 154-156 ° C).

Herstellung der Endprodukte der allgemeinen Formel II:

Beispiel 1

2-Amino-6-cinnamyl-4,5,7,8-tetrahydro-6H-thiazolo[5,4-d]azepin

Zum gerührten Gemisch von 2,50 g (14,8 mMol) 2-Amino-4,5,7,8-tetrahydro-6H-thiazolo[5,4-d]azepin und 2,10 g (15,2 mMol) Kaliumcarbonat in 25 ml wasserfreiem Dimethylformamid gibt man die Lösung von 2,3 g (15,1 mMol) Cinnamylchlorid in 10 ml wasserfreiem Dimethylformamid. Nach 2-stündigem Erhitzen im Bad von 80 ° C dampft man im Vakuum ein und verteilt den Eindampfrückstand zwischen Wasser und Chloroform. Aus dem über Natriumsulfat getrockneten und filtrierten Chloroform-Extrakt erhält man durch Eindampfen im Vakuum 6 g rotbraunes Öl, das durch Säulenchromatographie an Kieselgel (Chloroform/Methanol = 5:1) gereinigt wird.
Ausbeute: 1,90 g (45 % der Theorie),
Schmelzpunkt: 122-125 ° C (Äther)

| | | | | | | |
|---|---|---|---|---|---|---|
| Ber.:<br>Gef.: | C | 67,35<br>67,45 | H | 6,71<br>6,75 | N | 14,73<br>14,89 |

Zur Überführung ins Hydrochlorid gibt man zur Lösung von 1,88 g (6,6 mMol) der vorstehenden Base in 30 ml Methanol 6,6 ml 1N-Salzsäure und dampft im Vakuum zur Trockne ein. Den erhaltenen Schaum trocknet man im Vakuum über Phosphorpentoxid, beginnend bei 60 ° C und zuletzt 2 Stunden bei 100 ° C. Man erhält 1,80 g 2-Amino-6-cinnamyl-4,5,7,8-tetrahydro-6H-thiazolo[5,4-d]azepin-hydrochlorid-Hydrat vom Schmelzpunkt 120-125 ° C.

| | | | | | | | | |
|---|---|---|---|---|---|---|---|---|
| Ber.:<br>Gef.: | C | 58,09<br>58,22 | H | 6,70<br>6,60 | Cl | 10,72<br>11,09 | N | 12,70<br>12,66 |

Analog Beispeil 1 wurden folgende Verbindungen hergestellt:

1a) 2-Amino-6-(1H-inden-2-yl-methyl)-4,5,7,8-tetrahydro-6H-thiazolo[5,4-d]azepin

Hergestellt aus 2-Amino-4,5,7,8-tetrahydro-6H-thiazolo[5,4-d]azepin, Kaliumcarbonat und 2-Chlormethyl-1H-inden [hergestellt aus 1H-Inden, Paraformaldehyd und konzentrierter Salzsäure] in wasserfreiem Dimethylformamid während 2 Stunden bei 50 °C.
Ausbeute: 4 % der Theorie,
Schmelzpunkt: 134-138 °C

| | | | | | | |
|---|---|---|---|---|---|---|
| Ber.:<br>Gef.: | C | 68,67<br>68,86 | H | 6,44<br>6,40 | N | 14,13<br>13,97 |

1b) 2-Amino-6-(1,2-dihydronaphthalin-3-yl-methyl)-4,5,7,8-tetrahydro-6H-thiazolo[5,4-d]azepin

Hergestellt aus 2-Amino-4,5,7,8-tetrahydro-6H-thiazolo[5,4-d]azepin, Kaliumcarbonat und 3-Chlormethyl-1,2-dihydronaphthalin [hergestellt aus 1,2-Dihydronaphthalin, Paraformaldehyd und konzentrierter Salzsäure] in wasserfreiem Dimethylformamid während 2 Stunden bei 50 °C.
Ausbeute: 29 % der Theorie,
Schmelzpunkt: 158-160 °C (Äthylacetat)

| | | | | | | |
|---|---|---|---|---|---|---|
| Ber.:<br>Gef.: | C | 69,43<br>69,26 | H | 6,80<br>6,86 | N | 13,49<br>13,26 |

Beispiel 2

2-Acetylamino-6-cinnamyl-4,5,7,8-tetrahydro-6H-thiazolo[5,4-d]azepin

Man rührt das Gemisch aus 2,60 g (7 mMol) 2-Acetylamino-4,5,7,8-tetrahydro-6H-thiazolo[5,4-d]azepin-dihydrobromid und 2,13 g (15,4 mMol) Kaliumcarbonat in 30 ml wasserfreiem Dimethylformamid 30 Minuten bei 80 °C, kühlt auf Raumtemperatur ab, versetzt mit 1,07 g (7 mMol) Cinnamylchlorid und erhitzt 2 Stunden bei 80 °C. Danach dampft man im Vakuum ein und verteilt den Eindampfrückstand zwischen Wasser und Chloroform. Die getrocknete und filtrierte Chloroform-Lösung wird im Vakuum eingedampft. Den Eindampfrückstand reinigt man durch Säulenchromatographie an Kieselgel (Chloroform/Methanol = 25:1).
Ausbeute: 1,52 g (66 % der Theorie),
Schmelzpunkt: 133-135 °C (Äther)

| | | | | | | |
|---|---|---|---|---|---|---|
| Ber.:<br>Gef.: | C | 66,04<br>65,90 | H | 6,47<br>6,43 | N | 12,84<br>12,95 |

Analog Beispiel 2 wurden folgende Verbindungen hergestellt:

2a) 2-Acetylamino-6-(3-(4-chlor-phenyl)allyl)-4,5,7,8-tetrahydro-6H-thiazolo[5,4-d]azepin x 0,25 $H_2O$

Hergestellt aus 2-Acetylamino-4,5,7,8-tetrahydro-6H-thiazolo[5,4-d]azepin-dihydrobromid, Kaliumcarbonat und 4-Chlor-cinnamylchlorid in wasserfreiem Dimethylformamid.
Ausbeute: 68 % der Theorie,
Schmelzpunkt: 190-195 °C (Äther)

| | | | | | | | |
|---|---|---|---|---|---|---|---|
| Ber.:<br>Gef.: | (x 0,25 $H_2O$) | C | 58,96<br>59,15 | H | 5,70<br>5,54 | N | 11,46<br>11,35 |

2b) 2-Acetylamino-6-(3-phenyl-propyl)-4,5,7,8-tetrahydro-6H-thiazolo[5,4-d]azepin-hydrochlorid

Hergestellt aus 2-Acetylamino-4,5,7,8-tetrahydro-6H-thiazolo[5,4-d]azepin-dihydrobromid, Kaliumcarbonat und 3-Phenyl-n-propylbromid in wasserfreiem Dimethylformamid. - Die erhaltene Base überführt man in Äthanol mittels ätherischer Salzsäure ins Hydrochlorid.
Ausbeute: 49 % der Theorie,
Schmelzpunkt: 260-262 °C (Zers.)

| | | | | | | | | |
|---|---|---|---|---|---|---|---|---|
| Ber.:<br>Gef.: | C | 59,08<br>58,97 | H | 6,61<br>6,81 | N | 11,48<br>11,35 | Cl | 9,68<br>9,87 |

Beispiel 3

2-Amino-6-cinnamyl-4,5,7,8-tetrahydro-6H-thiazolo[5,4-d]azepin

Man erhitzt unter Rühren 1,2 g (3,7 mMol) 2-Acetylamino-6-cinnamyl-4,5,7,8-tetrahydro-6H-thiazolo[5,4-d]azepin zusammen mit 24 ml halbkonzentrierter Salzsäure drei Stunden bei 90 °C. Anschließend entfernt man den größten Teil der Salzsäure im Vakuum, stellt ammoniakalisch und extrahiert mit Chloroform. Den getrockneten und filtrierten organischen Extrakt dampft man im Vakuum ein; den Eindampfrückstand reinigt man durch Säulenchromatographie an Kieselgel (Chloroform/Methanol = 5:1).
Ausbeute: 0,6 g (60 % der Theorie),
Schmelzpunkt: 121-124 °C (Äther)

| | | | | | | |
|---|---|---|---|---|---|---|
| Ber.:<br>Gef.: | C | 67,35<br>67,37 | H | 6,71<br>6,79 | N | 14,73<br>14,92 |

Analog Beispiel 3 wurden folgende Verbindungen hergestellt:

3a) 2-Amino-6-(3-(4-chlor-phenyl)allyl)-4,5,7,8-tetrahydro-6H-thiazolo[5,4-d]azepin

Hergestellt aus 2-Acetylamino-6-(3-(4-chlor-phenyl)allyl)-4,5,7,8-tetrahydro-6H-thiazolo[5,4-d]azepin mit halbkonzentrierter Salzsäure.
Ausbeute: 71 % der Theorie,
Schmelzpunkt: 145-150 °C

| | | | | | | |
|---|---|---|---|---|---|---|
| Ber.:<br>Gef.: | C | 60,08<br>60,15 | H | 5,67<br>5,48 | N | 13,14<br>12,97 |

3b) 2-Amino-6-(3-phenyl-propyl)-4,5,7,8-tetrahydro-6H-thiazolo[5,4,-d]azepin-dihydrochlorid x 0,33 $H_2O$

Hergestellt aus 2-Acetylamino-6-(3-phenyl-propyl)-4,5,7,8-tetrahydro-6H-thiazolo[5,4-d]azepin-hydrochlorid mit halbkonzentrierter Salzsäure. Den durch Eindampfen zur Trockne erhaltenen Schaum kristallisiert man aus konzentrierter äthanolischer Lösung unter Zusatz von wenig Aceton.
Ausbeute: 66 % der Theorie,
Schmelzpunkt: 221-225 °C

| | | | | | | | |
|---|---|---|---|---|---|---|---|
| Ber.:<br>Gef.: | (x 0,33 $H_2O$) | C | 52,47<br>52,68 | H | 6,42<br>6,47 | N | 11,47<br>11,74 |

Beispiel 4

2-Amino-6-(3-(2-thienyl)allyl)-4,5,7,8-tetrahydro-6H-thiazolo[5,4-d]azepin

Die Lösung von 1,0 g (6,2 mMol) 3-(2-Thienyl)allylchlorid [frisch bereitet aus 3-(2-Thienyl)allylalkohol in Chloroform durch Zutropfen eines Äquivalentes Thionylchlorid bei 0 °C und - nach 15 Minuten bei 0 °C - Eindampfen im Vakuum bei 25 °C] in 10 ml Chloroform tropft man bei Raumtemperatur zur Suspension von 1,0 g (5,9 mMol) 2-Amino-4,5,7,8-tetrahydro-6H-thiazolo[5,4-d]azepin und 0,86 g (6,2 mMol) Kaliumcarbonat in 40 ml Chloroform. Nach 1,5 Stunden Rühren gibt man 80 ml Chloroform zu und schüttelt zweimal mit Wasser aus. Die getrocknete und filtrierte Chloroform-Lösung dampft man im Vakuum ein. Den Eindampfrückstand reinigt man durch Säulenchromatographie an Kieselgel (Chloroform/Methanol = 1O:1.
Ausbeute: 0,46 g (26,7 % der Theorie),
Schmelzpunkt: 116-120 °C

| | | | | | | |
|---|---|---|---|---|---|---|
| Ber.:<br>Gef.: | C | 57,70<br>57,70 | H | 5,97<br>5,86 | N | 14,42<br>14,21 |

Analog Beispiel 4 wurden folgende Verbindungen hergestellt:

4a) 2-Amino-6-(3-(3-thienyl)allyl)-4,5,7,8-tetrahydro-6H-thiazolo[5,4-d]azepin

Hergestellt aus 3-(3-Thienyl)allylchlorid und 1 Äquivalent 2-Amino-4,5,7,8-tetrahydro-6H-thiazolo[5,4-d]-azepin in Chloroform in Gegenwart von 1 Äquivalent Kaliumcarbonat.
Ausbeute: 8 % der Theorie,
Schmelzpunkt: 128-132 °C (Isopropanol)

| | | | | | | |
|---|---|---|---|---|---|---|
| Ber.:<br>Gef.: | C | 57,70<br>57,93 | H | 5,88<br>5,89 | N | 14,42<br>14,28 |

4b) 2-Amino-6-(3-(3-furyl)allyl)-4,5,7,8-tetrahydro-6H-thiazolo[5,4-d]azepin

Hergestellt aus 3-(3-Furyl)allylchlorid und 2 Äquivalenten 2-Amino-4,5,7,8-tetrahydro-6H-thiazolo[5,4-d]-azepin in Chloroform.
Ausbeute: 30 % der Theorie,
Schmelzpunkt: 130-136 °C

| | | | | | | |
|---|---|---|---|---|---|---|
| Ber.:<br>Gef.: | C | 61,06<br>61,18 | H | 6,22<br>6,21 | N | 15,26<br>14,97 |

4c) 2-Amino-6-(3-(3,5-dichlor-4-hydroxy-phenyl)allyl)-4,5,7,8-tetrahydro-6H-thiazolo[5,4-d]azepin x 0,5 $H_2O$

Hergestellt aus 3,5-Dichlor-4-hydroxy-cinnamylchlorid und 2 Äquivalenten 2-Amino-4,5,7,8-tetrahydro-6H-thiazolo[5,4-d]azepin in Chloroform während 12 Stunden bei Raumtemperatur.
Ausbeute: 9 % der Theorie,
Schmelzpunkt: 197 °C

| | | | | | | | |
|---|---|---|---|---|---|---|---|
| Ber.:<br>Gef.: | (x 0,5 $H_2O$) | C | 50,66<br>50,49 | H | 4,78<br>5,06 | N | 11,08<br>10,98 |

4d) 2-Amino-6-(3-phenyl-2-propin-1-yl)-4,5,7,8-tetrahydro-6H-thiazolo[5,4-d]azepin x 0,5 $H_2O$

Hergestellt aus 3-Phenyl-propargylchlorid und 2 Äquivalenten 2-Amino-4,5,7,8-tetrahydro-6Hthiazolo-[5,4-d]azepin in Chloroform.
Ausbeute: 16 % der Theorie,
Schmelzpunkt: 142-146 °C (Äther)

| | | | | | | | |
|---|---|---|---|---|---|---|---|
| Ber.:<br>Gef.: | (x 0,5 $H_2O$) | C | 65,74<br>65,56 | H | 6,20<br>6,01 | N | 14,38<br>14,42 |

4e) 2-Amino-6-(3-2-chlor-phenyl)allyl)-4,5,7,8-tetrahydro-6H-thiazolo[5,4-d]azepin

Hergestellt aus 2-Chlor-cinnamylchlorid und 1 Äquivalent 2-Amino-4,5,7,8-tetrahydro-6H-thiazolo[5,4-d]-azepin in Chloroform in Gegenwart von 1 Äquivalent Kaliumcarbonat.
Ausbeute: 20 % der Theorie,
Schmelzpunkt: 80 °C

| | | | | | | |
|---|---|---|---|---|---|---|
| Ber.:<br>Gef.: | C | 60,08<br>60,20 | H | 5,67<br>5,61 | N | 13,14<br>13,12 |

Aus der Base erhält man durch Lösen in Äthanol, Zugabe von überschüssiger ätherischer Salzsäure und von Äther das 2-Amino-6-(3-(2-chlor-phenyl)allyl))-4,5,7,8-tetrahydro-6H-thiazolo[5,4-d]azepin-dihydrochlorid vom Schmelzpunkt 236-240 °C.

| | | | | | | |
|---|---|---|---|---|---|---|
| Ber.:<br>Gef.: | C | 48,93<br>49,03 | H | 5,18<br>5,31 | N | 10,70<br>10,55 |

4f) 2-Amino-6-(3-(3-chlor-phenyl)allyl)-4,5,7,8-tetrahydro-6H-thiazolo[5,4-d]azepin

Hergestellt aus 3-Chlor-cinnamylchlorid und 1 Äquivalent 2-Amino-4,5,7,8-tetrahydro-6H-thiazolo[5,4-d]-azepin in Chloroform in Gegenwart von 1 Äquivalent Kaliumcarbonat.
Ausbeute: 20 % der Theorie,
Schmelzpunkt: 132-136 °C

| | | | | | | |
|---|---|---|---|---|---|---|
| Ber.:<br>Gef.: | C | 60,08<br>60,20 | H | 5,67<br>5,60 | N | 13,14<br>13,26 |

4g) 2-Amino-6-(3-(2-nitro-phenyl)allyl)-4,5,7,8-tetrahydro-6H-thiazolo[5,4-d]azepin

Hergestellt aus 2-Nitro-cinnamylchlorid und 2 Äquivalenten 2-Amino-4,5,7,8-tetrahydro-6H-thiazolo[5,4-d]azepin in Chloroform.
Ausbeute: 57 % der Theorie,
Schmelzpunkt: 125-128 °C (Äther)

| | | | | | | |
|---|---|---|---|---|---|---|
| Ber.:<br>Gef.: | C | 58,17<br>57,99 | H | 5,49<br>5,70 | N | 16,96<br>16,73 |

4h) 2-Amino-6-(3-(3-nitro-phenyl)allyl)-4,5,7,8-tetrahydro-6H-thiazolo[5,4-d]azepin

Hergestellt aus 3-Nitro-cinnamylchlorid und 2 Äquivalenten 2-Amino-4,5,7,8-tetrahydro-6H-thiazolo[5,4-d]azepin in Chloroform.
Ausbeute: 56 % der Theorie,
Schmelzpunkt: 165-168 ° C (Äther)

| | | | | | | |
|---|---|---|---|---|---|---|
| Ber.:<br>Gef.: | C | 58,17<br>57,97 | H | 5,49<br>5,34 | N | 16,96<br>16,89 |

4i) 2-Amino-6-(3-(4-nitro-phenyl)allyl)-4,5,7,8-tetrahydro-6H-thiazolo[5,4-d]azepin x 0,5 $H_2O$

Hergestellt aus 4-Nitro-cinnamylchlorid und 2 Äquivalenten 2-Amino-4,5,7,8-tetrahydro-6H-thiazolo[5,4-d]azepin in Chloroform.
Ausbeute: 38 % der Theorie,
Schmelzpunkt: 186-191 ° C (Äther)

| | | | | | | | |
|---|---|---|---|---|---|---|---|
| Ber.:<br>Gef.: | (x 0,5 $H_2O$) | C | 56,62<br>56,40 | H | 5,64<br>5,71 | N | 16,51<br>16,63 |

4k) 2-Amino-6-(3-(4-methoxy-phenyl)allyl)-4,5,7,8-tetrahydro-6H-thiazolo[5,4-d]azepin

Hergestellt aus 4-Methoxy-cinnamylchlorid und 1 Äquivalent 2-Amino-4,5,7,8-tetrahydro-6H-thiazolo[5,4-d]azepin in Gegenwart von 1 Äquivalent Kaliumcarbonat in Chloroform.
Ausbeute: 10 % der Theorie,
Schmelzpunkt: 155-160 ° C (Äther)

| | | | | | | |
|---|---|---|---|---|---|---|
| Ber.:<br>Gef.: | C | 64,73<br>64,58 | H | 6,70<br>6,55 | N | 13,32<br>13,16 |

4ℓ) 2-Amino-6-(3-(2-methyl-phenyl)allyl)-4,5,7,8-tetrahydro-6H-thiazolo[5,4-d]azepin

Hergestellt aus 2-Methyl-cinnamylchlorid und 2 Äquivalenten 2-Amino-4,5,7,8-tetrahydro-6H-thiazolo-[5,4-d]azepin Chloroform.
Ausbeute: 52 % der Theorie,
Schmelzpunkt: 112-115 ° C (Äthylacetat)

| | | | | | | |
|---|---|---|---|---|---|---|
| Ber.:<br>Gef.: | C | 68,21<br>68,15 | H | 7,07<br>7,15 | N | 14,04<br>14,24 |

4m) 2-Amino-6-(3-methyl-phenyl)allyl)-4,5,7,8-tetrahydro-6H-thiazolo[5,4-d]azepin

Hergestellt aus 3-Methyl-cinnamylchlorid und 2 Äquivalenten 2-Amino-4,5,7,8-tetrahydro-6H-thiazolo-[5,4-d]azepin Chloroform.
Ausbeute: 47 % der Theorie,
Schmelzpunkt: 116-119 ° C

| | | | | | | |
|---|---|---|---|---|---|---|
| Ber.:<br>Gef.: | C | 68,21<br>68,14 | H | 7,07<br>7,25 | N | 14,04<br>14,34 |

4n) 2-Amino-6-(3-(4-methyl-phenyl)allyl)-4,5,7,8-tetrahydro-6H-thiazolo[5,4-d]azepin

Hergestellt aus 4-Methyl-cinnamylchlorid und 1 Äquivalent 2-Amino-4,5,7,8-tetrahydro-6H-thiazolo[5,4-d]azepin in Acetonitril in Gegenwart von 1 Äquivalent Kaliumcarbonat während 40 Minuten bei 80 °C.
Ausbeute: 33 % der Theorie,
Schmelzpunkt: 126-130 °C (Äther)

| | | | | | | |
|---|---|---|---|---|---|---|
| Ber.:<br>Gef.: | C | 68,21<br>68,36 | H | 7,07<br>7,17 | N | 14,04<br>14,02 |

4o) 2-Amino-6-(3-(2,3-dimethoxy-phenyl)allyl)-4,5,7,8-tetrahydro-6H-thiazolo[5,4-d]azepin

Hergestellt aus 2,3-Dimethoxy-cinnamylchlorid und 2 Äquivalenten 2-Amino-4,5,7,8-tetrahydro-6H-thiazolo[5,4-d]azepin in Chloroform.
Ausbeute: 39 % der Theorie,
Schmelzpunkt: 87-91 °C (Äther)

| | | | | | | |
|---|---|---|---|---|---|---|
| Ber.:<br>Gef.: | C | 62,58<br>62,70 | H | 6,71<br>6,89 | N | 12,16<br>12,19 |

4p) 2-Amino-6-(3-(2,5-dimethoxy-phenyl)allyl)-4,5,7,8-tetrahydro-6H-thiazolo[5,4-d]azepin

Hergestellt aus 2,5-Dimethoxy-cinnamylchlorid [hergestellt bei -5 °C, 10 Minuten] und 2 Äquivalenten 2-Amino-4,5,7,8-tetrahydro-6H-thiazolo[5,4-d]azepin in Chloroform.
Ausbeute: 4 % der Theorie,
Schmelzpunkt: 112-115 °C (Äther)

| | | | | | | |
|---|---|---|---|---|---|---|
| Ber.:<br>Gef.: | C | 62,59<br>62,46 | H | 6,71<br>6,67 | N | 12,17<br>11,94 |

Molpeak (m/z) Ber.: 345 Gef.: 345

4q) 2-Amino-6-(3-(3,4-dimethoxy-phenyl)allyl)-4,5,7,8-tetrahydro-6H-thiazolo[5,4-d]azepin

Hergestellt aus 3,4-Dimethoxy-cinnamylchlorid [hergestellt bei -5 °C, 10 Minuten] und 2 Äquivalenten 2-Amino-4,5,7,8-tetrahydro-6H-thiazolo[5,4-d]azepin in Chloroform.
Ausbeute: 7,7 % der Theorie,
Schmelzpunkt: 122-126 °C (Äther)

| | | | | | | |
|---|---|---|---|---|---|---|
| Ber.:<br>Gef.: | C | 62,58<br>62,70 | H | 6,71<br>6,84 | N | 12,16<br>11,90 |

4r) 2-Amino-6-(3-(3,5-dimethoxy-phenyl)allyl)-4,5,7,8-tetrahydro-6H-thiazolo[5,4-d]azepin

Hergestellt aus 3,5-Dimethoxy-cinnamylchlorid und 2 Äquivalenten 2-Amino-4,5,7,8-tetrahydro-6H-thiazolo[5,4-d]azepin in Chloroform.

Ausbeute: 21 % der Theorie,
Schmelzpunkt: 114-119 °C (Petroläther)

| | | | | | | |
|---|---|---|---|---|---|---|
| Ber.:<br>Gef.: | C | 62,58<br>62,57 | H | 6,71<br>6,57 | N | 12,16<br>11,95 |

4s) 2-Amino-6-(3-(4-dimethylamino-phenyl)allyl)-4,5,7,8-tetrahydro-6H-thiazolo[5,4-d]azepin

Hergestellt aus 4-Dimethylamino-cinnamylchlorid-hydrochlorid und 3 Äquivalenten 2-Amino-4,5,7,8-tetrahydro-6H-thiazolo[5,4-d]azepin in reinem Chloroform während einer Stunde bei 50 °C.
Ausbeute: 2,4 % der Theorie,
Schmelzpunkt: 85-90 °C
Molpeak (m/z) Ber.: 328 Gef.: 328

4t) 2-Amino-6-(3-(1-naphthyl)allyl)-4,5,7,8-tetrahydro-6H-thiazolo[5,4-d]azepin

Hergestellt aus 3-(1-Naphthyl)allyl)chlorid und 2 Äquivalenten 2-Amino-4,5,7,8-tetrahydro-6H-thiazolo-[5,4-d]azepin in Chloroform während 3 Tagen bei 20 °C.
Ausbeute: 31 % der Theorie,
Schmelzpunkt: 178-180 °C (Chloroform/Methanol = 100:1)

| | | | | | | |
|---|---|---|---|---|---|---|
| Ber.:<br>Gef.: | C | 71,62<br>71,33 | H | 6,31<br>6,28 | N | 12,53<br>12,32 |

4u) 2-Amino-6-(3-(2-naphthyl)allyl)-4,5,7,8-tetrahydro-6H-thiazolo[5,4-d]azepin

Hergestellt aus 3-(2-Naphthyl)allyl)chlorid und 2 Äquivalenten 2-Amino-4,5,7,8-tetrahydro-6H-thiazolo-[5,4-d]azepin in Chloroform während 3 Tagen bei 20 °C.
Ausbeute: 20 % der Theorie,
Schmelzpunkt: 164-165 °C (Chloroform)

| | | | | | | |
|---|---|---|---|---|---|---|
| Ber.:<br>Gef.: | C | 71,62<br>71,49 | H | 6,31<br>6,43 | N | 12,53<br>12,45 |

4v) 2-Amino-6-(3-(2-biphenyl)allyl)-4,5,7,8-tetrahydro-6H-thiazolo[5,4-d]azepin

Hergestellt aus 3-(2-Biphenyl)allyl)chlorid und 2 Äquivalenten 2-Amino-4,5,7,8-tetrahydro-6H-thiazolo-[5,4-d]azepin in Chloroform während 5 Stunden bei 50 °C.
Ausbeute: 57 % der Theorie,
Schmelzpunkt: 154-158 °C (Äther)

| | | | | | | |
|---|---|---|---|---|---|---|
| Ber.:<br>Gef.: | C | 73,11<br>73,00 | H | 6,41<br>6,44 | N | 11,63<br>11,48 |

4w) 2-Amino-6-(3-(4-biphenyl)allyl)-4,5,7,8-tetrahydro-6H-thiazolo[5,4-d]azepin x 0,25 $H_2O$

Hergestellt aus 3-(4-Biphenyl)allyl)-chlorid und 2 Äquivalenten 2-Amino-4,5,7,8-tetrahydro-6H-thiazolo-[5,4-d]azepin in Chloroform.
Ausbeute: 54 % der Theorie,
Schmelzpunkt: 178-180 °C (Äther)

| | | | | | | | |
|---|---|---|---|---|---|---|---|
| Ber.:<br>Gef.: | (x 0,25 $H_2O$) | C | 72,19<br>72,11 | H | 6,47<br>6,12 | N | 11,48<br>11,33 |

4x) 2-Amino-6-(3-(2-benzyloxy-phenyl)allyl)-4,5,7,8-tetrahydro-6H-thiazolo[5,4-d]azepin

Hergestellt aus 2-Benzyloxy-cinnamylchlorid und 2 Äquivalenten 2-Amino-4,5,7,8-tetrahydro-6H-thiazolo[5,4-d]azepin in Chloroform während 5 Stunden bei 50 °C.
Ausbeute: 36 % der Theorie,
Schmelzpunkt: 103-107 °C (Äther)

| | | | | | | |
|---|---|---|---|---|---|---|
| Ber.:<br>Gef.: | C | 70,57<br>70,42 | H | 6,44<br>6,63 | N | 10,73<br>11,01 |

4y) 2-Amino-6-(3-(3-benzyloxy-phenyl)allyl)-4,5,7,8-tetrahydro-6H-thiazolo[5,4-d]azepin

Hergestellt aus 3-Benzyloxy-cinnamylchlorid und 2 Äquivalenten 2-Amino-4,5,7,8-tetrahydro-6H-thiazolo[5,4-d]azepin in Chloroform während 5 Stunden bei 50 °C.
Ausbeute: 59 % der Theorie,
Schmelzpunkt: 78-80 °C (Äther)

| | | | | | | |
|---|---|---|---|---|---|---|
| Ber.:<br>Gef.: | C | 70,57<br>70,45 | H | 6,44<br>6,54 | N | 10,73<br>10,72 |

4z) 2-Amino-6-(3-(4-benzyloxy-phenyl)allyl)-4,5,7,8-tetrahydro-6H-thiazolo[5,4-d]azepin

Hergestellt aus 4-Benzyloxy-cinnamylchlorid und 2 Äquivalenten 2-Amino-4,5,7,8-tetrahydro-6H-thiazolo[5,4-d]azepin in Chloroform während einer Stunden bei 50 °C.
Ausbeute: 13% der Theorie,
Schmelzpunkt: 135-140 °C (Äther)

| | | | | | | |
|---|---|---|---|---|---|---|
| Ber.:<br>Gef.: | C | 70,56<br>70,80 | H | 6,44<br>6,42 | N | 10,73<br>10,52 |

Beispiel 5

2-Amino-6-cinnamyl-4,5,7,8-tetrahydro-6H-thiazolo[5,4-d]azepin

Zur Lösung von 0,50 g (2,2 mMol) 1-Cinnamyl-hexahydro-4H-azepin-4-on in 3,7 ml Eisessig gibt man bei Raumtemperatur zunächst 1 ml einer 33%igen Bromwasserstoff/Eisessig-Lösung und tropft dann innerhalb 10 Minuten die Lösung von 0,11 ml (2,2 mMol) Brom in 0,65 ml Eisessig zu. Nach 1,5 Stunden Rühren bei Raumtemperatur dampft man im Vakuum bei 50 °C ein. Zum Eindampfungsrückstand gibt man 5 ml Äthanol, dampft im Vakuum ein und wiederholt diese Prozedur.
Den Eindampfrückstand (rohes 5-Brom-1-cinnamyl-hexahydro-4H-azepin-4-on-hydrobromid) löst man in 15 ml wasserfreiem Äthanol, gibt 0,167 g (2,2 mMol) Thioharnstoff zu und erhitzt das Gemisch 2 Stunden unter Rückfluß. Man dampft im Vakuum ein, alkalisiert mit Natronlauge und extrahiert mit Chloroform. Den über Natriumsulfat getrockneten und filtrierten Chloroform-Extrakt dampft man im Vakuum ein. Den Eindampfungsrückstand reinigt man durch Säulenchromatographie an Kieselgel (Chloroform/Methanol/konz. Ammoniak = 100:10:1).
Ausbeute: 0,13 g (21 % der Theorie),
Schmelzpunkt: 121-124 °C (Äther)

| | | | | | | |
|---|---|---|---|---|---|---|
| Ber.:<br>Gef.: | C | 67,35<br>67,20 | H | 6,71<br>6,69 | N | 14,73<br>14,50 |

Analog Beispiel 5 wurde folgende Verbindung hergestellt:

5a) 2-Amino-6-(3-phenyl-2-propin-1-yl)-4,5,7,8-tetrahydro-6H-thiazolo[5,4-d]azepin x 0,5 $H_2O$

Hergestellt aus 5-Brom-1-(3-phenyl-2-propin-1-yl)-hexahydro-4H-azepin-4-on-hydrobromid durch Umsetzung mit Thioharnstoff in Äthanol.
Ausbeute: 4 % der Theorie,
Schmelzpunkt: 145-148 °C

| | | | | | | | |
|---|---|---|---|---|---|---|---|
| Ber.:<br>Gef.: | (x 0,5 $H_2O$) | C | 65,74<br>65,72 | H | 6,20<br>5,96 | N | 14,38<br>14,50 |

Beispiel 6

2-Amino-6-cinnamyl-4,5,7,8-tetrahydro-6H-thiazolo[5,4-d]azepin

Zu 0,687 g (2,2 mMol) Formamidin-dihydrobromid in 3 ml Eisessig tropft man bei 80 °C die Lösung von 0,50 g (2,2 mMol) 1-Cinnamyl-hexahydro-4H-azepin-4-on in 1,5 ml Eisessig und rührt dann 2 Stunden bei 100 °C. Man dampft im Vakuum ein, stellt mit Natronlauge stark alkalisch und extrahiert mit Chloroform. Den getrockneten und filtrierten Chloroform-Extrakt dampft man im Vakuum ein. Den Eindampfrückstand reinigt man durch Säulenchromatographie an Kieselgel (Chloroform/Methanol/konz. Ammoniak = 100:10:1). Nach Elution einer geringen Menge der isomeren Verbindung 2-Amino-7-cinnamyl-4,5,7,8-tetrahydro-6H-thiazolo[5,4-c]azepin wird die Titelverbindung eluiert.
Ausbeute: 0,045 g (7 % der Theorie),
Schmelzpunkt: 120-124 °C (Äther)
Molpeak (m/z): Ber.: 285 Gef.: 285

Beispiel 7

2-Amino-6-(3-(2-amino-4-thiazolyl)allyl)-4,5,7,8-tetrahydro-6H-thiazolo[5,4-d]azepin

Zu 0,58 g (3,7 mMol) 3-(2-Amino-4-thiazolyl)allylalkohol in 10 ml Chloroform tropft man unter Rühren bei Raumtemperatur 0,81 ml (11,1 mMol) Thionylchlorid und rührt eine Stunde. Man dampft im Vakuum ein, trocknet den schaumartigen Eindampfrückstand [rohes 3-(2-Amino-4-thiazolyl)allylchlorid-hydrochlorid] bei 20 °C/0,1 Torr und löst ihn dann in 10 ml wasserfreiem Dimethylformamid. Zu dieser Lösung tropft man zügig unter Stickstoff die Lösung von 2,5 g (14,8 mMol) 2-Amino-4,5,7,8-tetrahydro-6H-thiazolo[5,4-d]azepin in 20 ml Dimethylformamid. Man rührt 1,5 Stunden bei 50-60 °C, dampft im Vakuum ein, entfernt Reste an Dimethylformamid bei 0,1 Torr und reinigt den Rückstand direkt durch Säulenchromatographie an Kieselgel (Chloroform/Methanol/konz.Ammoniak = 5:1:0,15).
Ausbeute: 0,40 g (35 % der Theorie),
Schmelzpunkt: 186-190 °C (Aceton)

| | | | | | | |
|---|---|---|---|---|---|---|
| Ber.:<br>Gef.: | C | 50,81<br>50,61 | H | 5,58<br>5,67 | N | 22,79<br>22,60 |

Analog Beispiel 7 wurde folgende Verbindung hergestellt:

7a) 2-Amino-6-(3-(1-isochinolinyl)allyl)-4,5,7,8-tetrahydro-6H-thiazolo[5,4-d]azepin x 0,25 $H_2O$

Hergestellt aus 3-(1-Isochinolinyl)-allylchlorid-hydrochlorid und 3 Äquivalenten 2-Amino-4,5,7,8-tetrahydro-6H-thiazolo[5,4-d]azepin in Chloroform.

Ausbeute: 20 % der Theorie,
Schmelzpunkt: 156-157 ° C (Äther)

| | | | | | | | |
|---|---|---|---|---|---|---|---|
| Ber.: | (x 0,25 $H_2O$) | C | 66,93 | H | 6,06 | N | 16,44 |
| Gef.: | | | 66,82 | | 6,02 | | 16,29 |

Beispiel 8

2-Amino-6-(3-(2-furyl)allyl)-4,5,7,8-tetrahydro-6H-thiazolo[5,4-d]azepin

Zur gerührten Lösung von 1,25 g (10 mMol) 3-(2-Furyl)allyl)alkohol in 25 ml wasserfreiem Äther tropft man bei -5 ° C 0,73 ml (10 mMol) Thionylchlorid. Man rührt 15 Minuten bei -5 ° C, dampft dann im Vakuum bei einer Badtemperatur von 0 bis 5 ° C ein, löst den Eindampfrückstand [rohes 3-(2-Furyl)allylchlorid] sofort in kaltem (-5 ° C) Chloroform, versetzt mit 3,08 g (10 mMol) 2-Amino-4,5,7,8-tetrahydro-6H-thiazolo[5,4-d]-azepin und rührt eine Stunde bei 50 ° C. Man schüttelt mit Wasser aus, trocknet und filtriert die Chloroform-Lösung und dampft sie im Vakuum ein. Den Eindampfrückstand reinigt man durch Säulenchromatographie an Kieselgel (Chloroform/Methanol = 10:1).
Ausbeute: 0,38 g (13,8 % der Theorie,
Schmelzpunkt: 112-119 ° C (Äther)

| | | | | | | |
|---|---|---|---|---|---|---|
| Ber.: | C | 61,06 | H | 6,22 | N | 15,26 |
| Gef.: | | 60,89 | | 6,17 | | 14,92 |

Analog Beispiel 8 wurden folgende Verbindungen hergestellt:

8a) 2-Amino-6-(3-(4-chlor-phenyl)allyl)-4,5,7,8-tetrahydro-6H-thiazolo[5,4-d]azepin

Hergestellt aus 4-Chlor-cinnamylchlorid und 1 Äquivalent 2-Amino-4,5,7,8-tetrahydro-6H-thiazolo[5,4-d]-azepin in Chloroform.
Ausbeute: 26 % der Theorie,
Schmelzpunkt: 148-153 ° C (Äther)

| | | | | | | |
|---|---|---|---|---|---|---|
| Ber.: | C | 60,08 | H | 5,67 | N | 13,14 |
| Gef.: | | 59,89 | | 5,51 | | 12,93 |

8b) 2-Amino-6-(3-(2-fluor-phenyl)allyl)-4,5,7,8-tetrahydro-6H-thiazolo[5,4-d]azepin

Hergestellt aus 2-Fluor-cinnamylchlorid und 2 Äquivalenten 2-Amino-4,5,7,8-tetrahydro-6H-thiazolo[5,4-d]azepin in Chloroform.
Ausbeute: 36 % der Theorie),
Schmelzpunkt: 96-102 ° C (Äther)

| | | | | | | |
|---|---|---|---|---|---|---|
| Ber.: | C | 63,34 | H | 5,98 | N | 13,85 |
| Gef.: | | 63,25 | | 6,03 | | 13,73 |

8c) 2-Amino-6-(3-(3-fluor-phenyl)allyl)-4,5,7,8-tetrahydro-6H-thiazolo[5,4-d]azepin

Hergestellt aus 3-Fluor-cinnamylchlorid und 2 Äquivalenten 2-Amino-4,5,7,8-tetrahydro-6H-thiazolo[5,4-d]azepin in Chloroform.
Ausbeute: 44 % der Theorie,
Schmelzpunkt: 128-132 ° C (Äther)

| | | | | | | |
|---|---|---|---|---|---|---|
| Ber.:<br>Gef.: | C | 63,34<br>63,43 | H | 5,98<br>6,12 | N | 13,85<br>13,60 |

8d) 2-Amino-6-(3-(4-fluor-phenyl)allyl)-4,5,7,8-tetrahydro-6H-thiazolo[5,4-d]azepin

Hergestellt aus 4-Fluor-cinnamylchlorid und 2 Äquivalenten 2-Amino-4,5,7,8-tetrahydro-6H-thiazolo[5,4-d]azepin in Chloroform.
Ausbeute: 13 % der Theorie,
Schmelzpunkt: 142-146 ° C

| | | | | | | |
|---|---|---|---|---|---|---|
| Ber.:<br>Gef.: | C | 63,34<br>63,25 | H | 5,98<br>5,97 | N | 13,85<br>13,70 |

8e) 2-Amino-6-(3-(2-methoxy-phenyl)allyl)-4,5,7,8-tetrahydro-6H-thiazolo[5,4-d]azepin

Hergestellt aus 2-Methoxy-cinnamylchlorid und 2 Äquivalenten 2-Amino-4,5,7,8-tetrahydro-6H-thiazolo-[5,4-d]azepin in Chloroform.
Ausbeute: 13 % der Theorie,
Schmelzpunkt: 80-84 ° C

| | | | | | | |
|---|---|---|---|---|---|---|
| Ber.:<br>Gef.: | C | 64,73<br>64,57 | H | 6,71<br>6,82 | N | 13,32<br>13,14 |

8f) 2-Amino-6-(3-(3-methoxy-phenyl)allyl)-4,5,7,8-tetrahydro-6H-thiazolo[5,4-d]azepin

Hergestellt aus 3-Methoxy-cinnamylchlorid und 2 Äquivalenten 2-Amino-4,5,7,8-tetrahydro-6H-thiazolo-[5,4-d]azepin in Chloroform.
Ausbeute: 21 % der Theorie,
Schmelzpunkt: 120-124 ° C

| | | | | | | |
|---|---|---|---|---|---|---|
| Ber.:<br>Gef.: | C | 64,73<br>64,80 | H | 6,71<br>6,48 | N | 13,32<br>13,15 |

8g) 2-Amino-6-(3-(4-methylthio-phenyl)allyl)-4,5,7,8-tetrahydro-6H-thiazolo[5,4-d]azepin x 0,25 $H_2O$

Hergestellt aus 4-Methylthio-cinnamylchlorid und 2 Äquivalenten 2-Amino-4,5,7,8-tetrahydro-6H-thiazolo[5,4-d]azepin in Chloroform.
Ausbeute: 31 % der Theorie,
Schmelzpunkt: 152-157 ° C

| | | | | | | | |
|---|---|---|---|---|---|---|---|
| Ber.:<br>Gef.: | (x 0,25 $H_2O$) | C | 60,74<br>60,53 | H | 6,45<br>6,23 | N | 12,51<br>12,36 |

8h) 2-Amino-6-(3-(4-methylsulfinyl-phenyl)allyl)-4,5,7,8-tetrahydro-6H-thiazolo[5,4-d]azepin-hydrat

Hergestellt aus 4-Methylsulfinyl-cinnamylchlorid und 2 Äquivalenten 2-Amino-4,5,7,8-tetrahydro-6H-thiazolo[5,4-d]azepin in Chloroform.
Ausbeute: 48 % der Theorie,

Schmelzpunkt: 171-176 °C (Äther)

| | | | | | | |
|---|---|---|---|---|---|---|
| Ber.:<br>Gef.: | C | 55,88<br>56,07 | H | 6,34<br>6,32 | N | 11,50<br>11,43 |

8i) 2-Amino-6-(3-(4-methylsulfonyl-phenyl)allyl)-4,5,7,8-tetrahydro-6H-thiazolo[5,4-d]azepin

Hergestellt aus 4-Methylsulfonyl-cinnamylchlorid und 2 Äquivalenten 2-Amino-4,5,7,8-tetrahydro-6H-thiazolo[5,4-d]azepin in Chloroform.
Ausbeute: 21 % der Theorie,
Schmelzpunkt: 157-161 °C (Äther)

| | | | | | | |
|---|---|---|---|---|---|---|
| Ber.:<br>Gef.: | C | 56,17<br>56,27 | H | 5,82<br>5,73 | N | 11,56<br>11,48 |

8k) 2-Amino-6-(4-phenyl-3-buten-1-yl)-4,5,7,8-tetrahydro-6H-thiazolo[5,4-d]azepin

Hergestellt analog Beispiel 8 aus 4-Phenyl-3-buten-1-yl-bromid [Siedepunkt 93 °C/1,5 Torr; hergestellt aus 1-Cyclopropyl-1-phenyl-carbinol mit Phosphortribromid] mit 4 Äquivalenten 2-Amino-4,5,7,8-tetrahydro-6H-thiazolo[5,4-d]azepin in reinem Chloroform während 8 Stunden bei 60 °C.
Ausbeute: 39 % der Theorie,
Schmelzpunkt: 157-158 °C (Chloroform/Toluol)

| | | | | | | |
|---|---|---|---|---|---|---|
| Ber.:<br>Gef.: | C | 68,19<br>68,06 | H | 7,07<br>7,09 | N | 14,03<br>14,01 |

8ℓ) 2-Amino-6-(3-(2,6-dimethoxy-phenyl)allyl)-4,5,7,8-tetrahydro-6H-thiazolo[5,4-d]azepin x 0,5 $H_2O$

Hergestellt aus 2,6-Dimethoxy-cinnamylchlorid und 2 Äquivalenten 2-Amino-4,5,7,8-tetrahydro-6H-thiazolo[5,4-d]azepin in Chloroform.
Ausbeute: 10 % der Theorie,
Schmelzpunkt: 100-102 °C (Äther)

| | | | | | | | |
|---|---|---|---|---|---|---|---|
| Ber.:<br>Gef.: | (x 0,5 $H_2O$) | C | 60,99<br>60,95 | H | 6,82<br>6,75 | N | 11,85<br>11,91 |

8m) 2-Amino-6-(3-(3,4,5-trimethoxy-phenyl)allyl)-4,5,7,8-tetrahydro-6H-thiazolo[5,4-d]azepin-hydrat

Hergestellt aus 3,4,5-Trimethoxy-cinnamylchlorid und 2 Äquivalenten 2-Amino-4,5,7,8-tetrahydro-6H-thiazolo[5,4-d]azepin in Chloroform.
Ausbeute: 17 % der Theorie,
Schmelzpunkt: 70-73 °C (Zers.)

| | | | | | | | |
|---|---|---|---|---|---|---|---|
| Ber.:<br>Gef.: | (x 1 $H_2O$) | C | 57,99<br>58,15 | H | 6,99<br>6,86 | N | 10,68<br>10,49 |

8n) 2-Amino-6-(3-(4-isobutoxy-phenyl)allyl)-4,5,7,8-tetrahydro-6H-thiazolo[5,4-d]azepin

Hergestellt aus 4-Isobutoxy-cinnamylchlorid und 2 Äquivalenten 2-Amino-4,5,7,8-tetrahydro-6H-thiazolo-[5,4-d]azepin in Chloroform.
Ausbeute: 6 % der Theorie,
Schmelzpunkt: 110-113 °C (Äther)

| | | | | | | |
|---|---|---|---|---|---|---|
| Ber.:<br>Gef.: | C | 67,20<br>67,01 | H | 7,61<br>7,71 | N | 11,76<br>11,50 |

8o) 2-Amino-6-(2,3-diphenyl)allyl)-4,5,7,8-tetrahydro-6H-thiazolo[5,4-d]azepin

Hergestellt aus 2,3-Diphenyl-allyl)chlorid und 2 Äquivalenten 2-Amino-4,5,7,8-tetrahydro-6H-thiazolo[5,4-d]azepin in Chloroform.
Ausbeute: 19 % der Theorie,
Schmelzpunkt: 112-115 °C (Petroläther)

| | | | | | | |
|---|---|---|---|---|---|---|
| Ber.:<br>Gef.: | C | 73,11<br>72,92 | H | 6,41<br>6,50 | N | 11,63<br>11,57 |

Beispiel 9

2-Amino-6-(3-(2-methyl-4-thiazolyl)allyl)-4,5,7,8-tetrahydro-6H-thiazolo[5,4-d]azepin-semihydrat

Zur gerührten Lösung von 3,1 g (20 mMol) 3-(2-Methyl-4-thiazolyl)allylalkohol in 120 ml wasserfreiem Äther tropft man unter Stickstoff bei 5 bis 10 °C 2,9 ml (40 mMol) Thionylchlorid, wobei sich ein farbloser Niederschlag bildet. Man rührt 10 Minuten und dampft dann bei 20 °C im Vakuum ein. Der Eindampfrückstand [rohes 3-(2-Methyl-4-thiazolyl)allyl)-chlorid-hydrochlorid] wird in 20 ml Chloroform gelöst und zügig mit einer Lösung von 10,1 g (60 mMol) 2-Amino4,5,7,8-tetrahydro-6H-thiazolo[5,4-d]azepin in 150 ml Chloroform versetzt. Man gibt 2,76 g (20 mMol) Kaliumcarbonat zu und rührt 3 Stunden bei 80-90 °C unter leichtem Rückfluß. Man gibt 250 ml Chloroform zu, kühlt auf Raumtemperatur ab und schüttelt dreimal mit Wasser aus. Die getrocknete und filtrierte Chloroform-Lösung dampft man im Vakuum ein. Den Eindampfrückstand reinigt man durch Säulenchromatographie an Kieselgel (Chloroform/Methanol = 5:1).
Ausbeute: 2,80 g (45 % der Theorie),
Schmelzpunkt: 182-185 °C

| | | | | | | | |
|---|---|---|---|---|---|---|---|
| Ber.:<br>Gef.: | (x 0,5 $H_2O$) | C | 53,32<br>53,48 | H | 6,07<br>5,86 | N | 17,77<br>17,79 |

Analog Beispiel 9 wurden folgende Verbindungen hergestellt:

9a) 2-Amino-6-(3-phenyl-2-buten-1-yl)-4,5,7,8-tetrahydro-6H-thiazolo[5,4-d]azepin x 0,25 $H_2O$

Hergestellt aus 3-Phenyl-2-buten-1-yl-chlorid und 1 Äquivalent 2-Amino-4,5,7,8-tetrahydro-6H-thiazolo-[5,4-d]azepin in wasserfreiem Dimethylformamid in Gegenwart von 1 Äquivalent Kaliumcarbonat während 12 Stunden bei Raumtemperatur.
Ausbeute: 34 % der Theorie,
Schmelzpunkt: 131-135 °C (Äther)

| | | | | | | | |
|---|---|---|---|---|---|---|---|
| Ber.:<br>Gef.: | (x 0,25 $H_2O$) | C | 67,18<br>67,35 | H | 7,13<br>7,12 | N | 13,83<br>13,75 |

9b) 2-Amino-6-(2-methyl-3-phenyl-2-propen-1-yl)-4,5,7,8-tetrahydro-6H-thiazolo[5,4-d]azepin

Hergestellt aus 2-Methyl-3-phenyl-allylchlorid und 2 Äquivalent 2-Amino-4,5,7,8-tetrahydro-6H-thiazolo-[5,4-d]azepin in Chloroform während 2 Stunden bei Raumtemperatur.
Ausbeute: 8 % der Theorie,
Schmelzpunkt: 112-115 ° C

| | | | | | | |
|---|---|---|---|---|---|---|
| Ber.:<br>Gef.: | C | 68,21<br>68,03 | H | 7,07<br>7,17 | N | 14,04<br>14,27 |

Beispiel 10

2-Amino-6-(3-(2-pyridyl)-2-propin-1-yl)-4,5,7,8-tetrahydro-6H-thiazolo[5,4-d]azepin

Zu einem unter Stickstoff bei Raumtemperatur gerührten Gemisch von 3,0 g (14,5 mMol) 2-Amino-6-propargyl-4,5,7,8-tetrahydro-6H-thiazolo[5,4-d)azepin (Fp. 157-160 ° C, hergestellt aus 2-Amino-4,5,7,8-tetrahydro-6H-thiazolo[5,4-d]azepin mit Propargylbromid in Chloroform), 1,38 ml (14,5 mMol) 2-Brompyridin und 100 ml Diäthylamin gibt man 61 mg (0,32 mMol) Kupfer(I)jodid und 225 mg (0,32 mMol) Bis-(triphenylphosphin)-palladiumchlorid und rührt 48 Stunden bei Raumtemperatur. Man dampft im Vakuum ein und verteilt den Eindampfrückstand zwischen Chloroform und Wasser. Den getrockneten und filtrierten Chloroform-Extrakt dampft man im Vakuum ein. Den Eindampfrückstand reinigt man durch Säulenchromatographie an Kieselgel (Chloroform/Methanol = 10:1).
Ausbeute: 2,7 g (65 % der Theorie),
Schmelzpunkt: 165-168 ° C (Aceton)

| | | | | | | |
|---|---|---|---|---|---|---|
| Ber.:<br>Gef.: | C | 63,37<br>63,20 | H | 5,67<br>5,50 | N | 19,71<br>20,00 |

Analog Beispiel 10 wurde folgende Verbindung hergestellt:

10a) 2-Amino-6-(3-(3-pyridyl)-2-propin-1-yl)-4,5,7,8-tetrahydro-6H-thiazolo[5,4-d]azepin-semihydrat

Hergestellt aus 2-Amino-6-propargyl-4,5,7,8-tetrahydro-6H-thiazolo[5,4-d]azepin und 3-Brompyridin. Die säulenchromatographische Reinigung erfolgt an Kieselgel (Toluol/Äthylacetat/Methanol = 4:2:2).
Ausbeute: 37 % der Theorie,
Schmelzpunkt: 118-121 ° C (Äther)

| | | | | | | | |
|---|---|---|---|---|---|---|---|
| Ber.:<br>Gef.: | (x 0,5 $H_2O$) | C | 61,40<br>61,51 | H | 5,84<br>5,52 | N | 19,10<br>19,25 |

Beispiel 11

2-Amino-6-(3-(6-chlor-2-pyridyl)allyl)-4,5,7,8-tetrahydro-6H-thiazolo[5,4-d]azepin

Zur gerührten Lösung von 0,90 g (5,3 mMol) 3-(6-Chlor-2-pyridyl)allylalkohol in 20 ml wasserfreiem Äther tropft man bei Raumtemperatur langsam die Lösung von 0,38 ml (5,3 mMol) Thionylchlorid in 0,5 ml wasserfreiem Äther, wobei ein Niederschlag ausfällt. Man rührt 20 Minuten und dampft bei Raumtemperatur im Vakuum ein. Den Eindampfrückstand [rohes 3-(6-Chlor-2-pyridyl)allylchlorid-hydrochlorid] löst man in 10 ml Chloroform. Diese Lösung wird zu einer bei 50-60 ° C gerührten Lösung von 2,70 g (15,9 mMol) 2-Amino-4,5,7,8-tetrahydro-6H-thiazolo[5,4-d]azepin in 41 ml Chloroform getropft. Man rührt 5 Stunden bei 50-60 ° C, verdünnt mit 200 ml Chloroform und schüttelt mehrmals mit Wasser aus. Die getrocknete und filtrierte Chloroform-Lösung dampft man im Vakuum ein. Den Eindampfrückstand reinigt man durch Säulenchromatographie an Kieselgel (Toluol/Äthylacetat/Methanol = 4:2:1).

Ausbeute: 1,1 g (64 % der Theorie),
Schmelzpunkt: 161-164 °C (Äther)

| | | | | | | | | |
|---|---|---|---|---|---|---|---|---|
| Ber.:<br>Gef.: | C | 56,15<br>56,28 | H | 5,34<br>5,41 | Cl | 11,05<br>10,99 | N | 17,46<br>17,43 |

Analog Beispiel 11 wurden folgende Verbindungen hergestellt:

11a) 2-Amino-6-(3-(2-pyridyl)allyl)-4,5,7,8-tetrahydro-6H-thiazolo[5,4-d]azepin

Hergestellt aus 3-(2-Pyridyl)allyl)chlorid-hydrochlorid und 3 Äquivalenten 2-Amino-4,5,7,8-tetrahydro-6H-thiazolo[5,4-d]azepin in Chloroform.
Ausbeute: 33 % der Theorie,
Schmelzpunkt: 162-165 °C

| | | | | | | |
|---|---|---|---|---|---|---|
| Ber.:<br>Gef.: | C | 62,92<br>63,16 | H | 6,34<br>6,35 | N | 19,57<br>19,36 |

200 MHz-$^1$H-NMR-Spektrum ($d^6$-DMSO/$CD_3OD$):
δ = 3,39 ppm (Dublett), 2H (allylisches $CH_2$)
δ = 6,65 ppm (Dublett), 1H (olefinisches H)
δ = 6,74 ppm (Triplett) und
δ = 6,82 ppm (Triplett), 1H (olefinisches H)
Durch Lösen der Base in Methanol, Zugabe von 1 Äquivalent 1N-Salzsäure, Eindampfen im Vakuum und Trocknen über Phosphorpentoxid bei 60 °C/20 Torr erhält man in 84%iger Ausbeute das 2-Amino-6-(3-(2-pyridyl)allyl)-4,5,7,8-tetrahydro-6H-thiazolo[5,4-d]azepin-hydrochlorid x 1,5 $H_2O$ mit dem Schmelzbereich von 100-120 °C.

| | | | | | | | |
|---|---|---|---|---|---|---|---|
| Ber.:<br>Gef.: | (x 1,5 $H_2O$) | C | 51,50<br>51,60 | H | 6,34<br>6,41 | N | 16,02<br>16,00 |

11b) 2-Amino-6-(3-(3-pyridyl)allyl)-4,5,7,8-tetrahydro-6H-thiazolo[5,4-d]azepin x 0,25 $H_2O$

Hergestellt aus 3-(3-Pyridyl)allyl)chlorid-hydrochlorid und 3 Äquivalenten 2-Amino-4,5,7,8-tetrahydro-6H-thiazolo[5,4-d]azepin in wasserfreiem Dimethylformamid durch zweitägiges Rühren bei Raumtemperatur.
Ausbeute: 59 % der Theorie,
Schmelzpunkt: 166-169 °C (Aceton)

| | | | | | | | |
|---|---|---|---|---|---|---|---|
| Ber.:<br>Gef.: | (x 0,25 $H_2O$) | C | 61,93<br>61,97 | H | 6,41<br>6,35 | N | 19,26<br>19,51 |

200 MHz-$^1$H-NMR-Spektrum ($d^6$-DMSO/$CD_3OD$):
δ = 3,38 ppm (Dublett), 2H (allylisches $CH_2$)
δ = 6,43 ppm (Triplett) und
δ = 6,50 ppm (Triplett), 1H (olefinisches H)
δ = 6,62 ppm (Dublett), 1H (olefinisches H)

11c) 2-Amino-6-(3-(4-pyridyl)allyl)-4,5,7,8-tetrahydro-6H-thiazolo[5,4-d]azepin x 0,4 $H_2O$

Hergestellt aus 3-(4-Pyridyl)allyl)chlorid-hydrochlorid und 3 Äquivalenten 2-Amino-4,5,7,8-tetrahydro-6H-thiazolo[5,4-d]azepin in Chloroform.
Ausbeute: 28 % der Theorie,
Schmelzpunkt: 210-215 °C (Äther)

| | | | | | | | |
|---|---|---|---|---|---|---|---|
| Ber.:<br>Gef.: | (x 0,4 $H_2O$) | C | 61,36<br>61,14 | H | 6,45<br>6,28 | N | 19,08<br>19,07 |

11d) 2-Amino-6-(3-(3-pyridyl)propyl)-4,5,7,8-tetrahydro-6H-thiazolo[5,4-d]azepin

Hergestellt aus 3-(3-Pyridyl)propylchlorid-hydrochlorid und 1 Äquivalent 2-Amino-4,5,7,8-tetrahydro-6H-thiazolo[5,4-d]azepin in wasserfreiem Dimethylformamid in Gegenwart von 2 Äquivalenten Kaliumcarbonat während 1,5 Stunden bei 80 °C.
Ausbeute: 8 % der Theorie,
Schmelzpunkt: 90-92 °C (Äther)
Molpeak (m/z) Ber.: 288 Gef.: 288

11e) 2-Amino-6-(3-(4-amino-3,5-dibrom-phenyl)allyl)-4,5,7,8-tetrahydro-6H-thiazolo[5,4-d]azepin

Hergestellt aus 4-Amino-4,5-dibrom-cinnamylchlorid-hydrochlorid [aus 4-Amino-3,5-dibrom-cinnamylalkohol mit 1,2 Äquivalenten Thionylchlorid in Chloroform] und 2 Äquivalenten 2-Amino-4,5,7,8-tetrahydro-6H-thiazolo[5,4-d]azepin in Chloroform.
Ausbeute: 12 % der Theorie,
Schmelzpunkt: 198-199 °C

| | | | | | | |
|---|---|---|---|---|---|---|
| Ber.:<br>Gef.: | C | 41,94<br>41,80 | H | 3,96<br>3,99 | N | 12,23<br>12,17 |

11f) 2-Amino-6-(3-(4-amino-3,5-dichlor-phenyl)allyl)-4,5,7,8-tetrahydro-6H-thiazolo[5,4-d]azepin x 0,5 $H_2O$

Hergestellt aus 4-Amino-3,5-dichlor-cinnamylchlorid-hydrochlorid [aus 4-Amino-3,5-dichlor-cinnamylalkohol mit 1,2 Äquivalenten Thionylchlorid in Chloroform] und 2 Äquivalenten 2-Amino-4,5,7,8-tetrahydro-6H-thiazolo[5,4-d]azepin in Chloroform.
Ausbeute: 7 % der Theorie,
Schmelzpunkt: 163-165 °C

| | | | | | | | |
|---|---|---|---|---|---|---|---|
| Ber.:<br>Gef.: | (x 0,5 $H_2O$) | C | 50,79<br>50,98 | H | 5,06<br>5,17 | N | 18,74<br>18,80 |

11g) 2-Amino-6-(3-(4-hydroxy-phenyl)allyl)-4,5,7,8-tetrahydro-6H-thiazolo[5,4-d]azepin x 0,6 $H_2O$

Hergestellt aus 3-(4-Hydroxy-phenyl)allylchlorid und 2 Äquivalenten 2-Amino-4,5,7,8-tetrahydro-6H-thiazolo[5,4-d]azepin in Chloroform.
Ausbeute: 1 % der Theorie,
Schmelzpunkt: 153-161 °C (Äther); Sintern 105 °C.

| | | | | | | | |
|---|---|---|---|---|---|---|---|
| Ber.:<br>Gef.: | (x 0,6 $H_2O$) | C | 61,55<br>61,50 | H | 6,52<br>6,40 | N | 13,49<br>13,08 |

11h) 2-Amino-6-(3-(4-chlorphenyl)propyl)-4,5,7,8-tetrahydro-6H-thiazolo[5,4-d]azepin

Hergestellt aus 3-(4-Chlorphenyl)propylbromid und 2 Äquivalenten 2-Amino-4,5,7,8-tetrahydro-6H-thiazolo[5,4-d]azepin in wasserfreiem Dimethylformamid.
Ausbeute: 37 % der Theorie,
Schmelzpunkt: 153 °C

| | | | | | | |
|---|---|---|---|---|---|---|
| Ber.:<br>Gef.: | C | 59,71<br>59,53 | H | 6,26<br>6,06 | N | 13,05<br>13,21 |

11i) 2-Amino-6-(3-(3-methyl-2-pyridyl)allyl)-4,5,7,8-tetrahydro-6H-thiazolo[5,4-d]azepin

Hergestellt aus 3-(3-Methyl-2-pyridyl)allylchlorid-hydrochlorid und 3 Äquivalenten 2-Amino-4,5,7,8-tetrahydro-6H-thiazolo[5,4-d]azepin in Chloroform.
Ausbeute: 45 % der Theorie,
Schmelzpunkt: 188-190 °C (Äther)

| | | | | | | |
|---|---|---|---|---|---|---|
| Ber.:<br>Gef.: | C | 63,98<br>63,87 | H | 6,71<br>6,59 | N | 18,65<br>18,62 |

11k) 2-Amino-6-(3-(5-methyl-2-pyridyl)allyl)-4,5,7,8-tetrahydro-6H-thiazolo[5,4-d]azepin

Hergestellt aus 3-(5-Methyl-2-pyridyl)allylchlorid-hydrochlorid und 3 Äquivalenten 2-Amino-4,5,7,8-tetrahydro-6H-thiazolo[5,4-d]azepin in Chloroform.
Ausbeute: 22 % der Theorie,
Schmelzpunkt: 172-175 °C (Isopropanol)

| | | | | | | |
|---|---|---|---|---|---|---|
| Ber.:<br>Gef.: | C | 63,98<br>63,82 | H | 6,71<br>6,59 | N | 18,65<br>18,61 |

11ℓ) 2-Amino-6-(3-(6-methyl-2-pyridyl)allyl)-4,5,7,8-tetrahydro-6H-thiazolo[5,4-d]azepin x 0,5 $H_2O$

Hergestellt aus 3-(6-Methyl-2-pyridyl)allylchlorid-hydrochlorid und 3 Äquivalenten 2-Amino-4,5,7,8-tetrahydro-6H-thiazolo[5,4-d]azepin in Chloroform.
Ausbeute: 21 % der Theorie,
Schmelzpunkt: 122-125 °C (Petroläther)

| | | | | | | | |
|---|---|---|---|---|---|---|---|
| Ber.:<br>Gef.: | (x 0,5 $H_2O$) | C | 62,10<br>62,13 | H | 6,84<br>6,80 | N | 18,11<br>17,98 |

11m) 2-Amino-6-(3-(6-methyl-3-pyridyl)allyl)-4,5,7,8-tetrahydro-6H-thiazolo[5,4-d]azepin

Hergestellt aus 3-(6-Methyl-3-pyridyl)allylchlorid-hydrochlorid und 3 Äquivalenten 2-Amino-4,5,7,8-tetrahydro-6H-thiazolo[5,4-d]azepin in Chloroform.
Ausbeute: 30 % der Theorie,
Schmelzpunkt: 180-184 °C (Äther)

| | | | | | | |
|---|---|---|---|---|---|---|
| Ber.:<br>Gef.: | C | 63,98<br>63,79 | H | 6,71<br>6,72 | N | 18,65<br>18,44 |

11n) 2-Amino-6-(2-äthyl-3-phenyl-2-propen-1-yl)-4,5,7,8-tetrahydro-6H-thiazolo[5,4-d]azepin

Hergestellt aus 2-Äthyl-1-chlor-3-phenyl-2-propen und 2 Äquivalenten 2-Amino-4,5,7,8-tetrahydro-6H-thiazolo[5,4-d]azepin in Chloroform.
Ausbeute: 22 % der Theorie,

Schmelzpunkt: 110-113 °C (Petroläther)

| | | | | | | |
|---|---|---|---|---|---|---|
| Ber.:<br>Gef.: | C | 68,98<br>68,68 | H | 7,40<br>7,27 | N | 13,41<br>13,55 |

11o) 2-Amino-6-(3-phenyl-2-n-propyl-2-propen-1-yl)-4,5,7,8-tetrahydro-6H-thiazolo[5,4-d]azepin

Hergestellt aus 1-Chlor-3-phenyl-2-n-propyl-2-propen und 2 Äquivalenten 2-Amino-4,5,7,8-tetrahydro-6H-thiazolo[5,4-d]azepin in Chloroform.
Ausbeute: 15 % der Theorie,
Schmelzpunkt: 77-80 °C (Petroläther)

| | | | | | | |
|---|---|---|---|---|---|---|
| Ber.:<br>Gef.: | C | 69,70<br>69,64 | H | 7,70<br>7,57 | N | 12,83<br>12,66 |

11p) 2-Amino-6-(3-(2-(1-piperidino)phenyl)-2-propen-1-yl)-4,5,7,8-tetrahydro-6H-thiazolo[5,4-d]azepin

Hergestellt aus 2-(1-Piperidino)-cinnamylchlorid-hydrochlorid und 3 Äquivalenten 2-Amino-4,5,7,8-tetrahydro-6H-thiazolo[5,4-d]azepin in Chloroform während 5 Stunden bei 50 °C. - Bei der säulenchromatographischen Reinigung an Kieselgel (Toluol/Äthylacetat/Methanol = 4:2:1) wird zunächst die isomere Verbindung 2-Amino-6-(1-(2-(1-piperidino)phenyl)-2-propen-1-yl)-4,5,7,8-tetrahydro-6H-thiazolo[5,4-d]azepin eluiert [7 % der Theorie; Schmelzpunkt: 85-95 °C (Äther)].
Anschließend wird die Titelverbindung eluiert.
Ausbeute: 6 % der Theorie,
Schmelzpunkt: 113-115 °C (Äther)

| | | | | | | |
|---|---|---|---|---|---|---|
| Ber.:<br>Gef.: | C | 68,45<br>68,36 | H | 7,66<br>7,96 | N | 15,20<br>15,15 |

Beispiel 12

(Z)-2-Amino-6-(3-(2-pyridyl)-2-propen-1-yl)-4,5,7,8-tetrahydro-6H-thiazolo[5,4-d]azepin x 0,25 $H_2O$

Man hydriert 1,5 g (5,3 mMol) 2-Amino-6-(3-(2-pyridyl)-2-propin-1-yl)-4,5,7,8-tetrahydro-6H-thiazolo[5,4-d]azepin in 75 ml absolutem Äthanol bei 1 bar Wasserstoffdruck an 0,75 g Palladium/Bariumsulfat (5 %) 2 Stunden bei Raumtemperatur. Man filtriert vom Katalysator ab und dampft im Vakuum ein. Den Eindampfrückstand reinigt man durch Säulenchromatographie an Kieselgel (Chloroform/Methanol = 3:1).
Ausbeute: 0,47 g (31 % der Theorie),
Schmelzpunkt: 156-158 °C (Aceton)

| | | | | | | | |
|---|---|---|---|---|---|---|---|
| Ber.:<br>Gef.: | (x 0,25 $H_2O$) | C | 61,95<br>61,93 | H | 6,41<br>6,21 | N | 19,27<br>19,16 |

200 MHz-$^1$H-NMR-Spektrum ($CDCl_3$):
$\delta$ = 3,85 ppm (Dublett), 2H (allylisches $CH_2$)
$\delta$ = 6,03 ppm (Triplett) und
$\delta$ = 6,08 ppm (Triplett), J = 12 Hz 1H (olefinisches H)
$\delta$ = 6,58 ppm (Dublett), 1H (olefinisches H)
Analog Beispiel 12 wurden folgende Verbindungen hergestellt:

12a) (Z)-2-Amino-6-(3-(3-pyridyl)-2-propen-1-yl)-4,5,7,8-tetrahydro-6H-thiazolo[5,4-d]azepin

Hergestellt durch katalytische Hydrierung von 2-Amino-6-(3-(3-pyridyl)-2-propin-1-yl)-4,5,7,8-tetrahydro-6H-thiazolo[5,4-d]azepin an Palladium/Bariumsulfat(5 %) in Äthanol.
Ausbeute: 42 % der Theorie,
Schmelzpunkt: 125-126 ° C (Aceton)

| | | | | | | |
|---|---|---|---|---|---|---|
| Ber.:<br>Gef.: | C | 62,92<br>62,89 | H | 6,34<br>6,53 | N | 19,57<br>19,32 |

200 MHz-$^1$H-NMR-Spektrum ($CDCl_3$):
δ = 3,50 ppm (Dublett), 2H (allylisches $CH_2$)
δ = 6,00 ppm (Triplett) und
δ = 6,06 ppm (Triplett), J = 12 Hz 1H (olefinisches H)
δ = 6,60 ppm (Dublett), 1H (olefinisches H)

12b) (Z)-2-Amino-6-(3-phenyl-2-propen-1-yl)-4,5,7,8-tetrahydro-6H-thiazolo[5,4-d]azepin

Hergestellt durch katalytische Hydrierung von 2-Amino-6-(3-phenyl-2-propin-1-yl)-4,5,7,8-tetrahydro-6H-thiazolo[5,4-d]azepin an Palladium/Bariumsulfat(5 %) in Äthanol.
Ausbeute: 43 % der Theorie,
Schmelzpunkt: 140-142 ° C (Aceton)

| | | | | | | |
|---|---|---|---|---|---|---|
| Ber.:<br>Gef.: | C | 67,35<br>67,33 | H | 6,75<br>6,89 | N | 14,73<br>14,89 |

200 MHz-$^1$H-NMR-Spektrum ($CDCl_3$):
δ = 3,50 ppm (Dublett), 2H (allylisches $CH_2$)
δ = 5,81 ppm (Triplett) und
δ = 5,86 ppm (Triplett), 1H (olefinisches H)
δ = 6,60 ppm (Dublett), 1H (olefinisches H)
Eine geringe Menge (≦ 2%) des (E)-Isomeren ist nachweisbar durch δ = 3,40 ppm (Dublett; allylisches $CH_2$).

Beispiel 13

2-Amino-6-(3-(3-pyridyl)propyl)-4,5,7,8-tetrahydro-6H-thiazolo[5,4-d]azepin

2,0 g (7 mMol) 2-Amino-6-(3-(3-pyridyl)allyl)-4,5,7,8-tetrahydro-6H-thiazolo[5,4-d]azepin hydriert man an 1 g Palladium/Kohle(10 %) bei Raumtemperatur und 1 bar Wasserstoff in 45 ml Äthanol. Man gibt weitere 1 g bzw. 0,5 g des Katalysators nach 2 bzw. 3 Stunden Hydrierzeit zu. Nach insgesamt 4,5-stündiger Hydrierung wird vom Katalysator abfiltriert und im Vakuum eingedampft. Man verteilt zwischen Chloroform und Wasser, dampft die getrocknete und filtrierte Chloroform-Lösung im Vakuum ein und reinigt den Eindampfrückstand durch Säulenchromatographie an Kieselgel (Chloroform/Methanol = 1:1).
Ausbeute: 0,44 g (22 % der Theorie),
Schmelzpunkt: 90-92 ° C (Äther)

| | | | | | | |
|---|---|---|---|---|---|---|
| Ber.:<br>Gef.: | C | 62,48<br>62,31 | H | 6,99<br>7,01 | N | 19,43<br>19,55 |

Analog Beispiel 13 wurde folgende Verbindung hergestellt:

13a) 2-Amino-6-(3-(2-pyridyl)propyl)-4,5,7,8-tetrahydro-6H-thiazolo[5,4-d]azepin x 0,5 $H_2O$

Hergestellt durch katalytische Hydrierung von 2-Amino-6-(3-(2-pyridyl)allyl)-4,5,7,8-tetrahydro-6H-thiazolo[5,4-d]azepin an Palladium/Kohle(10 %) bei Raumtemperatur.
Ausbeute: 15 % der Theorie,
Schmelzpunkt: 122-125 °C (Äther)

| | | | | | | | |
|---|---|---|---|---|---|---|---|
| Ber.:<br>Gef.: | (x 0,5 $H_2O$) | C | 60,59<br>60,72 | H | 7,12<br>7,22 | N | 18,85<br>19,06 |

Beispiel 14

2-Amino-6-(3-phenyl-2-propin-1-yl)-4,5,7,8-tetrahydro-6H-thiazolo[5,4-d]azepin

Zur gerührten Lösung von 5,8 g (34,5 mMol) 2-Amino-4,5,7,8-tetrahydro-6H-thiazolo[5,4-d]azepin und 2,0 ml (34,5 mMol) Eisessig in 50 ml reinem Methanol tropft man bei 0 °C die Lösung von 4,5 g (34,5 mMol) 3-Phenyl-propargylaldehyd in 180 ml reinem Methanol. Danach gibt man 2,17 g (34,5 mMol) Natriumcyanoborhydrid bei 0 °C zu und rührt 1,5 Stunden unter Eiskühlung. Man dampft im Vakuum ein, versetzt mit Wasser und stellt mit konzentrierter Salzsäure sauer. Anschließend alkalisiert man durch Zugabe von festem Natriumbicarbonat und extrahiert mehrmals mit Chloroform. Die getrocknete und filtrierte Chloroform-Lösung dampft man im Vakuum ein. Den Eindampfrückstand reinigt man durch Säulenchromatographie an Kieselgel (Chloroform/Methanol/konz. Ammoniak = 100:10:0,5).
Ausbeute: 3,4 g (35 % der Theorie),
Schmelzpunkt: 155-159 °C (Aceton)

| | | | | | | |
|---|---|---|---|---|---|---|
| Ber.:<br>Gef.: | C | 67,83<br>67,69 | H | 6,05<br>6,12 | N | 14,83<br>14,69 |

Analog Beispiel 14 wurden folgende Verbindungen hergestellt:

14a) 2-Amino-6-(3-(3-pyridyl)allyl)-4,5,7,8-tetrahydro-6H-thiazolo[5,4-d]azepin

Hergestellt aus 3-(3-Pyridyl)acrolein und 2-Amino-4,5,7,8-tetrahydro-6H-thiazolo[5,4-d]azepin durch reduktive Aminierung mit Natriumcyanoborhydrid.
Ausbeute: 40 % der Theorie,
Schmelzpunkt: 162-165 °C

| | | | | | | |
|---|---|---|---|---|---|---|
| Ber.:<br>Gef.: | C | 62,92<br>63,72 | H | 6,34<br>6,32 | N | 19,57<br>19,42 |

Molpeak (m/z): Ber.: 286 Gef.: 286

14b) 2-Amino-6-(3-(4-cyano-phenyl)allyl)-4,5,7,8-tetrahydro-6H-thiazolo[5,4-d]azepin

Hergestellt aus 3-(4-Cyano-phenyl)acrolein und 2-Amino-4,5,7,8-tetrahydro-6H-thiazolo[5,4-d]azepin durch reduktive Aminierung mit Natriumcyanoborhydrid.
Ausbeute: 8 % der Theorie,
Schmelzpunkt: 199-205 °C (Zers.)

| | | | | | | |
|---|---|---|---|---|---|---|
| Ber.:<br>Gef.: | C | 65,79<br>65,59 | H | 5,85<br>6,00 | N | 18,05<br>17,88 |

Molpeak (m/z): Ber.: 286 Gef.: 286

14c) 2-Amino-6-(3-(3-indolyl)allyl)-4,5,7,8-tetrahydro-6H-thiazolo[5,4-d]azepin

Hergestellt aus 3-(3-Indolyl)acrolein und 1 Äquivalent 2-Amino-4,5,7,8-tetrahydro-6H-thiazolo[5,4-d]-azepin durch reduktive Aminierung mit 0,9 Äquivalenten Natriumcyanoborhydrid. - Die säulenchromatographische Reinigung des Rohproduktes erfolgt an neutralem Aluminiumoxid der Aktivitätsstufe I (Toluol/Äthylacetat/Äthanol = 4:1:0,5).
Ausbeute: 11 % der Theorie,
Schmelzpunkt: 155-160 °C

| | | | | | | |
|---|---|---|---|---|---|---|
| Ber.:<br>Gef.: | C | 66,65<br>66,32 | H | 6,21<br>6,34 | N | 17,27<br>17,00 |

Molpeak (m/z): Ber.: 324 Gef.: 324

14d) 2-Amino-6-(3-(3-chinolinyl)allyl)-4,5,7,8-tetrahydro-6H-thiazolo[5,4-d]azepin x 0,3 $H_2O$

Hergestellt aus 3-(3-Chinolinyl)acrolein und 2-Amino-4,5,7,8-tetrahydro-6H-thiazolo[5,4-d]azepin durch reduktive Aminierung mit Natriumcyanoborhydrid.
Ausbeute: 18 % der Theorie,
Schmelzpunkt: 172-177 °C (Aceton)

| | | | | | | | |
|---|---|---|---|---|---|---|---|
| Ber.:<br>Gef.: | (x 0,3 $H_2O$) | C | 66,75<br>66,73 | H | 6,07<br>5,96 | N | 16,39<br>16,52 |

14e) 2-Amino-6-(3-(4-isochinolinyl)-3-methoxy-1-propyl)-4,5,7,8-tetrahydro-6H-thiazolo[5,4-d]azepin (A) und 2-Amino-6-(3-(4-isochinolinyl)allyl)-4,5,7,8,-tetrahydro-6H-thiazolo[5,4-d]azepin x 0,25 $H_2O$ (B)

Hergestellt aus 3-(4-Isochinolinyl)acrolein und 2-Amino-4,5,7,8-tetrahydro-6H-thiazolo[5,4-d]azepin durch reduktive Aminierung mit Natriumcyanoborhydrid (in Methanol in Gegenwart von 1 Äquivalent Eisessig). - Die säulenchromatographische Reinigung des Rohproduktes wird an Kieselgel (Aceton/Methanol/konz. Ammoniak = 50:12:0,5) durchgeführt.
Zunächst wird die Titelverbindung (A) eluiert.
Ausbeute: 4 % der Theorie,
Schmelzpunkt: 205-208 °C (Äther)

| | | | | | | |
|---|---|---|---|---|---|---|
| Ber.:<br>Gef.: | C | 65,20<br>65,32 | H | 6,57<br>6,63 | N | 15,21<br>15,00 |

Molpeak (m/z): Ber.: 368 Gef.: 368
Danach wird die Titelverbindung (B) eluiert.
Ausbeute: 6,4 % der Theorie,
Schmelzpunkt: 210-215 °C (Aceton); Sintern 205 °C

| | | | | | | | |
|---|---|---|---|---|---|---|---|
| Ber.:<br>Gef.: | (x 0,25 $H_2O$) | C | 66,92<br>66,79 | H | 6,06<br>6,05 | N | 16,43<br>16,36 |

Molpeak (m/z): Ber.: 336 Gef.: 336

Beispiel 15

2-Amino-6-(3-(2-hydroxy-phenyl)allyl)-4,5,7,8-tetrahydro-6H-thiazolo[5,4-d]azepin x 0,5 $H_2O$

Man rührt 0,75 g (1,9 mMol) 2-Amino-6-(3-(2-benzyloxy-phenyl)allyl)-4,5,7,8-tetrahydro-6H-thiazolo[5,4-d]azepin in 30 ml reinem Methylenchlorid bei einer Innentemperatur von -25 °C und tropft bei dieser Temperatur langsam 3,8 ml (3,8 mMol) einer 1M-Bortribromid-Lösung in Methylenchlorid zu, wobei ein Niederschlag ausfällt. Nach 65 Minuten ist entsprechend dünnschichtchromatographischer Analyse keine Ausgangsverbindung mehr nachweisbar. Man versetzt mit Wasser und dann mit 10 ml halbkonzentrierter Salzsäure. Nach Trennung der Phasen neutralisiert man die saure wäßrige Phase mit festem Natriumbicarbonat. Man extrahiert mehrmals mit Chloroform unter Zusatz von etwas (ca. 1%) Methanol. Die getrocknete und filtrierte Chloroformphase dampft man im Vakuum ein. Den Eindampfrückstand reinigt man durch Säulenchromatographie an Kieselgel (Chloroform/Methanol/konz. Ammoniak = 50:10:0,5).
Ausbeute: 0,30 g (52 % der Theorie),
Schmelzpunkt: 50 °C (Äther); schaumartig

| | | | | | | | |
|---|---|---|---|---|---|---|---|
| Ber.:<br>Gef.: | (x 0,5 $H_2O$) | C | 61,93<br>61,98 | H | 6,50<br>6,58 | N | 13,54<br>13,45 |

Analog Beispiel 15 wurde folgende Verbindung hergestellt:

15a) 2-Amino-6-(3-(3-hydroxy-phenyl)allyl)-4,5,7,8-tetrahydro-6H-thiazolo[5,4-d]azepin

Hergestellt aus 2-Amino-6-(3-(3-benzyloxy-phenyl)allyl)-4,5,7,8-tetrahydro-6H-thiazolo[5,4-d]azepin mit 2 Äquivalenten Bortribromid in Methylenchlorid.
Ausbeute: 71 % der Theorie,
Schmelzpunkt: 85-95 °C (Äther); schaumartig

| | | | | | | |
|---|---|---|---|---|---|---|
| Ber.:<br>Gef.: | C | 63,77<br>63,69 | H | 6,36<br>6,59 | N | 13,96<br>13,77 |

Beispiel 16

2-Amino-6-(3-(2-amino-phenyl)allyl)-4,5,7,8-tetrahydro-6H-thiazolo[5,4-d]azepin

Man hydriert 1,0 g (3 mMol) 2-Amino-6-(3-(2-nitro-phenyl)allyl)-4,5,7,8-tetrahydro-6H-thiazolo[5,4-d]-azepin bei 1 bar Wasserstoff an 1 g Raney-Nickel in Äthanol bei Raumtemperatur (45 Minuten). Man filtriert vom Katalysator ab, dampft im Vakuum ein und reinigt den Eindampfrückstand durch Säulenchromatographie an Kieselgel (Chloroform/Methanol/konz. Ammoniak = 90:10:0,5).
Ausbeute: 0,24 g (26 % der Theorie),
Schmelzpunkt: 111-116 °C (Äther)

| | | | | | | |
|---|---|---|---|---|---|---|
| Ber.:<br>Gef.: | C | 63,98<br>63,70 | H | 6,71<br>6,50 | N | 18,65<br>18,43 |

Analog Beispiel 16 wurden folgende Verbindungen hergestellt:

16a) 2-Amino-6-(3-(4-amino-phenyl)allyl)-4,5,7,8-tetrahydro-6H-thiazolo[5,4-d]azepin x 0,5 $H_2O$

Hergestellt durch katalytische Hydrierung von 2-Amino-6-(3-(4-nitrophenyl)allyl)-4,5,7,8-tetrahydro-6H-thiazolo[5,4-d]azepin an Raney-Nickel in Äthanol.
Ausbeute: 71 % der Theorie,
Schmelzpunkt: 183-187 °C (Äther); Sintern 174 °C.

| | | | | | | | |
|---|---|---|---|---|---|---|---|
| Ber.:<br>Gef.: | (x 0,5 $H_2O$) | C | 62,10<br>62,24 | H | 6,84<br>6,85 | N | 18,11<br>17,95 |

16b) 2-Amino-6-(3-(3-amino-phenyl)allyl)-4,5,7,8-tetrahydro-6H-thiazolo[5,4-d]azepin-dihydrochlorid-dihydrat x 0,33 Isopropanol

Hergestellt durch katalytische Hydrierung von 2-Amino-6-(3-(3-nitro-phenyl)allyl)-4,5,7,8-tetrahydro-6H-thiazolo[5,4-d]azepin an Raney-Nickel in wasserfreiem Dimethylformamid (4,5 Stunden bei Raumtemperatur). Nach der säulenchromatographischen Reinigung wird die Base in Isopropanol gelöst. Durch Versetzen mit ätherischer Salzsäure, Abkühlen, Filtrieren und Trocknen bei 100 °C/0,1 Torr über Phosphorpentoxid erhält man die Titelverbindung.
Ausbeute: 37 % der Theorie,
Schmelzpunkt: 160 °C (Zers.)

| | | | | | | | |
|---|---|---|---|---|---|---|---|
| Ber.<br>Gef.: | (x 2 HCl x 2 $H_2O$ x 0,33 Isopropanol): | C | 45,61<br>45,46 | H | 6,54<br>6,22 | N | 12,52<br>12,92 |

Beispiel 17

2-Amino-6-(2-phenyl-1-cyclopropyl-methyl)-4,5,7,8-tetrahydro-6H-thiazolo[5,4-d]azepin

Zu 0,73 g (19,1 mMol) Lithiumaluminiumhydrid in 40 ml Tetrahydrofuran tropft man unter Rühren und unter Stickstoff zügig die Lösung von 2,0 g (6,4 mMol) 2-Amino-6-(2-phenyl-1-cyclopropyl-carbonyl)-4,5,7,8-tetrahydro-6H-thiazolo[5,4-d]azepin [Schmelzpunkt 122-126 °C; hergestellt aus 2-Phenyl-1-cyclopropyl-carbonsäurechlorid und 2 Äquivalenten 2-Amino-4,5,7,8-tetrahydro-6H-thiazolo[5,4-d]azepin in Chloroform] in 40 ml Tetrahydrofuran. Man rührt zwei Stunden im Bad von 40 °C, kühlt ab, gibt Äthylacetat zur Zersetzung überschüssigen Lithiumaluminiumhydrids zu und hydrolysiert durch Zutropfen von gesättigter Ammoniumchlorid-Lösung. Man filtriert den Niederschlag ab und dampft das Filtrat im Vakuum ein. Den Eindampfrückstand reinigt man durch Säulenchromatographie an Kieselgel (Chloroform/Methanol = 10:1).
Ausbeute: 0,65 g (34 % der Theorie),
Schmelzpunkt: 109-113 °C (Isopropanol)

| | | | | | | |
|---|---|---|---|---|---|---|
| Ber.:<br>Gef.: | C | 68,21<br>68,25 | H | 7,07<br>7,11 | N | 14,04<br>14,10 |

Beispiel 18

2-Amino-6-(3-(4-aminocarbonyl-phenyl)allyl)-4,5,7,8-tetrahydro-6H-thiazolo[5,4-d]azepin x 0,5 $H_2O$

0,50 g (1,6 mMol) 2-Amino-6-(3-(4-cyano-phenyl)allyl)-4,5,7,8-tetrahydro-6H-thiazolo[5,4-d]azepin erhitzt man in 5 g Polyphosphorsäure (85 % Diphosphorpentoxid) 30 Minuten bei 100 °C. Man kühlt ab und stellt unter Zugabe von Eis mit konzentriertem Ammoniak alkalisch. Man extrahiert mehrfach mit Chloroform (unter Zusatz von 1 % Methanol), wäscht die Chloroform-Lösung einmal mit Wasser, trocknet und filtriert sie und dampft sie im Vakuum ein. Den Eindampfrückstand kristallisiert man aus Methanol.
Ausbeute: 0,33 (63 % der Theorie),
Schmelzpunkt: 205-210 °C (Zers.)

| | | | | | | | |
|---|---|---|---|---|---|---|---|
| Ber.:<br>Gef.: | (x 0,5 $H_2O$) | C | 60,55<br>60,69 | H | 6,27<br>6,18 | N | 16,60<br>16,48 |

Beispiel 19

2-Amino-6-(3-(4-äthoxycarbonyl-phenyl)allyl)-4,5,7,8-tetrahydro-6H-thiazolo[5,4-d]azepin

Zu einer Lösung von 0,50 g (1,6 mMol) 2-Amino-6-(3-(4-cyano-phenyl)allyl)-4,5,7,8-tetrahydro-6H-thiazolo[5,4-d]azepin in 50 ml Äthanol leitet man unter Rühren und Erhitzen auf Rückfluß so lange trockenen Chlorwasserstoff ein, bis kein Ausgangsprodukt mehr nachweisbar ist. Man dampft im Vakuum ein, verteilt zwischen 1/4 konzentriertem Ammoniak und Chloroform, und dampft den getrockneten und filtrierten Chloroform-Extrakt im Vakuum ein. Den Eindampfrückstand reinigt man durch Säulenchromatographie an Kieselgel (Toluol/Äthylacetat/Äthanol = 4:2:2).
Ausbeute: 0,23 g (40 % der Theorie),
Schmelzpunkt: 111-115 °C

| | | | | | | |
|---|---|---|---|---|---|---|
| Ber.:<br>Gef.: | C | 63,85<br>63,65 | H | 6,48<br>6,64 | N | 11,75<br>11,61 |

Beispiel 20

2-Amino-6-(4-phenyl-1-butyl)-4,5,7,8-tetrahydro-6H-thiazolo[5,4-d]azepin

Man rührt 3,0 g (20 mMol) 4-Phenyl-1-butanol und 1,9 ml (24,4 mMol) Methansulfonylchlorid in 150 ml wasserfreiem Methylenchlorid und tropft bei 20 °C Reaktionstemperatur die Lösung von 5,5 ml (40 mMol) Triäthylamin in 30 ml wasserfreiem Methylenchlorid zu. Nach Rühren bei Raumtemperatur über Nacht schüttelt man nacheinander je einmal mit 2N-Salzsäure (gesättigt mit Natriumchlorid), mit gesättigter Natriumchlorid-Lösung und mit Wasser aus. Die getrocknete und filtrierte Methylenchlorid-Phase dampft man im Vakuum bei 30 °C ein.
Den Eindampfrückstand (rohes 4-Phenyl-1-butyl-mesylat) löst man in 75 ml Chloroform und tropft diese Lösung bei Raumtemperatur rasch zu 6,7 g (40 mMol) 2-Amino-4,5,7,8-tetrahydro-6H-thiazolo[5,4-d]azepin in 130 ml Chloroform. Man rührt 15 Stunden bei 60 °C, verdünnt mit 300 ml Chloroform und schüttelt einmal mit 1N-Natronlauge und zweimal mit Wasser aus. Die getrocknete und filtrierte organische Phase dampft man im Vakuum ein. Den Eindampfrückstand reinigt man durch Säulenchromatographie an Kieselgel (Chloroform/Methanol = 10:1).
Ausbeute: 1,28 g (21 % der Theorie),
Schmelzpunkt: 147-150 °C

| | | | | | | |
|---|---|---|---|---|---|---|
| Ber.:<br>Gef.: | C | 67,75<br>67,90 | H | 7,69<br>7,52 | N | 13,94<br>13,99 |

Analog Beispiel 20 wurde folgende Verbindung hergestellt:

20a) 2-Amino-6-(5-phenyl-1-pentyl)-4,5,7,8-tetrahydro-6H-thiazolo[5,4-d]azepin

Hergestellt aus 5-Phenyl-1-pentyl-mesylat und 2 Äquivalenten 2-Amino-4,5,7,8-tetrahydro-6H-thiazolo-[5,4-d]azepin in Chloroform.
Ausbeute: 19 % der Theorie,
Schmelzpunkt: 102-105 °C (Petroläther)

| | | | | | | |
|---|---|---|---|---|---|---|
| Ber.:<br>Gef.: | C | 68,54<br>67,99 | H | 7,99<br>7,88 | N | 13,32<br>13,61 |

Beispiel 21

2-Amino-6-(3-(2,4-dimethoxy-phenyl)-1-propyl)-4,5,7,8-tetrahydro-6H-thiazolo[5,4-d]azepin (A) und
2-Amino-6-(3-(2,4-dimethoxy-phenyl)-3-hydroxy-1-propyl)-4,5,7,8-tetrahydro-6H-thiazolo[5,4-d]azepin (B)

Zu einer Lösung von 5,0 g (13,8 mMol) 2-Amino-6-(3-(2,4-dimethoxy-phenyl)-3-oxo-1-propyl)-4,5,7,8-tetrahydro-6H-thiazolo[5,4-d]azepin [ölige Base (Schmelzpunkt des Dihydrochlorides 125-130°C); hergestellt aus 3-Chlor-1-(2,4-dimethoxy-phenyl)-1-oxo-propan mit 1 Äquivalent 2-Amino-4,5,7,8-tetrahydro-6H-thiazolo[5,4-d]azepinin wasserfreiem Dimethylformamid in Gegenwart von 1 Äquivalent Kaliumcarbonat (40°C, 2 Stunden)] in einem Gemisch aus 50 ml Tetrahydrofuran und 5 ml Wasser gibt man unter Rühren im Bad von 40°C 0,26 g (7 mMol) Natriumborhydrid und nach jeweils 30 Minuten zwei weitere Portionen à 0,26 g (7 mMol) Natriumborhydrid. Nach 2-tägigem Stehen bei Raumtemperatur stellt man mit 2N-Salzsäure sauer, rührt 30 Minuten und dampft dann im Vakuum nicht ganz zur Trockne ein. Man stellt mit konzentriertem Ammoniak alkalisch und extrahiert mit Chloroform. Die getrocknete und filtrierte Chloroform-Lösung dampft man im Vakuum ein. Den Eindampfrückstand reinigt man durch Säulenchromatographie an Kieselgel (Toluol/Äthylacetat/Methanol/konz.Ammoniak = 4:3:1:0,15).
Zunächst wird die Titelverbindung A eluiert.
Ausbeute: 0,13 g (2,6 % der Theorie),
Schmelzpunkt: 105-110°C (Äther)

| | | | | | | |
|---|---|---|---|---|---|---|
| Ber.: | C | 62,21 | H | 7,25 | N | 12,09 |
| Gef.: | | 62,11 | | 7,38 | | 11,95 |

Molpeak (m/z): Ber.: 347 Gef.: 347
Anschließend wird die Titelverbindung B eluiert.
Ausbeute: 0,46 g (9,2 % der Theorie),
Schmelzpunkt: 45-50°C (Schaum)
Molpeak (m/z): Ber.: 363 Gef.: 363

Beispiel 22

2-Amino-6-(3-(2,4-dimethoxy-phenyl)allyl)-4,5,7,8-tetrahydro-6H-thiazolo[5,4-d]azepin

0,45 g (1,2 mMol) 2-Amino-6-(3-(2,4-dimethoxy-phenyl)-3-hydroxy-1-propyl)-4,5,7,8-tetrahydro-6H-thiazolo[5,4-d]azepin rührt man in 35 ml wasserfreiem Toluol zusammen mit 0,47 g (2,7 mMol) p-Toluolsulfonsäure-hydrat und 7 g Molekularsieb (4Å) im Bad von 40°C. Nach einer Stunde gibt man weitere 5 g Molekularsieb, nach insgesamt 2 Stunden nochmals 2,5 g Molekularsieb zu. Nach insgesamt 3 Stunden bei 40°C filtriert man durch eine mit Celite beschichtete Glasfritte und wäscht den Filterkuchen mehrfach mit Chloroform aus. Die Toluol- und die Chloroform-Lösungen schüttelt man mit halbkonzentriertem Ammoniak und mit Wasser, trocknet und filtriert sie und dampft sie im Vakuum ein. Die vereinigten Eindampfrückstände reinigt man durch Säulenchromatographie an Kieselgel (Toluol/Äthylacetat/Methanol/konz. Ammoniak = 4:3:1:0,5).
Ausbeute: 43 mg (10 % der Theorie),
Schmelzpunkt: 95-100°C (Äther)

| | | | | | | |
|---|---|---|---|---|---|---|
| Ber.: | C | 62,59 | H | 6,71 | N | 12,17 |
| Gef.: | | 62,43 | | 6,96 | | 11,97 |

Molpeak (m/z): Ber.: 345 Gef.: 345

Beispiel 23

2-Amino-6-(3-(2,4-dimethoxy-phenyl)-3-hydroxy-1-propyl)-4,5,7,8-tetrahydro-6H-thiazolo[5,4-d]azepin (B) und 2-Amino-6-(3-(3-äthoxy-3-(2,4-dimethoxy-phenyl)-1-propyl)-4,5,7,8-tetrahydro-6H-thiazolo[5,4-d]azepin (C)

Hergestellt analog Beispiel 21 durch Reduktion von 2-Amino-6-(3-(2,4-dimethoxy-phenyl)-3-oxo-1-propyl)-4,5,7,8-tetrahy dro-6H-thiazolo[5,4-d]azepin mit Natriumborhydrid in Äthanol. - Reinigung durch Säulenchromatographie an Kieselgel (Toluol/Äthylacetat/Methanol/konz.Ammoniak = 4:3:1:0,15).
Zunächst wird die Titelverbindung C eluiert.
Ausbeute: 9 % der Theorie,
Schmelzpunkt: < 20 ° C
Molpeak (m/z): Ber.: 391 Gef.: 391
Die Base wird mit 2 Äquivalenten Fumarsäure in Aceton in das Bis-Fumarsäure-Salz von (C) vom Schmelzpunkt 158-160 ° C (Zers.) überführt.

| | | | | | | |
|---|---|---|---|---|---|---|
| Ber.:<br>Gef.: | C | 53,92<br>54,02 | H | 5,98<br>6,05 | N | 6,74<br>6,92 |

Anschließend wird die Titelverbindung B eluiert.
Ausbeute: 17 % der Theorie,
Schmelzpunkt: 40-50 ° C (Schaum)
Molpeak (m/z): Ber.: 363 Gef.: 363

Beispiel 24

2-Amino-6-(3-(2-chinolinyl)allyl)-4,5,7,8-tetrahydro-6H-thiazolo[5,4-d]azepin x 0,2 $H_20$

Zur Lösung von 3,1 g (16,7 mMol) 3-(2-Chinolinyl)allylalkohol in 20 ml Chloroform tropft man unter Rühren und Eiskühlung bei einer Reaktionstemperatur von 5 ° C die Lösung von 1,2 ml (16,7 mMol) Thionylchlorid in 10 ml Chloroform zu. Man rührt noch 15 Minuten und dampft im Vakuum bei 20 ° C ein. Den Eindampfrückstand [rohes 3-(2-Chinolinyl)allylchlorid-hydrochlorid] löst man bei Raumtemperatur in 50 ml wasserfreiem Dimethylformamid. Dazu gibt man 8,5 g (50 mMol) festes 2-Amino-4,5,7,8-tetrahydro-6H-thiazolo[5,4-d]azepin und rührt 12 Stunden bei Raumtemperatur.
Man dampft im Vakuum ein und verteilt den Eindampfrückstand zwischen Wasser und Chloroform. Die getrocknete und filtrierte Chloroform-Lösung dampft man im Vakuum ein. Den Eindampfrückstand reinigt man durch Säulenchromatographie an Kieselgel (Chloroform/Methanol = 10:1).
Ausbeute: 2,6 g (46 % der Theorie),
Schmelzpunkt: 165-170 ° C (Äther)

| | | | | | | | |
|---|---|---|---|---|---|---|---|
| Ber.:<br>Gef.: | (x 0,2 $H_2O$) | C | 67,10<br>67,11 | H | 6,04<br>6,03 | N | 16,56<br>16,45 |

Analog Beispiel 24 wurde folgende Verbindung hergestellt:

24a) 2-Amino-6-(3-(3-isochinolinyl)allyl)-4,5,7,8-tetrahydro-6H-thiazolo[5,4-d]azepin x 0,5 $H_2O$

Hergestellt aus 3-(3-Isochinolinyl)allylchlorid-hydrochlorid [gewonnen aus dem entsprechenden Allylalkohol mit Thionylchlorid in Chloroform bei Raumtemperatur] und 3 Äquivalenten 2-Amino-4,5,7,8-tetrahydro-6H-thiazolo[5,4-d]azepin in wasserfreiem Dimethylformamid während 4 Stunden bei 50 ° C.
Ausbeute: 45 % der Theorie,
Schmelzpunkt: 196-199 ° C (Äther)

| | | | | | | | |
|---|---|---|---|---|---|---|---|
| Ber.:<br>Gef.: | (x 0,5 $H_2O$) | C | 66,06<br>65,98 | H | 6,13<br>6,01 | N | 16,22<br>16,14 |

Beispiel 25

2-(3-(4-Methoxy-phenyl)propionyl-amino)-6-(3-(3-pyridyl)allyl)-4,5,7,8-tetrahydro-6H-thiazolo[5,4-d]azepin

Zum gerührten Gemisch von 1,0 g (3,5 mMol) 2-Amino-6-(3-(3-pyridyl)allyl)-4,5,7,8-tetrahydro-6H-thiazolo[5,4-d]azepin und 0,53 ml (3,8 mMol) Triäthylamin in 40 ml wasserfreiem Chloroform tropft man bei Raumtemperatur die Lösung von 3-(4-Methoxy-phenyl)-propionylchlorid (Kp: 150 °C/15 Torr) in 7 ml Chloroform. Man rührt 3 Stunden bei 60-70 °C, kühlt ab und schüttelt mit Wasser aus. Die getrocknete und filtrierte Chloroform-Lösung dampft man im Vakuum ein und reinigt den Eindampfrückstand durch Säulenchromatographie an Kieselgel (Chloroform/Methanol = 5:1).
Ausbeute: 0,74 g (47 % der Theorie),
Schmelzpunkt: 168-170 °C (Äther)

| | | | | | | |
|---|---|---|---|---|---|---|
| Ber.:<br>Gef.: | C | 66,95<br>66,74 | H | 6,29<br>6,21 | N | 12,49<br>12,51 |

Analog Beispiel 25 wurde folgende Verbindung hergestellt:

25a) 2-(3-(4-Methoxy-phenyl)propionyl-amino)-6-(3-(2-pyridyl)allyl)-4,5,7,8-tetrahydro-6H-thiazolo[5,4-d]-azepin x 0,5 HCl

Hergestellt durch Umsetzung von 2-Amino-6-(3-(2-pyridyl)allyl)-4,5,7,8-tetrahydro-6H-thiazolo[5,4-d]-azepin mit 3-(4-Methoxy-phenyl)-propionylchlorid.
Ausbeute: 42 % der Theorie,
Schmelzpunkt: 37-40 °C

| | | | | | | | | | |
|---|---|---|---|---|---|---|---|---|---|
| Ber.:<br>Gef.: | (x 0,5 HCl) | C | 64,33<br>64,11 | H | 6,15<br>6,03 | N | 12,00<br>11,98 | Cl | 6,86<br>6,90 |

Beispiel 26

2-Amino-6-(3-(4-isochinolinyl)-2-propin-1-yl)-4,5,7,8-tetrahydro-6H-thiazolo[5,4-d]azepin-Hydrat

Zu einer unter Stickstoff bei 50 °C gerührten Lösung von 0,50 g (2,4 mMol) 2-Amino-6-propargyl-4,5,7,8-tetrahydro-6H-thiazolo[5,4-d]azepin und 0,50 g (2,4 mMol) 4-Brom-isochinolin in 30 ml wasserfreiem Triäthylamin gibt man 11 mg (0,06 mMol) Kupfer(I)jodid und 42 mg (0,06 mMol) Bis(triphenylphosphin)-palladiumdichlorid. Man rührt 3 Stunden bei 50 °C und 2 Stunden bei 70 °C, wobei sich ein Niederschlag absetzt. Dann versetzt man mit weiteren 11 mg Kupfer(I) jodid, 42 mg Bis(triphenylphosphin)-palladiumdichlorid und 0,20 g (0,96 mMol) 4-Brom-isochinolin sowie mit 30 ml wasserfreiem Acetonitril, wodurch Lösung eintritt. Nach einer Stunde Rühren bei 70 °C ist die Ausgangsverbindung im Dünnschichtchromatogramm nicht mehr nachweisbar. Man dampft im Vakuum ein und verteilt den Eindampfrückstand zwischen Chloroform und Wasser. Die getrocknete und filtrierte Chloroform-Lösung dampft man im Vakum ein. Den Eindampfrückstand reinigt man durch Säulenchromatographie an Kieselgel (Aceton/Methanol/konz. Ammoniak = 50:12:0,4).
Ausbeute: 0,032 g (4 % der Theorie),
Schmelzpunkt: 138-142 °C (Äther)

| | | | | | | | |
|---|---|---|---|---|---|---|---|
| Ber.:<br>Gef.: | (x 1 $H_2O$) | C | 64,76<br>64,68 | H | 5,72<br>5,72 | N | 15,90<br>16,14 |

Molpeak (m/z) Ber.: 334 Gef.: 334
Analog Beispiel 26 wurde folgende Verbindung erhalten:

26a) 2-Amino-6-(3-(2-oxo-indolin-4-yl)-2-propin-1-yl)-4,5,7,8-tetrahydro-6H-thiazolo[5,4-d]azepin x 0,7 $H_2O$

Hergestellt aus 2-Amino-6-propargyl-4,5,7,8-tetrahydro-6H-thiazolo[5,4-d]azepin und 4-Brom-2-oxo-indolin [Schmelzpunkt: 216-220 °C] in Triäthylamin/Acetonitril (1:1) in Gegenwart von katalytischen Mengen Kupfer(I)jodid und Bis(triphenylphosphin)palladiumdichlorid in einer Parr-Apparatur unter Schütteln während 4 Stunden bei 95 bis 100 °C. Nach der säulenchromatographischen Reinigung an Kieselgel (Chloroform/Methanol/Eisessig = 10:1:0,03) wird zwischen Chloroform und gesättigter Natriumbicarbonatlösung verteilt.
Ausbeute: 9,7 % der Theorie,
Schmelzpunkt: 197-199 °C (Äther)

| | | | | | | | |
|---|---|---|---|---|---|---|---|
| Ber.:<br>Gef.: | x (0,75 $H_2O$) | C | 61,42<br>61,62 | H | 5,58<br>5,44 | N | 15,92<br>15,80 |

Molpeak (m/z) Ber.: 338 Gef.: 338

Beispiel 27

2-Amino-6-(3-(5-indolyl)allyl)-4,5,7,8-tetrahydro-6H-thiazolo[5,4-d]azepin

Zur gerührten Lösung von 95 mg (0,55 mMol) 3-(5-Indolyl)allyl-alkohol und 142 mg (1,10 mMol) N-Äthyl-N,N-diisopropylamin in 6 ml wasserfreien Methylenchlorid tropft man bei -15 °C die Lösung von 63 mg (0,55 mMol) Methansulfochlorid in 6 ml Chloroform und rührt 15 Stunden bei -5 °C. Man dampft im Vakuum bei 20 °C ein, löst den Eindampfrückstand in 3 ml wasserfreiem Dimethylformamid, versetzt mit 370 mg (2,20 mMol) 2-Amino-4,5,7,8-tetrahydro-6H-thiazolo[5,4-d]azepin und rührt 4 Stunden bei 50 °C und 3 Stunden bei 70 °C. Man dampft im Vakuum ein, verteilt zwischen Chloroform und Wasser und dampft die getrocknete und filtrierte Chloroformlösung im Vakuum ein. Den Eindampfrückstand reinigt man durch 3-fache Säulenchromatographie an neutralem Aluminiumoxid (Toluol/Äthylacetat/Äthanol = 4:1:0,5).
Ausbeute: 8 mg (4,5 % der Theorie),
Schmelzpunkt: 60-65 °C (schaumartig)
Molpeak (m/z) Ber.: 324 Gef.: 324

Beispiel 28

2-Amino-6-(3-(2-brom-phenyl)allyl)-4,5,7,8-tetrahydro-6H-thiazolo[5,4-d]azepin

Hergestellt analog Beispiel 1 aus 2-Amino-4,5,7,8-tetrahydro-6H-thiazolo[5,4-d]azepin, Kaliumcarbonat und 2-Brom-cinnamylbromid in wasserfreiem Dimethylformamid während 2 Stunden bei 20 °C.
Ausbeute: 42 % der Theorie,
Schmelzpunkt: 103-106 °C (Diisopropyläther)

| | | | | | | | | |
|---|---|---|---|---|---|---|---|---|
| Ber.:<br>Gef.: | C | 52,75<br>52,84 | H | 4,98<br>4,98 | Br | 21,94<br>22,14 | N | 11,54<br>11,53 |

Beispiel 29

2-Amino-6-(3-(3-trifluormethyl-phenyl)allyl)-4,5,7,8-tetrahydro-6H-thiazolo[5,4-d]azepin

Hergestellt analog Beispiel 1 aus 2-Amino-4,5,7,8-tetrahydro-6H-thiazolo[5,4-d]azepin, Kaliumcarbonat und 3-Trifluormethyl-cinnamylbromid in wasserfreiem Dimethylformamid während einer Stunde bei 20 °C.
Ausbeute: 41 % der Theorie,
Schmelzpunkt: 102-105 °C (Petroläther)

| | | | | | | | | |
|---|---|---|---|---|---|---|---|---|
| Ber.:<br>Gef.: | C | 57,77<br>57,91 | H | 5,13<br>5,08 | N | 11,89<br>11,97 | S | 9,07<br>9,38 |

Beispiel 30

2-Amino-6-(3-(2-cyano-phenyl)allyl)-4,5,7,8-tetrahydro-6H-thiazolo[5,4-d]azepin

Hergestellt analog Beispiel 1 aus 2-Amino-4,5,7,8-tetrahydro-6H-thiazolo[5,4-d]azepin, Kaliumcarbonat und 2-Cyano-cinnamylbromid (Schmelzpunkt: 69-71 ° C) in wasserfreiem Dimethylformamid während 15 Stunden bei 20 ° C.
Ausbeute: 31 % der Theorie,
Schmelzpunkt: 140-144 ° C (Aceton)

| | | | | | | |
|---|---|---|---|---|---|---|
| Ber.:<br>Gef.: | C | 65,79<br>65,74 | H | 5,85<br>5,75 | N | 18,05<br>18,10 |

Beispiel 31

2-Amino-6-(3-(3-cyano-phenyl)allyl)-4,5,7,8-tetrahydro-6H-thiazolo[5,4-d]azepin

Hergestellt analog Beispiel 1 aus 2-Amino-4,5,7,8-tetrahydro-6H-thiazolo[5,4-d]azepin, Kaliumcarbonat und 3-Cyano-cinnamylbromid (Schmelzpunkt: 52-55 ° C) in wasserfreiem Dimethylformamid während 2 Stunden bei 20 ° C.
Ausbeute: 48 % der Theorie,
Schmelzpunkt: 136-140 ° C (Äther)

| | | | | | | |
|---|---|---|---|---|---|---|
| Ber.:<br>Gef.: | C | 65,79<br>65,54 | H | 5,85<br>5,88 | N | 18,05<br>18,27 |

Beispiel 32

(E)-2-Amino-6-(3-(4-cyano-phenyl)allyl)-4,5,7,8-tetrahydro-6H-thiazolo[5,4-d]azepin

Hergestellt analog Beispiel 1 aus 2-Amino-4,5,7,8-tetrahydro-6H-thiazolo[5,4-d]azepin, Kaliumcarbonat und 4-Cyano-cinnamylbromid (Schmelzpunkt: 79-82 ° C) in wasserfreiem Dimethylformamid während einer Stunde bei 20 ° C. Nach dem Abdestillieren des Dimethylformamids im Vakuum wird der Eindampfrückstand zwischen Äthylacetat und Wasser verteilt. Nach Trocknen, Filtrieren und Eindampfen der organischen Phase kristallisiert man den Eindampfrückstand aus Aceton und wäscht das Kristallisat mit Äther.
Ausbeute: 49 % der Theorie,
Schmelzpunkt: 199-205 ° C (Zers.)

| | | | | | | |
|---|---|---|---|---|---|---|
| Ber.:<br>Gef.: | C | 65,79<br>65,51 | H | 5,85<br>6,02 | N | 18,05<br>18,12 |

200 MHz-$^1$H-NMR-Spektrum ($CDCl_3$):
$\delta$ = 3,40 ppm (Dublett), 2H (allylisches $CH_2$)
$\delta$ = 6,40 ppm (Triplett) und
$\delta$ = 6,48 ppm (Triplett), 1H (olefinisches H)
$\delta$ = 6,58 ppm (Dublett), 1H (olefinisches H)

Beispiel 33

(E)-2-Amino-6-(3-(4-cyano-phenyl)allyl)-4,5,7,8-tetrahydro-6H-thiazolo[5,4-d]azepin-hydrochlorid

Zu 0,70 g (2,25 mMol) (E)-2-Amino-6-(3-(4-cyano-phenyl)allyl)-4,5,7,8-tetrahydro-6H-thiazolo[5,4-d]-azepin in 10 ml Äthanol gibt man 2,25 ml 1N-Salzsäure und erhitzt, bis die Lösung klar ist. Anschließend kühlt man im Eisbad ab. Vom langsam ausgefallenen Kristallisat filtriert man ab. Nach Waschen mit Äthanol und mit Äther und nach Trocknen bei 100°/4 Torr erhält man das Monohydrochlorid.
Ausbeute: 0,46 g (59 % der Theorie),
Schmelzpunkt: 237°C (Zers.)

| | | | | | | | | |
|---|---|---|---|---|---|---|---|---|
| Ber.:<br>Gef.: | C | 58,85<br>58,67 | H | 5,52<br>5,45 | Cl | 10,22<br>10,18 | N | 16,15<br>16,05 |

Beispiel 34

(E)-2-Amino-6-(3-(4-cyano-phenyl)allyl)-4,5,7,8-tetrahydro-6H-thiazolo[5,4-d]azepin-dihydrochlorid x 0,25 $H_2O$

Zu 0,70 g (2,25 mMol) (E)-2-Amino-6-(3-(4-cyano-phenyl)allyl)-4,5,7,8-tetrahydro-6H-thiazolo[5,4-d]-azepin in 10 ml Äthanol gibt man 4,50 ml 1N-Salzsäure und erhitzt, bis die Lösung klar ist. Anschließend dampft man im Vakuum zur Trockne ein, versetzt den schaumartigen Rückstand mit Äthanol, dampft erneut ein und wiederholt diese Prozedur mit Äthanol noch dreimal. Danach löst man in Äthanol unter Erhitzen und kühlt in Eis ab. Man filtriert vom Kristallisat ab und trocknet bei 100°C/4 Torr.
Ausbeute: 0,70 g (81 % der Theorie),
Schmelzpunkt: 247-250°C (Zers.)

| | | | | | | | | | |
|---|---|---|---|---|---|---|---|---|---|
| Ber.:<br>Gef.: | x (0,25 $H_2O$) | C | 52,65<br>52,63 | H | 5,30<br>5,12 | Cl | 18,29<br>18,39 | N | 14,40<br>14,55 |

Beispiel 35

2-Amino-6-(3-(4-cyano-phenyl)-2-propin-1-yl)-4,5,7,8-tetrahydro-6H-thiazolo[5,4-d]azepin x 0,3 $H_2O$

Hergestellt analog Beispiel 10 aus 2-Amino-6-propargyl-4,5,7,8-tetrahydro-6H-thiazolo[5,4-d]azepin, 4-Brom-benzonitril und Kupfer(I)jodid/Bis(triphenylphosphin)palladiumdichlorid in Diäthylamin während 6 Stunden bei Raumtemperatur.
Ausbeute: 46 % der Theorie,
Schmelzpunkt: 174-176°C (Aceton)

| | | | | | | | |
|---|---|---|---|---|---|---|---|
| Ber.:<br>Gef.: | x (0,3 $H_2O$) | C | 64,90<br>64,86 | H | 5,34<br>5,29 | N | 17,83<br>17,76 |

Beispiel 36

(Z)-2-Amino-6-(3-(4-cyano-phenyl)allyl)-4,5,7,8-tetrahydro-6H-thiazolo[5,4-d]azepin

Hergestellt analog Beispiel 12 durch katalytische Hydrierung von 1,30 g (4,21 mMol) 2-Amino-6-(3-(4-cyano-phenyl)-2-propin-1-yl)-4,5,7,8-tetrahydro-6H-thiazolo[5,4-d]azepin x 0,3 $H_2O$ in wasserfreiem Dimethylformamid bei 1 bar Wasserstoffdruck an Lindlar-Katalysator [Palladium (5 %) auf Calciumcarbonat, vergiftet mit Blei] bei Raumtemperatur.
Ausbeute: 29,5 % der Theorie,

Schmelzpunkt: 186-188 °C (Äther)

| | | | | | | |
|---|---|---|---|---|---|---|
| Ber.:<br>Gef.: | C | 65,79<br>65,68 | H | 5,85<br>5,84 | N | 18,05<br>18,07 |

200 MHz-$^{1}$H-NMR-Spektrum (d6-DMSO):
δ = 3,42 ppm (Dublett), 2H (allylisches $CH_2$)
δ = 5,95 ppm (Triplett) und
δ = 6,01 ppm (Triplett), J = 11,7 Hz, 1H (olefinisches H)
δ = 6,62 ppm (Dublett), 1H (olefinisches H)

Beispiel 37

2-Amino-6-(3-(2-aminocarbonyl-phenyl)allyl)-4,5,7,8-tetrahydro-6H-thiazolo[5,4-d]azepin x 0,25 HCl

Hergestellt analog Beispiel 18 aus 2-Amino-6-(3-(2-cyano-phenyl)allyl)-4,5,7,8-tetrahydro-6H-thiazolo-[5,4-d]azepin durch vierstündiges Erhitzen in Polyphosphorsäure im Bad von 100 °C.
Ausbeute: 58 % der Theorie,
Schmelzpunkt: 185-188 °C

| | | | | | | | | | |
|---|---|---|---|---|---|---|---|---|---|
| Ber.<br>Gef.: | (x 0,25 HCl): | C | 60,55<br>60,30 | H | 6,05<br>5,96 | Cl | 2,63<br>2,24 | N | 16,61<br>16,45 |

Beispiel 38

2-Amino-6-(3-(3-aminocarbonyl-phenyl)allyl)-4,5,7,8-tetrahydro-6H-thiazolo[5,4-d]azepin x 0,25 $H_2O$

Hergestellt analog Beispiel 18 aus 2-Amino-6-(3-(3-cyano-phenyl)allyl)-4,5,7,8-tetrahydro-6H-thiazolo-[5,4-d]azepin durch 1,5 Stunden Erhitzen in Polyphosphorsäure bei 100 °C.
Ausbeute: 69 % der Theorie,
Schmelzpunkt: 210-212 °C

| | | | | | | |
|---|---|---|---|---|---|---|
| Ber.:<br>Gef.: | C | 61,33<br>61,54 | H | 6,21<br>5,98 | N | 16,83<br>16,62 |

Beispiel 39

2-Amino-6-(3-(3-(2-pyridylmethoxy)phenyl)allyl)-4,5,7,8-tetrahydro-6H-thiazolo[5,4-d]azepin

Zu 100mg (0,33 mMol) 2-Amino-6-(3-(3-hydroxy-phenyl)allyl)-4,5,7,8-tetrahydro-6H-thiazolo[5,4-d]azepin und 36 mg (0,33 mMol) 2-(Hydroxymethyl)-pyridin und 86 mg (0,33 mMol) Triphenylphosphin in 1 ml wasserfreiem Tetrahydrofuran tropft man unter Rühren und Kühlen (Innentemperatur 0 °C) die Lösung von 57 mg (0,33 mMol) Azodicarbonsäure-diäthylester in 0,15 ml wasserfreiem Tetrahydrofuran. Nach Rühren über Nacht unter Erwärmen auf Raumtemperatur gibt man bei 0 °C weitere 0,33 mMol von Triphenylphosphin und Azodicarbonsäure-diäthylester zu und rührt eine weitere Stunde bei Raumtemperatur. Man dampft im Vakuum ein und verteilt zwischen Äthylacetat und Wasser. Die Äthylacetat-Phase extrahiert man mit 2N-Salzsäure. Die salzsaure wäßrige Phase wird mit konz. Ammoniak alkalisiert und mit Äthylacetat extrahiert; die organische Phase wird getrocknet, filtriert und im Vakuum eingedampft. Den Eindampfrückstand reinigt man durch Säulenchromatographie an Kieselgel (Chloroform/Methanol = 5/1).
Ausbeute: 13 mg (10 % der Theorie),
Schmelzpunkt: 35-40 °C (schaumartig)
Molpeak (m/z): Ber.: 392 Gef.: 392

Beispiel 40

(Z)-2-Amino-6-(3-(2-oxo-indolin-4-yl)-2-propen-1-yl)-4,5,7,8-tetrahydro-6H-thiazolo[5,4-d]azepin

Hergestellt analog Beispiel 12 durch katalytische Hydrierung von 2-Amino-6-(3-(2-oxo-indolin-4-yl)-2-propin-1-yl)-4,5,7,8-tetrahydro-6H-thiazolo[5,4-d]azepin x 0,75 $H_2O$ an Lindlar-Katalysator (Palladium auf Calciumcarbonat, vergiftet mit Blei) 3 Stunden bei Raumtemperatur in Äthanol.
Ausbeute: 51 % der Theorie,
Schmelzpunkt: 192-195 °C (Aceton/Äther)

| | | | | | | |
|---|---|---|---|---|---|---|
| Ber.:<br>Gef.: | C | 63,52<br>63,38 | H | 5,92<br>6,01 | N | 16,46<br>16,29 |

Molpeak (m/z): Ber.: 340 Gef.: 340
200 MHz-$^1$H-NMR-Spektrum (d6-DMSO/$CD_3OD$):
$\delta$ = 3,38 ppm (Dublett), 2H (allylisches $CH_2$)
$\delta$ = 5,82 ppm (Triplett) und
$\delta$ = 5,88 ppm (Triplett), J = 12 Hz 1H (olefinisches H)
$\delta$ = 6,49 ppm (Dublett), 1H (olefinisches H)

Beispiel 41

2-Amino-6-(2H-1-benzopyran-3-yl)methyl-4,5,7,8-tetrahydro-6H-thiazolo[5,4-d]azepin

Hergestellt analog Beispiel 1 aus 2-Amino-4,5,7,8-tetrahydro-6H-thiazolo[5,4-d]azepin, Kaliumcarbonat und 3-Brommethyl-2H-1-benzopyran in wasserfreiem Dimethylformamid während 72 Stunden bei Raumtemperatur.
Ausbeute: 13 % der Theorie,
Schmelzpunkt: 150-153 °C (Petroläther/Äther)
Molpeak (m/z): Ber.: 313 Gef.: 313

Beispiel 42

2-Amino-6-(3-(3-benzothiophenyl)allyl-4,5,7,8-tetrahydro-6H-thiazolo[5,4-d]azepin

Hergestellt analog Beispiel 1 aus 2-Amino-4,5,7,8-tetrahydro-6H-thiazolo[5,4-d]azepin, Kaliumcarbonat und 3-(3-Benzothiophenyl)allylbromid in wasserfreiem Dimethylformamid während 15 Stunden bei Raumtemperatur.
Ausbeute: 12 % der Theorie,
Schmelzpunkt: 148-152 °C
Molpeak (m/z): Ber.: 341 Gef.: 341

Beispiel 43

2-Amino-6-(3-(3(2)-hydroxy-2(3)-methoxy-phenyl)allyl-4,5,7,8-tetrahydro-6H-thiazolo[5,4-d]azepin

Hergestellt analog Beispiel 15 aus 2-Amino-6-(3-(2,3-dimethoxy-phenyl)allyl-4,5,7,8-tetrahydro-6H-thiazolo[5,4-d]azepin mit 2 Äquivalenten Bortribromid in Methylenchlorid.
Ausbeute: 29 % der Theorie,
Schmelzpunkt: 50-55 °C
Molpeak (m/z): Ber.: 331 Gef.: 331
Analog den vorstehenden Beispielen können folgende Verbindungen hergestellt werden:
2-Amino-6-(3-(2-trifluormethyl-phenyl)allyl)-4,5,7,8-tetrahydro-6H-thiazolo[5,4-d]azepin
2-Amino-6-(3-(4-trifluormethyl-phenyl)allyl)-4,5,7,8-tetrahydro-6H-thiazolo[5,4-d]azepin
2-Amino-6-(3-(2-äthoxy-phenyl)allyl)-4,5,7,8-tetrahydro-6H-thiazolo[5,4-d]azepin
2-Amino-6-(3-(3-äthoxy-phenyl)allyl)-4,5,7,8-tetrahydro-6H-thiazolo[5,4-d]azepin
2-Amino-6-(3-(4-äthoxy-phenyl)allyl)-4,5,7,8-tetrahydro-6H-thiazolo[5,4-d]azepin

2-Amino-6-(3-(2-dimethylamino-phenyl)allyl)-4,5,7,8-tetrahydro-6H-thiazolo[5,4-d]azepin
2-Amino-6-(3-(3-dimethylamino-phenyl)allyl)-4,5,7,8-tetrahydro-6H-thiazolo[5,4-d]azepin
2-Amino-6-(3-(3-piperidino-phenyl)allyl)-4,5,7,8-tetrahydro-6H-thiazolo[5,4-d]azepin
2-Amino-6-(3-(4-piperidino-phenyl)allyl)-4,5,7,8-tetrahydro-6H-thiazolo[5,4-d]azepin
2-Amino-6-(3-(2-acetylamino-phenyl)allyl)-4,5,7,8-tetrahydro-6H-thiazolo[5,4-d]azepin
2-Amino-6-(3-(3-acetylamino-phenyl)allyl)-4,5,7,8-tetrahydro-6H-thiazolo[5,4-d]azepin
2-Amino-6-(3-(4-acetylamino-phenyl)allyl)-4,5,7,8-tetrahydro-6H-thiazolo[5,4-d]azepin
2-Amino-6-(3-(2-carboxy-phenyl)allyl)-4,5,7,8-tetrahydro-6H-thiazolo[5,4-d]azepin
2-Amino-6-(3-(3-carboxy-phenyl)allyl)-4,5,7,8-tetrahydro-6H-thiazolo[5,4-d]azepin
2-Amino-6-(3-(4-carboxy-phenyl)allyl)-4,5,7,8-tetrahydro-6H-thiazolo[5,4-d]azepin
2-Amino-6-(3-(2-(2-pyridylmethoxy)phenyl)allyl)-4,5,7,8-tetrahydro-6H-thiazolo[5,4-d]azepin
2-Amino-6-(3-(4-(2-pyridylmethoxy)phenyl)allyl)-4,5,7,8-tetrahydro-6H-thiazolo[5,4-d]azepin
2-Amino-6-(3-(2-(3-pyridylmethoxy)phenyl)allyl)-4,5,7,8-tetrahydro-6H-thiazolo[5,4-d]azepin
2-Amino-6-(3-(3-(3-pyridylmethoxy)phenyl)allyl)-4,5,7,8-tetrahydro-6H-thiazolo[5,4-d]azepin
2-Amino-6-(3-(4-(3-pyridylmethoxy)phenyl)allyl)-4,5,7,8-tetrahydro-6H-thiazolo[5,4-d]azepin
2-Amino-6-(3-(2-(4-pyridylmethoxy)phenyl)allyl-4,5,7,8-tetrahydro-6H-thiazolo[5,4-d]azepin
2-Amino-6-(3-(3-(4-pyridylmethoxy)phenyl)allyl)-4,5,7,8-tetrahydro-6H-thiazolo[5,4-d]azepin
2-Amino-6-(3-(4-(4-pyridylmethoxy)phenyl)allyl)-4,5,7,8-tetrahydro-6H-thiazolo[5,4-d]azepin
2-Amino-6-(3-(4-chinolinyl)allyl)-4,5,7,8-tetrahydro-6H-thiazolo[5,4-d]azepin
2-Amino-6-(3-(5-chinolinyl)allyl)-4,5,7,8-tetrahydro-6H-thiazolo[5,4-d]azepin
2-Amino-6-(3-(6-chinolinyl)allyl)-4,5,7,8-tetrahydro-6H-thiazolo[5,4-d]azepin
2-Amino-6-(3-(7-chinolinyl)allyl)-4,5,7,8-tetrahydro-6H-thiazolo[5,4-d]azepin
2-Amino-6-(3-(8-chinolinyl)allyl)-4,5,7,8-tetrahydro-6H-thiazolo[5,4-d]azepin
2-Amino-6-(3-(5-isochinolinyl)allyl)-4,5,7,8-tetrahydro-6H-thiazolo[5,4-d]azepin
2-Amino-6-(3-(6-isochinolinyl)allyl)-4,5,7,8-tetrahydro-6H-thiazolo[5,4-d]azepin
2-Amino-6-(3-(7-isochinolinyl)allyl)-4,5,7,8-tetrahydro-6H-thiazolo[5,4-d]azepin
2-Amino-6-(3-(8-isochinolinyl)allyl)-4,5,7,8-tetrahydro-6H-thiazolo[5,4-d]azepin
2-Amino-6-(3-(indolin-2-on-4-yl)-allyl)-4,5,7,8-tetrahydro-6H-thiazolo[5,4-d]azepin
2-Amino-6-(3-indolin-2-on-5-yl)-allyl)-4,5,7,8-tetrahydro-6H-thiazolo[5,4-d]azepin
2-Amino-6-(3-indolin-2-on-6-yl)-allyl)-4,5,7,8-tetrahydro-6H-thiazolo[5,4-d]azepin
2-Amino-6-(3-indolin-2-on-7-yl)-allyl)-4,5,7,8-tetrahydro-6H-thiazolo[5,4-d]azepin
2-Amino-6-(3-(5-carbostyril)allyl)-4,5,7,8-tetrahydro-6H-thiazolo[5,4-d]azepin
2-Amino-6-(3-(6-carbostyril)allyl)-4,5,7,8-tetrahydro-6H-thiazolo[5,4-d]azepin
2-Amino-6-(3-(3,4-dihydro-5-carbostyril)allyl)-4,5,7,8-tetrahydro-6H-thiazolo[5,4-d]azepin
2-Amino-6-(3-(3,4-dihydro-6-carbostyril)allyl)-4,5,7,8-tetrahydro-6H-thiazolo[5,4-d]azepin
2-Amino-6-(3-(2-methyl-4-benzothiazolyl)allyl)-4,5,7,8-tetrahydro-6H-thiazolo[5,4-d]azepin
2-Amino-6-(3-(2-methyl-5-benzothiazolyl)allyl)-4,5,7,8-tetrahydro-6H-thiazolo[5,4-d]azepin
2-Amino-6-(3-(2-methyl-6-benzothiazolyl)allyl)-4,5,7,8-tetrahydro-6H-thiazolo[5,4-d]azepin
2-Amino-6-(3-(2-methyl-7-benzothiazolyl)allyl)-4,5,7,8-tetrahydro-6H-thiazolo[5,4-d]azepin
2-Amino-6-(3-(2-amino-4-benzothiazolyl)allyl)-4,5,7,8-tetrahydro-6H-thiazolo[5,4-d]azepin
2-Amino-6-(3-(2-amino-5-benzothiazolyl)allyl)-4,5,7,8-tetrahydro-6H-thiazolo[5,4-d]azepin
2-Amino-6-(3-(2-amino-6-benzothiazolyl)allyl)-4,5,7,8-tetrahydro-6H-thiazolo[5,4-d]azepin
2-Amino-6-(3-(2-amino-7-benzothiazolyl)allyl)-4,5,7,8-tetrahydro-6H-thiazolo[5,4-d]azepin
2-Amino-6-(3-(2-methyl-4-benzoxazolyl)allyl)-4,5,7,8-tetrahydro-6H-thiazolo[5,4-d]azepin
2-Amino-6-(3-(2-methyl-5-benzoxazolyl)allyl)-4,5,7,8-tetrahydro-6H-thiazolo[5,4-d]azepin
2-Amino-6-(3-(2-methyl-4-benzimidazolyl)allyl)-4,5,7,8-tetrahydro-6H-thiazolo[5,4-d]azepin
2-Amino-6-(3-(2-methyl-5-benzimidazolyl)allyl)-4,5,7,8-tetrahydro-6H-thiazolo[5,4-d]azepin
2-Amino-6-(3-(2-phenyl-4-benzimidazolyl)allyl)-4,5,7,8-tetrahydro-6H-thiazolo[5,4-d]azepin
2-Amino-6-(3-(2-phenyl-5-benzimidazolyl)allyl)-4,5,7,8-tetrahydro-6H-thiazolo[5,4-d]azepin
2-Amino-6-(3-(2-amino-4-benzimidazolyl)allyl)-4,5,7,8-tetrahydro-6H-thiazolo[5,4-d]azepin
2-Amino-6-(3-(2-amino-5-benzimidazolyl)allyl)-4,5,7,8-tetrahydro-6H-thiazolo[5,4-d]azepin
2-Amino-6-(3-(2,3-dihydroxy-phenyl)allyl)-4,5,7,8-tetrahydro-6H-thiazolo[5,4-d]azepin
2-Amino-6-(3-(2,5-dihydroxy-phenyl)allyl)-4,5,7,8-tetrahydro-6H-thiazolo[5,4-d]azepin
2-Amino-6-(3-(2,6-dihydroxy-phenyl)allyl)-4,5,7,8-tetrahydro-6H-thiazolo[5,4-d]azepin
2-Amino-6-(3-(3,4-dihydroxy-phenyl)allyl)-4,5,7,8-tetrahydro-6H-thiazolo[5,4-d]azepin
2-Amino-6-(3-(3,5-dihydroxy-phenyl)allyl)-4,5,7,8-tetrahydro-6H-thiazolo[5,4-d]azepin
2-Amino-6-(3-(2-hydroxy-3-methyl-phenyl)allyl)-4,5,7,8-tetrahydro-6H-thiazolo[5,4-d]azepin
2-Amino-6-(3-(2-hydroxy-4-methyl-phenyl)allyl)-4,5,7,8-tetrahydro-6H-thiazolo[5,4-d]azepin

2-Amino-6-(3-(2-hydroxy-5-methyl-phenyl)allyl)-4,5,7,8-tetrahydro-6H-thiazolo[5,4-d]azepin
2-Amino-6-(3-(2-hydroxy-6-methyl-phenyl)allyl)-4,5,7,8-tetrahydro-6H-thiazolo[5,4-d]azepin
2-Amino-6-(3-(3-hydroxy-2-methyl-phenyl)allyl)-4,5,7,8-tetrahydro-6H-thiazolo[5,4-d]azepin
2-Amino-6-(3-(3-hydroxy-4-methyl-phenyl)allyl)-4,5,7,8-tetrahydro-6H-thiazolo[5,4-d]azepin
2-Amino-6-(3-(3-hydroxy-5-methyl-phenyl)allyl)-4,5,7,8-tetrahydro-6H-thiazolo[5,4-d]azepin
2-Amino-6-(3-(5-hydroxy-2-methyl-phenyl)allyl)-4,5,7,8-tetrahydro-6H-thiazolo[5,4-d]azepin
2-Amino-6-(3-(2-hydroxy-3-methoxy-phenyl)allyl)-4,5,7,8-tetrahydro-6H-thiazolo[5,4-d]azepin
2-Amino-6-(3-(2-hydroxy-4-methoxy-phenyl)allyl)-4,5,7,8-tetrahydro-6H-thiazolo[5,4-d]azepin
2-Amino-6-(3-(2-hydroxy-5-methoxy-phenyl)allyl)-4,5,7,8-tetrahydro-6H-thiazolo[5,4-d]azepin
2-Amino-6-(3-(2-hydroxy-6-methoxy-phenyl)allyl)-4,5,7,8-tetrahydro-6H-thiazolo[5,4-d]azepin
2-Amino-6-(3-(3-hydroxy-2-methoxy-phenyl)allyl)-4,5,7,8-tetrahydro-6H-thiazolo[5,4-d]azepin
2-Amino-6-(3-(3-hydroxy-4-methoxy-phenyl)allyl)-4,5,7,8-tetrahydro-6H-thiazolo[5,4-d]azepin
2-Amino-6-(3-(3-hydroxy-5-methoxy-phenyl)allyl)-4,5,7,8-tetrahydro-6H-thiazolo[5,4-d]azepin
2-Amino-6-(3-(5-hydroxy-2-methoxy-phenyl)allyl)-4,5,7,8-tetrahydro-6H-thiazolo[5,4-d]azepin
2-Amino-6-(3-(3-benzyloxy-2-methyl-phenyl)allyl)-4,5,7,8-tetrahydro-6H-thiazolo[5,4-d]azepin
2-Amino-6-(3-(3-benzyloxy-4-methyl-phenyl)allyl)-4,5,7,8-tetrahydro-6H-thiazolo[5,4-d]azepin
2-Amino-6-(3-(3-benzyloxy-5-methyl-phenyl)allyl)-4,5,7,8-tetrahydro-6H-thiazolo[5,4-d]azepin
2-Amino-6-(3-(5-benzyloxy-2-methyl-phenyl)allyl)-4,5,7,8-tetrahydro-6H-thiazolo[5,4-d]azepin
2-Amino-6-(3-(3-benzyloxy-2-methoxy-phenyl)allyl)-4,5,7,8-tetrahydro-6H-thiazolo[5,4-d]azepin
2-Amino-6-(3-(3-benzyloxy-4-methoxy-phenyl)allyl)-4,5,7,8-tetrahydro-6H-thiazolo[5,4-d]azepin
2-Amino-6-(3-(3-benzyloxy-5-methoxy-phenyl)allyl)-4,5,7,8-tetrahydro-6H-thiazolo[5,4-d]azepin
2-Amino-6-(3-(5-benzyloxy-2-methoxy-phenyl)allyl)-4,5,7,8-tetrahydro-6H-thiazolo[5,4-d]azepin
2-Amino-6-(3-(3,4,5-trihydroxy-phenyl)allyl)-4,5,7,8-tetrahydro-6H-thiazolo[5,4-d]azepin
2-Amino-6-(3-(4-methyl-2-pyridyl)allyl)-4,5,7,8-tetrahydro-6H-thiazolo[5,4-d]azepin
2-Amino-6-(3-(5-chlor-2-pyridyl)allyl)-4,5,7,8-tetrahydro-6H-thiazolo[5,4-d]azepin
2-Amino-6-(3-(3-methoxy-2-pyridyl)allyl)-4,5,7,8-tetrahydro-6H-thiazolo[5,4-d]azepin
2-Amino-6-(3-(3-benzyloxy-2-pyridyl)allyl)-4,5,7,8-tetrahydro-6H-thiazolo[5,4-d]azepin
2-Amino-6-(3-(6-benzyloxy-2-pyridyl)allyl)-4,5,7,8-tetrahydro-6H-thiazolo[5,4-d]azepin
2-Amino-6-(3-(2-methoxy-3-pyridyl)allyl)-4,5,7,8-tetrahydro-6H-thiazolo[5,4-d]azepin
2-Amino-6-(3-(2-methoxy-phenyl)-2-propin-1-yl)-4,5,7,8-tetrahydro-6H-thiazolo[5,4-d]azepin
2-Amino-6-(3-(3-methoxy-phenyl)-2-propin-1-yl)-4,5,7,8-tetrahydro-6H-thiazolo[5,4-d]azepin
2-Amino-6-(3-(4-methoxy-phenyl)-2-propin-1-yl)-4,5,7,8-tetrahydro-6H-thiazolo[5,4-d]azepin
2-Amino-6-(3-(2-benzyloxy-phenyl)-2-propin-1-yl)-4,5,7,8-tetrahydro-6H-thiazolo[5,4-d]azepin
2-Amino-6-(3-(3-benzyloxy-phenyl)-2-propin-1-yl)-4,5,7,8-tetrahydro-6H-thiazolo[5,4-d]azepin
2-Amino-6-(3-(4-benzyloxy-phenyl)-2-propin-1-yl)-4,5,7,8-tetrahydro-6H-thiazolo[5,4-d]azepin
2-Amino-6-(3-(5-chlor-2-pyridyl)-2-propin-1-yl)-4,5,7,8-tetrahydro-6H-thiazolo[5,4-d]azepin
2-Amino-6-(3-(1-naphthyl)-2-propin-1-yl)-4,5,7,8-tetrahydro-6H-thiazolo[5,4-d]azepin
2-Amino-6-(3-(2-naphthyl)-2-propin-1-yl)-4,5,7,8-tetrahydro-6H-thiazolo[5,4-d]azepin
2-Amino-6-(3-(2-chinolyl)-2-propin-1-yl)-4,5,7,8-tetrahydro-6H-thiazolo[5,4-d]azepin
2-Amino-6-(3-(3-chinolyl)-2-propin-1-yl)-4,5,7,8-tetrahydro-6H-thiazolo[5,4-d]azepin
2-Amino-6-(3-(4-chinolyl)-2-propin-1-yl)-4,5,7,8-tetrahydro-6H-thiazolo[5,4-d]azepin
2-Amino-6-(3-(1-isochinolyl)-2-propin-1-yl)-4,5,7,8-tetrahydro-6H-thiazolo[5,4-d]azepin
2-Amino-6-(3-(3-isochinolyl)-2-propin-1-yl)-4,5,7,8-tetrahydro-6H-thiazolo[5,4-d]azepin
2-Amino-6-(2-(1-naphthyl)-1-cyclopropyl-methyl)-4,5,7,8-tetrahydro-6H-thiazolo[5,4-d]azepin
2-Amino-6-(2-(2-naphthyl)-1-cyclopropyl-methyl)-4,5,7,8-tetrahydro-6H-thiazolo[5,4-d]azepin
2-Amino-6-(2-(2-pyridyl)-1-cyclopropyl-methyl)-4,5,7,8-tetrahydro-6H-thiazolo[5,4-d]azepin
2-Amino-6-(2-(3-pyridyl)-1-cyclopropyl-methyl)-4,5,7,8-tetrahydro-6H-thiazolo[5,4-d]azepin
2-Amino-6-(2-(4-pyridyl)-1-cyclopropyl-methyl)-4,5,7,8-tetrahydro-6H-thiazolo[5,4-d]azepin
2-Amino-6-(2H-1-benzopyran-3-yl)methyl-4,5,7,8-tetrahydro-6H-thiazolo[5,4-d]azepin
2-Amino-6-(2-methyl-2H-1-benzopyran-3-yl)methyl-4,5,7,8-tetrahydro-6H-thiazolo[5,4-d]azepin
2-Amino-6-(2,2-dimethyl-2H-1-benzopyran-3-yl)methyl-4,5,7,8-tetrahydro-6H-thiazolo[5,4-d]azepin
2-Amino-6-(2H-1-benzothiopyran-3-yl)methyl-4,5,7,8-tetrahydro-6H-thiazolo[5,4-d]azepin
2-Amino-6-(2-methyl-2H-1-benzothiopyran-3-yl)methyl-4,5,7,8-tetrahydro-6H-thiazolo[5,4-d]azepin
2-Amino-6-(2,2-dimethyl-2H-1-benzothiopyran-3-yl)methyl-4,5,7,8-tetrahydro-6H-thiazolo[5,4-d]azepin
2-Amino-6-(3-(3-hydroxy-2-pyridyl)allyl-4,5,7,8-tetrahydro-6H-thiazolo[5,4-d]azepin
2-Amino-6-(3-(6-hydroxy-2-pyridyl)allyl-4,5,7,8-tetrahydro-6H-thiazolo[5,4-d]azepin
2-Amino-6-(3-(2-benzothiophenyl)allyl)-4,5,7,8-tetrahydro-6H-thiazolo[5,4-d]azepin
2-Amino-6-(3-(4-benzothiophenyl)allyl)-4,5,7,8-tetrahydro-6H-thiazolo[5,4-d]azepin

2-Amino-6-(3-(5-benzothiophenyl)allyl)-4,5,7,8-tetrahydro-6H-thiazolo[5,4-d]azepin
2-Amino-6-(3-(6-benzothiophenyl)allyl)-4,5,7,8-tetrahydro-6H-thiazolo[5,4-d]azepin
2-Amino-6-(3-(7-benzothiophenyl)allyl)-4,5,7,8-tetrahydro-6H-thiazolo[5,4-d]azepin

Beispiel A

Tabletten mit 5 mg 2-Amino-6-(3-(4-cyano-phenyl)allyl-4,5,7,8-tetrahydro-6H-thiazolo[5,4-d]azepin

Zusammensetzung:

1 Tablette enthält:

| | | |
|---|---|---|
| Wirksubstanz | (1) | 5,0 mg |
| Maisstärke | (2) | 62,0 mg |
| Milchzucker | (3) | 48,0 mg |
| Polyvinylpyrrolidon | (4) | 4,0 mg |
| Magnesiumstearat | (5) | 1,0 mg |
| | | 120,0 mg |

Herstellungsverfahren:

(1), (2), (3) und (4) werden gemischt und mit Wasser befeuchtet. Die feuchte Mischung wird durch ein Sieb mit 1,5 mm Maschenweite gedrückt und bei ca. 45 °C getrocknet. Das trockene Granulat wird durch ein Sieb mit 1,0 mm Maschenweite geschlagen und mit (5) vermischt. Die fertige Mischung preßt man auf einer Tablettenpresse mit Stempeln von 7 mm Durchmesser, die mit einer Teilkerbe versehen sind, zu Tabletten.

| | |
|---|---|
| Tablettengewicht: | 120 mg |

Beispiel B

Tabletten mit 2,5 mg 2-Amino-6-(3-(4-cyano-phenyl)allyl-4,5,7,8-tetrahydro-6H-thiazolo[5,4-d]azepin

Zusammensetzung:

1 Tablette enthält:

| | |
|---|---|
| Wirksubstanz | 2,5 mg |
| Maisstärke | 64,5 mg |
| Milchzucker | 48,0 mg |
| Polyvinylpyrrolidon | 4,0 mg |
| Magnesiumstearat | 1,0 mg |
| | 120,0 mg |

Herstellungsverfahren:

Die Mischung aus der Wirksubstanz, Milchzucker und Maisstärke wird mit einer 20%igen Lösung von Polyvinylpyrrolidon in Wasser befeuchtet. Die feuchte Masse wird durch ein Sieb mit 1,5 mm Maschenweite granuliert und bei 45 °C getrocknet. Das getrocknete Granulat wird durch ein Sieb mit 1 mm Maschenweite gerieben und mit Magnesiumstearat homogen vermischt.

| | | |
|---|---|---|
| Tablettengewicht:<br>Stempel: | 120 mg<br>7 mm | <br>Durchmesser mit Teilkerbe |

Beispiel C

Dragées mit 2,5 mg 2-Amino-6-(3-(4-cyano-phenyl)allyl-4,5,7,8-tetrahydro-6H-thiazolo[5,4-d]azepin

Zusammensetzung:

1 Dragéekern enthält:

| | | |
|---|---|---|
| Wirksubstanz | (1) | 2,5 mg |
| Kartoffelstärke | (2) | 44,0 mg |
| Milchzucker | (3) | 30,0 mg |
| Polyvinylpyrrolidon | (4) | 3,0 mg |
| Magnesiumstearat | (5) | 0,5 mg |
| | | 80,0 mg |

Herstellungsverfahren:

1, 2, 3 und 4 werden gut gemischt und mit Wasser befeuchtet. Die feuchte Masse drückt man durch ein Sieb mit 1,0 mm Maschenweite, trocknet bei ca. 45°C und schlägt das Granulat anschließend durch dasselbe Sieb. Nach dem Zumischen von 5 werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht.
Die fertigen Dragées werden mit Wachs poliert.

| | |
|---|---|
| Dragéegewicht: | 120 mg |

Beispiel D

Dragées mit 5 mg 2-Amino-6-(3-(4-cyano-phenyl)allyl-4,5,7,8-tetrahydro-6H-thiazolo[5,4-d]azepin

Zusammensetzung:

1 Dragéekern enthält:

| | |
|---|---|
| Wirksubstanz | 5,0 mg |
| Calciumphosphat sekundär | 70,0 mg |
| Maisstärke | 50,0 mg |
| Polyvinylpyrrolidon | 4,0 mg |
| Magnesiumstearat | 1,0 mg |
| | 130,0 mg |

Herstellungsverfahren:

Die Mischung aus der Wirksubstanz, Calciumphosphat und Maisstärke wird mit einer 15%igen Lösung von Polyvinylpyrrolidon in Wasser befeuchtet. Die feuchte Masse wird durch ein Sieb mit 1 mm Maschenweite geschlagen, bei 45°C getrocknet und anschließend durch dasselbe Sieb gerieben. Nach dem Vermischen mit der angegebenen Menge Magnesiumstearat werden daraus Dragéekerne gepreßt.

| | | |
|---|---|---|
| Kerngewicht:<br>Stempel: | 130 mg<br>7 mm | <br>Durchmesser |

Auf die so hergestellten Dragéekerne wird auf bekannte Art eine Schicht aus Zucker und Talkum aufgetragen. Die fertigen Dragées werden mit Wachs poliert.

| | |
|---|---|
| Dragéegewicht: | 180 mg |

## Patentansprüche

**1.** 4,5,7,8-Tetrahydro-6H-thiazolo[5,4-d]azepine der allgemeinen Formel II

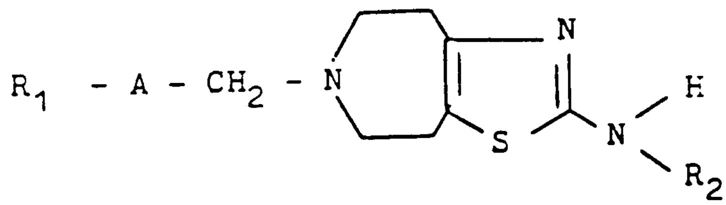

,(II)

in der
A eine Gruppe der Formeln

$-\underset{*}{C}(R_3)=CH-$, $-\underset{*}{C}H=C(R_4)-$, 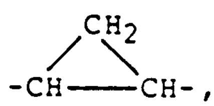, $-C{\equiv}C-$, $-\underset{*}{C}H=CH-CH_2-$,

$-\underset{*}{C}H(OR_5)-CH_2-$ oder $-(CH_2)_n-$

in welchen
n die Zahlen 2, 3 oder 4,
$R_3$ ein Wasserstoffatom oder eine Methylgruppe,
$R_4$ eine Alkylgruppe mit 1 bis 3 Kohlenstoffatomen oder eine Phenylgruppe und
$R_5$ ein Wasserstoffatom, eine Methyl- oder Äthylgruppe bedeuten und das mit * gekennzeichnete Kohlenstoffatom mit dem Rest $R_1$ verknüpft ist, und
$R_1$ einen gegebenenfalls durch ein Halogenatom, eine Alkoxygruppe mit 1 bis 4 Kohlenstoffatomen, eine Methyl-, Trifluormethyl-, Phenyl-, Nitro-, Amino-, Dimethylamino-, Piperidino-, Acetylamino-, Methylthio-, Methylsulfinyl-, Methylsulfonyl-, Cyano-, Aminocarbonyl-, Carboxy-, Methoxycarbonyl-, Äthoxycarbonyl-, Benzyloxy-, Pyridylmethoxy- oder Hydroxygruppe monosubstituierten Phenylrest, einen durch Methoxy-, Benzyloxy-, Hydroxy- oder Methylgruppen disubstituierten Phenylrest, wobei die Substituenten gleich oder verschieden sein können, oder einen durch drei Methoxygruppen, durch drei Hydroxygruppen oder durch eine Hydroxy- oder Aminogruppe und durch zwei Chlor- oder Bromatome trisubstituierten Phenylrest,
einen gegebenenfalls durch ein Chloratom, eine Methyl-, Methoxy-, Benzyloxy- oder Hydroxygruppe substituierten Pyridylrest,
einen Naphthyl-, Chinolyl-, Isochinolyl-, Indolyl-, Furyl-, Thienyl-, (2-Indolinon)yl-, Carbostyril- oder 3,4-Dihydrocarbostyrilrest,
einen gegebenenfalls in 2-Position durch eine Methyl- oder Aminogruppe substituierten Thiazolylrest,
einen Benzothiophenyl- oder Benzofuranylrest,
einen gegebenenfalls in 2-Position durch eine Methyl-, Phenyl- oder Aminogruppe substituierten Benzothiazolyl-, Benzoxazolyl- oder Benzimidazolylrest oder

A eine Kohlenstoff-Kohlenstoff-Bindung und
$R_1$ eine 1H-Inden-2-yl- oder 1,2-Dihydronaphthalin-3-yl-gruppe oder eine gegebenenfalls durch eine oder zwei Methylgruppen substituierte 2H-1-Benzopyran-3-yl- oder 2H-1-Benzothiopyran-3-yl-gruppe und
$R_2$ ein Wasserstoffatom oder eine gegebenenfalls in omega-Stellung durch einen Phenyl- oder 4-Methoxyphenylrest substituierte Acetyl- oder Propionylgruppe bedeuten, und deren Säureadditionssalze.

**2.** 4,5,7,8-Tetrahydro-6H-thiazolo[5,4-d]azepine der allgemeinen Formel II gemäß Anspruch 1, in der
$R_2$ wie im Anspruch 1 definiert ist,
A eine Gruppe der Formeln

$-\underset{*}{C}(R_3)=CH-$, $-\underset{*}{C}H=C(R_4)-$, 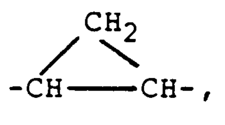 $-C\equiv C-$, $-\underset{*}{C}H=CH-CH_2-$,

$-\underset{*}{C}H(OR_5)-CH_2-$ oder $-(CH_2)_n-$

in welchen
n die Zahlen 2, 3 oder 4,
$R_3$ ein Wasserstoffatom oder eine Methylgruppe,
$R_4$ eine Alkylgruppe mit 1 bis 3 Kohlenstoffatomen oder eine Phenylgruppe und
$R_5$ ein Wasserstoffatom, eine Methyl- oder Äthylgruppe bedeuten und das mit * gekennzeichnete Kohlenstoffatom mit dem Rest $R_1$ verknüpft ist, und
$R_1$ eine gegebenenfalls durch ein Fluor-, Chlor- oder Bromatom, eine Alkoxygruppe mit 1 bis 4 Kohlenstoffatomen, eine Methyl-, Trifluormethyl-, Phenyl-, Hydroxy-, Benzyloxy-, Nitro-, Amino-, Dimethylamino-, Piperidino-, Cyano-, Aminocarbonyl-, Methoxycarbonyl-, Ethoxycarbonyl-, Methylmercapto-, Methylsulfinyl-, Methylsulfonyl- oder Pyridylmethoxygruppe substituierten Phenylrest, eine Dimethoxyphenyl-, Dihydroxy-phenyl-, 4-Hydroxy-3,5-dichlor-phenyl-, 4-Hydroxy-3,5-dibrom-phenyl-, 4-Amino-3,5-dichlor-phenyl-, 4-Amino-3,5-dibrom-phenyl-, 3,4,5-Trimethoxy-phenyl-, Naphthyl-, 6-Chlor-2-pyridyl-, Thienyl-, Furyl-, Chinolyl-, Isochinolyl-, Benzothio-phenyl-, Indolyl- oder Indolin-2-on-4-yl-gruppe oder eine gegebenenfalls durch eine Methylgruppe substituierte Pyridylgruppe oder
A eine Kohlenstoff-Kohlenstoff-Bindung und
$R_1$ eine 1H-Inden-2-yl-, 1,2-Dihydronaphthalin-3-yl- oder 2H-1-Benzopyran-3-yl-gruppe bedeuten, und deren Säureadditionssalze.

**3.** 4,5,7,8-Tetrahydro-6H-thiazolo[5,4-d]azepine der allgemeinen Formel II gemäß Anspruch 2, in der
$R_1$ und A wie im Anspruch 2 definiert sind und
$R_2$ ein Wasserstoffatom darstellt, und deren Säureadditionssalze.

**4.** 4,5,7,8-Tetrahydro-6H-thiazolo[5,4-d]azepine der allgemeinen Formel II gemäß Anspruch 2, in der
A eine Vinylen-, Äthinylen-, Cyclopropylen- oder Äthylengruppe,
$R_1$ eine gegebenenfalls durch ein Chloratom, eine Hydroxy-, Methoxy-, Benzyloxy-, Isobutoxy-, Phenyl-, Nitro-, Amino-, Cyano- oder Piperidinogruppe substituierte Phenylgruppe, eine gegebenenfalls durch eine Methylgruppe substituierte Pyridylgruppe, eine Dimethoxyphenyl-, Naphthyl-, Isochinolyl-, 2-Methyl-thiazolyl-, Furyl- oder Thienylgruppe und
$R_2$ ein Wasserstoffatom bedeuten, und deren Säureadditionssalze.

**5.** Eine Verbindung gemäß Anspruch 1, das 2-Amino-6-(3-(4-cyano-phenyl)allyl-4,5,7,8-tetrahydro-6H-thiazolo[5,4-d]azepin und dessen Säureadditionssalze.

**6.** Eine Verbindung gemäß Anspruch 1, das 2-Amino-6-(3-(3-benzyloxy-phenyl)allyl-4,5,7,8-tetrahydro-6H-thiazolo[5,4-d]azepin und dessen Säureadditionssalze.

**7.** Eine Verbindung gemäß Anspruch 1, das 2-Amino-6-(3-(1-naphthyl)allyl-4,5,7,8-tetrahydro-6H-thiazolo-[5,4-d]azepin und dessen Säureadditionssalze.

**8.** Physiologisch verträgliche Säureadditionssalze mit anorganischen oder organischen Säuren der Verbindungen nach mindestens einem der Ansprüche 1 bis 7.

**9.** Arzneimittel, enthaltend als Wirkstoff eine Verbindung nach mindestens einem der Ansprüche 1 bis 7 oder ein physiologisch verträgliches Säureadditionssalz gemäß Anspruch 8 neben gegebenenfalls einem oder mehreren inerten Trägerstoffen und/oder Verdünnungsmitteln.

**10.** Arzneimittel gemäß Anspruch 9 zur Behandlung von Krankheiten des Zentralnervensystems wie Parkinsonismus, Hyperprolactinämie und Schizophrenie.

**11.** Arzneimittel gemäß Anspruch 9 zur Behandlung von cardiovasculären Erkrankungen.

**12.** Verwendung einer Verbindung gemäß den Ansprüchen 1 bis 8 zur Herstellung eines Arzneimittels gemäß den Ansprüchen 9 bis 11.

**13.** Verfahren zur Herstellung eines Arzneimittels gemäß den Ansprüchen 9, 10 oder 11, dadurch gekennzeichnet, daß eine Verbindung nach mindestens einem der Ansprüche 1 bis 7 oder ein physiologisch verträgliches Säureadditionssalz gemäß Anspruch 8 in einen oder mehrere inerte Trägerstoffe und/oder Verdünnungsmittel eingearbeitet wird.

**14.** Verfahren zur Herstellung der 4,5,7,8-Tetrahydro-6H-thiazolo[5,4-d]azepine der allgemeinen Formel II nach mindestens einem der Ansprüche 1 bis 8, dadurch gekennzeichnet, daß

a) eine Verbindung der allgemeinen Formel III

$$R_1 - A - CH_2 - X \quad ,(III)$$

in der
A und $R_1$ wie mindestens in einem der Ansprüche 1 bis 7 definiert sind und
X eine nukleophile Austrittsgruppe bedeutet,
mit einer Verbindung der allgemeinen Formel IV

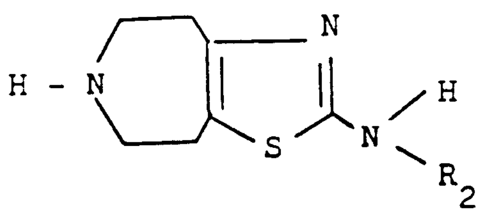

, (IV)

in der
$R_2$ wie mindestens in einem der Ansprüche 1 bis 7 definiert ist, umgesetzt wird oder

b) zur Herstellung von Verbindungen der allgemeinen Formel II, in der $R_1$ mit Ausnahme eines gegebenenfalls durch ein Chloratom, eine Methyl-, Methoxy-, Benzyloxy- oder Hydroxygruppe substituierten 2-Pyridylrestes, eines 2-Chinolyl-, 1-Isochinolyl-, 3-Isochinolyl-, 2-Thiazolyl-, 2-Benzoxazolyl-oder 2-Benzimidazolylrestes die für $R_1$ in mindestens einem der Ansprüche 1 bis 7 erwähnten Bedeutungen besitzt, ein Aldehyd der allgemeinen Formel V

$$R_1' - A - \overset{H}{C} = O \quad , (V)$$

in der
A wie mindestens in einem der Ansprüche 1 bis 7 definiert ist und

$R_1'$ mit Ausnahme eines gegebenenfalls durch ein Chloratom, eine Methyl-, Methoxy-, Benzyloxy- oder Hydroxygruppe substituierten 2-Pyridylrestes, eines 2-Chinolyl-, 1-Isochinolyl-, 3-Isochinolyl-, 2-Thiazolyl-, 2-Benzoxazolyl- oder 2-Benzimidazolylrestes die für $R_1$ in mindestens einem der Ansprüche 1 bis 7 erwähnten Bedeutungen besitzt,
mit einer Verbindung der allgemeinen Formel IV

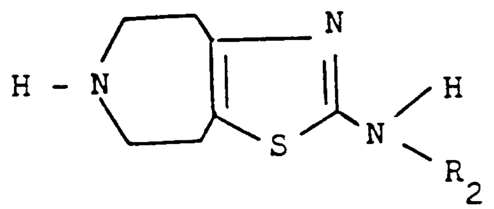

, (IV)

in der
$R_2$ wie mindestens in einem der Ansprüche 1 bis 7 definiert ist, reduktiv aminiert wird oder
c) ein 5-Halogen-azepin-4-on der allgemeinen Formel VI

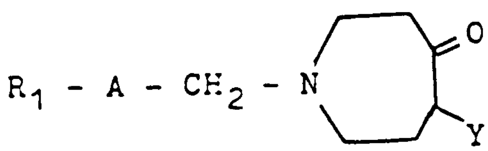

x HY , (VI)

in der
A und $R_1$ wie mindestens in einem der Ansprüche 1 bis 7 definiert sind und
Y ein Brom- oder Chloratom bedeutet,
mit einem Thioharnstoff der allgemeinen Formel VII

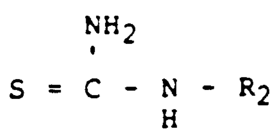

, (VII)

in der
$R_2$ wie mindestens in einem der Ansprüche 1 bis 7 definiert ist, umgesetzt wird oder
d) zur Herstellung von Verbindungen der allgemeinen Formel II, in der $R_2$ ein Wasserstoffatom bedeutet, ein Azepin-4-on der allgemeinen Formel VIII

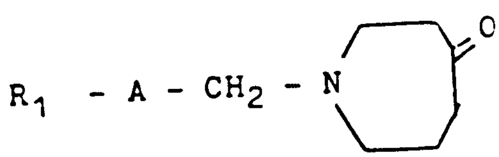

, (VIII)

in der
A und $R_1$ wie mindestens in einem der Ansprüche 1 bis 7 definiert sind,
mit einem Formamidin-disulfid-Salz der allgemeinen Formel IX

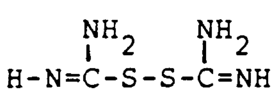

x 2 HZ , (IX)

in der
Z einen Rest einer anorganischen oder organischen Säure darstellt, umgesetzt wird oder

e) zur Herstellung von Verbindungen der allgemeinen Formel II, in der $R_1$ einen Phenyl-, Naphthyl-, Pyridyl-, Chinolinyl-, Isochinolinyl-, Furyl-, Thienyl-, Benzofuryl-, Benzothienyl-, (2-Indolinon)yl-, Carbostyril- oder 3,4-Dihydro-carbostyrilrest und A eine Äthinylengruppe bedeuten, eine Verbindung der allgemeinen Formel X

$R_1''$ - Hal ,(X)

in der
$R_1''$ einen Phenyl-, Naphthyl-, Pyridyl-, Chinolinyl-, Isochinolinyl-, Furyl-, Thienyl-, Benzofuryl-, Benzothienyl-, (2-Indolinon)yl-, Carbostyril- oder 3,4-Dihydro-carbostyrilrest und
Hal ein Brom- oder Jodatom bedeuten,
mit einer Propargyl-Verbindung der allgemeinen Formel XI

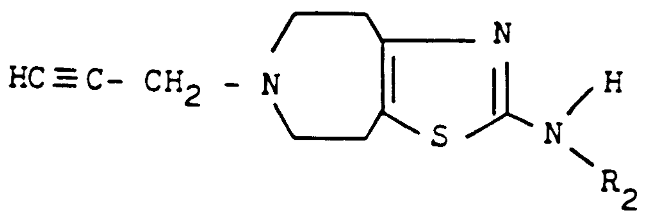

,(XI)

in der
$R_2$ wie mindestens in einem der Ansprüche 1 bis 7 definiert ist, umgesetzt wird oder
f) zur Herstellung von Verbindungen der allgemeinen Formel II, in der A eine Vinylengruppe bedeutet, eine Propargyl-Verbindung der allgemeinen Formel XII

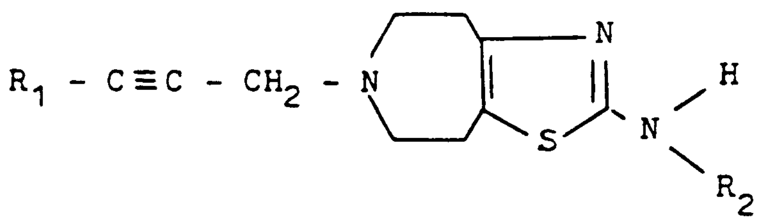

,(XII)

in der
$R_1$ und $R_2$ wie mindestens in einem der Ansprüche 1 bis 7 definiert sind, reduziert wird oder
g) zur Herstellung von Verbindungen der allgemeinen Formel II, in der A eine Äthylengruppe bedeutet, eine Verbindung der allgemeinen Formel XIII

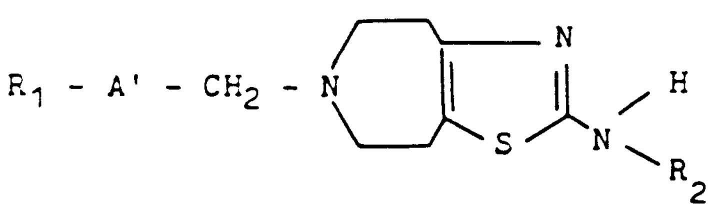

,(XIII)

in der
$R_1$ und $R_2$ wie mindestens in einem der Ansprüche 1 bis 7 definiert sind und
A' eine Vinylen- oder Äthinylengruppe bedeutet, reduziert wird oder
h) zur Herstellung von Verbindungen der allgemeinen Formel II, in der A eine Cyclopropylen- oder eine n-Alkylengruppe mit zwei bis vier Kohlenstoffatomen und $R_2$ ein Wasserstoffatom darstellen, ein Amid der allgemeinen Formel XIV

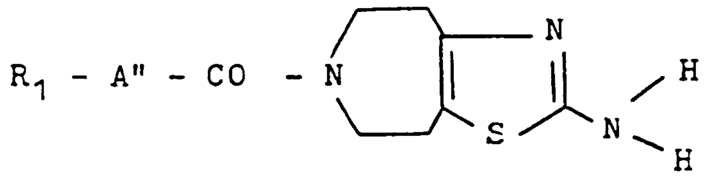

, (XIV)

in der
$R_1$ wie mindestens in einem der Ansprüche 1 bis 7 definiert ist und
A″ eine Cyclopropylen- oder eine n-Alkylengruppe mit zwei bis vier Kohlenstoffatomen bedeuten, reduziert wird oder
i) zur Herstellung von Verbindungen der allgemeinen Formel II, in der A eine Gruppe der Formel $-CH(OR_5)-CH_2-$ bedeutet, ein Keton der allgemeinen Formel XV

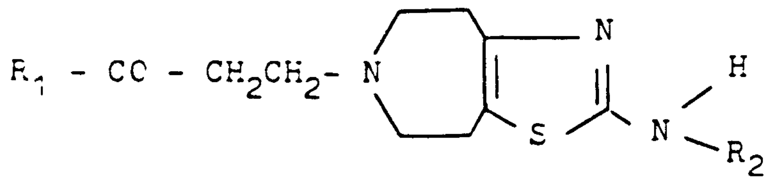

, (XV)

in der
$R_1$ und $R_2$ wie mindestens in einem der Ansprüche 1 bis 7 definiert sind, reduziert wird oder
k) zur Herstellung von Verbindungen der allgemeinen Formel II, in der A eine Vinylengruppe bedeutet, ein Alkohol der allgemeinen Formel XVI

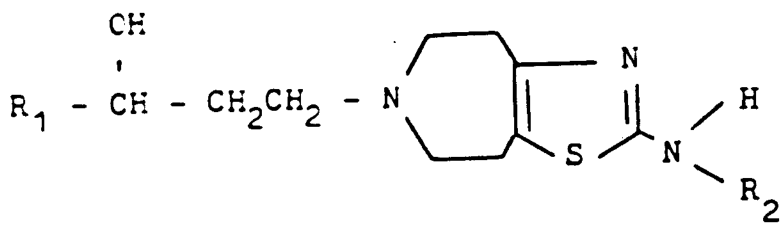

, (XVI)

in der
$R_1$ und $R_2$ wie mindestens in einem der Ansprüche 1 bis 7 definiert sind, dehydratisiert wird oder
l) zur Herstellung von Verbindungen der allgemeinen Formel II, in der $R_2$ eine gegebenenfalls in omega-Stellung durch einen Phenyl- oder 4-Methoxyphenylrest substituierte Acetyl-oder Propionylgruppe bedeutet, ein Amin der allgemeinen Formel XVII

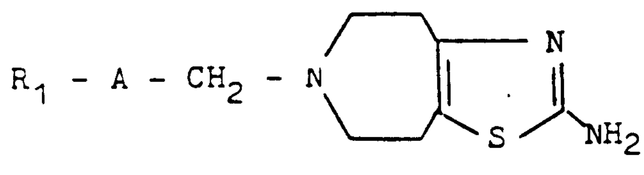

, (XVII)

in der
A und $R_1$ wie mindestens in einem der Ansprüche 1 bis 7 definiert sind,
mit einer Carbonsäure der allgemeinen Formel XVIII

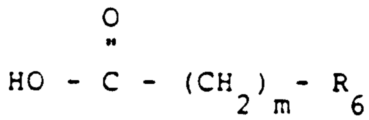
, (XVIII)

in der
m die Zahl 1 oder 2 und
$R_6$ ein Wasserstoffatom, einen Phenyl- oder 4-Methoxyphenylrest bedeuten,
oder mit deren gegebenenfalls im Reaktionsgemisch hergestellten reaktionsfähigen Derivaten acyliert wird oder
m) zur Herstellung von Verbindungen der allgemeinen Formel II, in der $R_2$ ein Wasserstoffatom bedeutet, eine Verbindung der allgemeinen Formel XIX

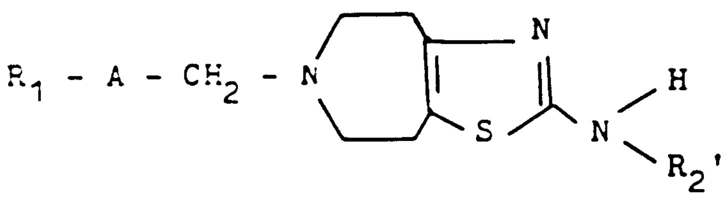
, (XIX)

in der
A und $R_1$ wie mindestens in einem der Ansprüche 1 bis 7 definiert sind und
$R_2'$ einen hydrolytisch abspaltbaren Rest wie einen Acyl- oder Kohlensäureesterrest darstellt, desacyliert wird oder
n) zur Herstellung von Verbindungen der allgemeinen Formel II, in der A mit Ausnahme der -CH-($OR_5$)-$CH_2$-Gruppe die für A in mindestens einem der Ansprüche 1 bis 7 erwähnten Bedeutungen besitzt und $R_1$ einen durch eine Hydroxygruppe substituierten Phenyl-, Methylphenyl-, Methoxyphenyl- oder Pyridylrest oder einen durch zwei oder drei Hydroxygruppen substituierten Phenylrest und $R_2$ ein Wasserstoffatom bedeuten, eine Verbindung der allgemeinen Formel XX

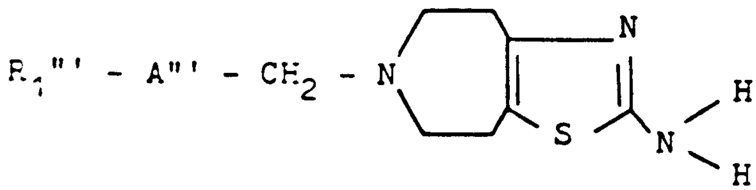
, (XX)

in der
$A'''$ mit Ausnahme der -CH($OR_5$)-$CH_2$-Gruppe die für A eingangs erwähnten Bedeutungen besitzt und
$R_1'''$ einen durch eine Benzyloxy- oder Methoxygruppe substituierten Phenyl-, Methylphenyl-, Methoxyphenyl- oder Pyridylrest oder einen durch zwei oder drei Benzyloxy- oder Methoxygruppen substituierten Phenylrest bedeutet, gespalten wird und
gewünschtenfalls anschließend eine so erhaltene Verbindung der allgemeinen Formel II, in der $R_1$ einen Nitrophenylrest bedeutet, mittels Reduktion in eine entsprechende Verbindung, in der $R_1$ einen Aminophenylrest bedeutet, übergeführt wird oder
eine so erhaltene Verbindung der allgemeinen Formel II, in der $R_1$ einen Cyanophenylrest bedeutet, mittels Hydratisierung in eine entsprechende Verbindung, in der $R_1$ einen Aminocarbonylphenylrest bedeutet, übergeführt wird oder
eine so erhaltene Verbindung der allgemeinen Formel II, in der $R_1$ einen Cyanophenylrest bedeutet, mittels Alkoholyse in eine Verbindung, in der $R_1$ einen Methoxycarbonylphenyl-oder Äthoxycarbonylphenylrest bedeutet, übergeführt wird oder
eine so erhaltene Verbindung der allgemeinen Formel II, in der $R_1$ einen Cyanophenylrest bedeutet, mittels Hydrolyse in eine Verbindung, in der $R_1$ einen Carboxyphenylrest bedeutet, übergeführt wird oder

eine so erhaltene Verbindung der allgemeinen Formel II, in der $R_1$ einen Hydroxyphenylrest bedeutet, mittels Benzylalkohol oder eines Pyridylmethanols in Gegenwart von einem Azodicarbonsäurediester in eine Verbindung, in der $R_1$ einen Benzyloxyphenyl- oder Pyridylmethoxyphenylrest bedeutet, übergeführt wird oder

eine so erhaltene Verbindung der allgemeinen Formel II in ihr Säureadditionssalz, insbesondere für die pharmazeutische Anwendung in ihr physiologisch verträgliches Säureadditionssalz mit einer anorganischen oder organischen Säure übergeführt wird.

## Claims

**1.** 4,5,7,8-tetrahydro-6H-thiazolo[5,4-d]azepines of general formula II

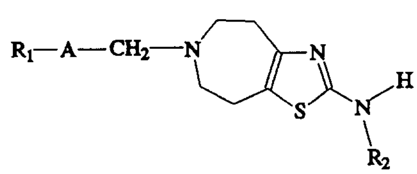

(II)

(wherein

A represents a group of the formulae

$\underset{*}{-C}(R_3)=CH-$, $\underset{*}{-CH}=C(R_4)-$, 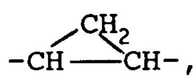, $-C\equiv C-$, $\underset{*}{-CH}=CH-CH_2-$,

$\underset{*}{-CH}(OR_5)-CH_2-$ or $-(CH_2)_n-$

(wherein

n represents the numbers 2, 3 or 4,

$R_3$ represents a hydrogen atom or a methyl group,

$R_4$ represents a $C_{1-3}$-alkyl group or a phenyl group and

$R_5$ represents a hydrogen atom, a methyl or ethyl group and the carbon atom designated * is linked to the group $R_1$) and

$R_1$ represents a phenyl group optionally monosubstituted by a halogen atom or by an alkoxy group with 1 to 4 carbon atoms, a methyl, trifluoromethyl, phenyl, nitro, amino, dimethylamino, piperidino, acetylamino, methylthio, methylsulphinyl, methylsulphonyl, cyano, aminocarbonyl, carboxy, methoxycarbonyl, ethoxycarbonyl, benzyloxy, pyridylmethoxy or hydroxy group, a phenyl group disubstituted by methoxy, benzyloxy, hydroxy or methyl groups, whilst the substituents may be identical or different, or a phenyl group trisubstituted by three methoxy groups, by three hydroxy groups or by one hydroxy or amino group and by two chlorine or bromine atoms,

a pyridyl group optionally substituted by a chlorine atom or by a methyl, methoxy, benzyloxy or hydroxy group,

a naphthyl, quinolyl, isoquinolyl, indolyl, furyl, thienyl, (2-indolinon)yl, carbostyril or 3,4-dihydrocarbostyril group,

a thiazolyl group optionally substituted in the 2-position by a methyl or amino group,

a benzothiophenyl or benzofuranyl group, or

a benzothiazolyl, benzoxazolyl or benzimidazolyl group optionally substituted in the 2-position by a methyl, phenyl or amino group; or

A represents a carbon-carbon bond, and

$R_1$ represents a 1H-inden-2-yl or 1,2-dihydronaphthalen-3-yl group or a 2H-1-benzopyran-3-yl or 2H-1-benzothiopyran-3-yl group optionally substituted by one or two methyl groups and

$R_2$ represents a hydrogen atom or an acetyl or propionyl group optionally substituted in the omega-position by a phenyl or 4-methoxyphenyl group) and the acid addition salts thereof.

**2.** 4,5,7,8-tetrahydro-6H-thiazolo[5,4-d]azepines of general formula II as claimed in claim 1, wherein:
$R_2$ is defined as in claim 1,
A represents a group of formulae

$-\overset{*}{C}(R_3)=CH-$, $-\overset{*}{C}H=C(R_4)-$, 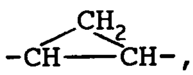, $-C\equiv C-$, $-\overset{*}{C}H=CH-CH_2-$,

$-\overset{*}{C}H(OR_5)-CH_2-$ or $-(CH_2)_n-$

(wherein
n represents the numbers 2, 3 or 4,
$R_3$ represents a hydrogen atom or a methyl group,
$R_4$ represents a $C_{1-3}$-alkyl group or a phenyl group and
$R_5$ represents a hydrogen atom, a methyl or ethyl group and the carbon atom designated * is linked to the group $R_1$), and
$R_1$ represents a phenyl group optionally substituted by a fluorine, chlorine or bromine atom, or by an alkoxy group with 1 to 4 carbon atoms, a methyl, trifluoromethyl, phenyl, hydroxy, benzyloxy, nitro, amino, dimethylamino, piperidino, cyano, aminocarbonyl, methoxycarbonyl, ethoxycarbonyl, methylmercapto, methylsulphinyl, methylsulphonyl or pyridylmethoxy group,
a dimethoxyphenyl, dihydroxyphenyl, 4-hydroxy-3,5-dichlorophenyl, 4-hydroxy-3,5-dibromophenyl, 4-amino-3,5-dichlorophenyl, 4-amino-3,5-dibromo-phenyl, 3,4,5-trimethoxy-phenyl, naphthyl, 6-chloro-2-pyridyl, thienyl, furyl, quinolyl, isoquinolyl, benzothiophenyl, indolyl or indolin-2-on-4-yl group or a pyridyl group optionally substituted by a methyl group; or
A represents a carbon-carbon bond and
$R_1$ represents a 1H-inden-2-yl, 1,2-dihydronaphthalen-3-yl or 2H-1-benzopyran-3-yl group;
and the acid addition salts thereof.

**3.** 4,5,7,8-tetrahydro-6H-thiazolo[5,4-d]azepines of general formula II according to claim 2, wherein:
$R_1$ and A are defined as in claim 2 and
$R_2$ represents a hydrogen atom;
and the acid addition salts thereof.

**4.** 4,5,7,8-tetrahydro-6H-thiazolo[5,4-d]azepines of general formula II according to claim 2, wherein
A represents a vinylene, ethynylene, cyclopropylene or ethylene group,
$R_1$ represents a phenyl group optionally substituted by a chlorine atom or by a hydroxy, methoxy, benzyloxy, isobutoxy, phenyl, nitro, amino, cyano or piperidino group,
a pyridyl group optionally substituted by a methyl group, or a dimethoxyphenyl, naphthyl, isoquinolyl, 2-methyl-thiazolyl, furyl or thienyl group and
$R_2$ represents a hydrogen atom;
and the acid addition salts thereof.

**5.** A compound according to claim 1, 2-amino-6-(3-(4-cyano-phenyl)allyl)-4,5,7,8-tetrahydro-6H-thiazolo-[5,4-d]azepine and the acid addition salts thereof.

**6.** A compound according to claim 1, 2-amino-6-(3-(3-benzyloxy-phenyl)allyl)-4,5,7,8-tetrahydro-6H-thiazolo[5,4-d]azepine and the acid addition salts thereof.

**7.** A compound according to claim 1, 2-amino-6-(3-(1-naphthyl)allyl)-4,5,7,8-tetrahydro-6H-thiazolo[5,4-d]-azepine and the acid addition salts thereof.

**8.** Physiologically acceptable acid addition salts with organic or inorganic acids of the compounds according to at least one of claims 1 to 7.

**9.** Pharmaceutical compositions containing as active substance a compound according to at least one of claims 1 to 7 or a physiologically acceptable acid addition salt as claimed in claim 8, optionally together with one or more inert carriers and/or diluents.

**10.** Pharmaceutical composition as claimed in claim 9 for the treatment of diseases of the central nervous system such as Parkinson's disease, hyperprolactinaemia and schizoprenia.

**11.** Pharmaceutical composition as claimed in claim 9 for treating cardiovascular diseases.

**12.** Use of a compound according to claims 1 to 8 for preparing a pharmaceutical composition according to claims 9 to 11.

**13.** Process for preparing a pharmaceutical composition according to claim 9, 10 or 11, characterised in that a compound according to at least one of claims 1 to 7 or a physiologically acceptable acid addition salt according to claim 8 is incorporated in one or more inert carriers and/or diluents.

**14.** Process for preparing the 4,5,7,8-tetrahydro-6H-thiazolo[5,4-d]azepines of general formula II according to at least one of claims 1 to 8, characterised in that

a) a compound of general formula III

$R_1$ - A - $CH_2$ - X (III)

wherein
A and $R_1$ are defined as in one of claims 1 to 7 and
X represents a nucleophilic leaving group,
is reacted with a compound of general formula IV

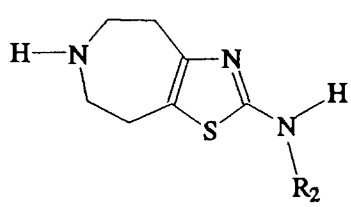

(IV)

wherein
$R_2$ is defined as in at least one of claims 1 to 7, or

b) in order to prepare compounds of general formula II wherein $R_1$ has the meanings given for $R_1$ in at least one of claims 1 to 7, with the exception of a 2-pyridyl group optionally substituted by a chlorine atom or by a methyl, methoxy, benzyloxy or hydroxy group, or a 2-quinolyl, 1-isoquinolyl, 3-isoquinolyl, 2-thiazolyl, 2-benzoxazolyl or 2-benzimidazolyl group:
an aldehyde of general formula V

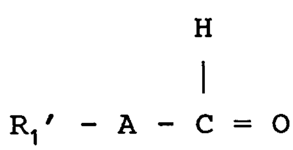

(V)

wherein
A is defined as in at least one of claims 1 to 7 and
$R_1$' has the meanings given for $R_1$ in at least one of claims 1 to 7 with the exception of a 2-pyridyl

group optionally substituted by a chlorine atom or by a methyl, methoxy, benzyloxy or hydroxy group, or a 2-quinolyl, 1-isoquinolyl, 3-isoquinolyl, 2-thiazolyl, 2-benzoxazolyl or 2-benzimidazolyl group,
is reductively aminated with a compound of general formula IV

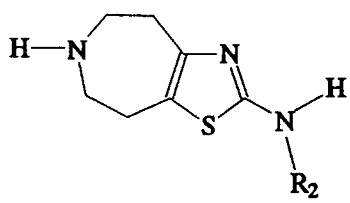

(IV)

wherein
$R_2$ is defined as in at least one of claims 1 to 7, or
c) a 5-halo-azepin-4-one of general formula VI

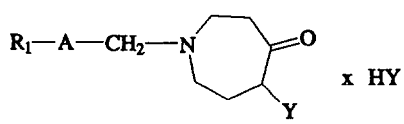

(VI)

wherein
A and $R_1$ are defined as in at least one of claims 1 to 7 and
Y represents a bromine or chlorine atom,
is reacted with a thiourea of general formula VII

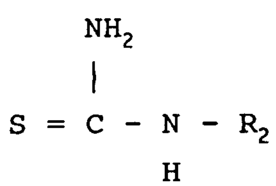

(VII)

wherein
$R_2$ is defined as in at least one of claims 1 to 7, or
d) in order to prepare compounds of general formula II wherein $R_2$ represents a hydrogen atom:
an azepin-4-one of general formula VIII

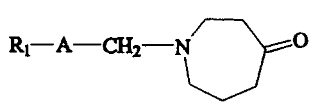

(VIII)

wherein
A and $R_1$ are defined as in at least one of claims 1 to 7, is reacted with a formamidine disulphide salt of general formula IX

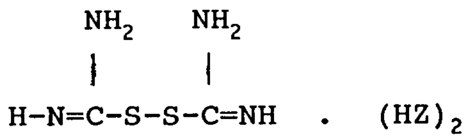

(IX)

wherein

Z represents a group of an inorganic or organic acid, or

e) in order to prepare compounds of general formula II wherein $R_1$ represents a phenyl, naphthyl, pyridyl, quinolinyl, isoquinolinyl, furyl, thienyl, benzofuryl, benzothienyl, (2-indolinon)yl, carbostyril or 3,4-dihydrocarbostyril group and A represents an ethynylene group:

a compound of general formula X

$R_1''$ - Hal (X)

wherein

$R_1''$ represents a phenyl, naphthyl, pyridyl, quinolinyl, isoquinolinyl, furyl, thienyl, benzofuryl, benzothienyl, (2-indolinon)yl, carbostyril or 3,4-dihydro-carbostyril group and

Hal represents a bromine or iodine atom,

is reacted with a propargyl compound of general formula XI

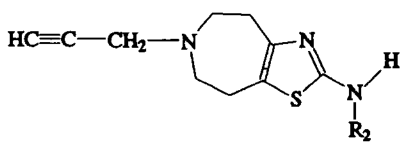

(XI)

wherein

$R_2$ is defined as in at least one of claims 1 to 7, or

f) in order to prepare compounds of general formula II wherein A represents a vinylene group:

a propargyl compound of general formula XII

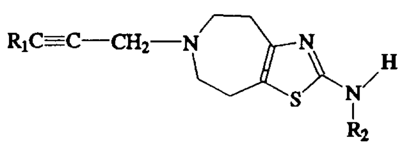

(XII)

wherein

$R_1$ and $R_2$ are defined as in at least one of claims 1 to 7, is reduced, or

g) in order to prepare compounds of general formula II wherein A represents an ethylene group:

a compound of general formula XIII

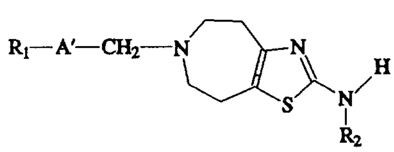

(XIII)

wherein

$R_1$ and $R_2$ are defined as in at least one of claims 1 to 7 and

A' represents a vinylene or ethynylene group, is reduced, or
h) in order to prepare compounds of general formula II wherein A represents a cyclopropylene or an n-alkylene group with 2 to 4 carbon atoms and $R_2$ represents a hydrogen atom:
an amide of general formula XIV

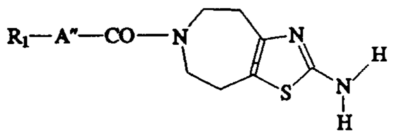

(XIV)

wherein
$R_1$ is defined as in at least one of claims 1 to 7 and
A'' represents a cyclopropylene or n-alkylene group with two to four carbon atoms, is reduced, or
i) in order to prepare compounds of general formula II wherein A represents a group of formula -CH-$(OR_5)$-$CH_2$-:
a ketone of general formula XV

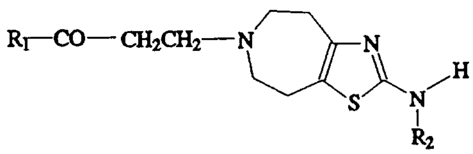

(XV)

wherein
$R_1$ and $R_2$ are defined as in at least one of claims 1 to 7, is reduced, or
k) in order to prepare compounds of general formula II wherein A represents a vinylene group:
an alcohol of general formula XVI

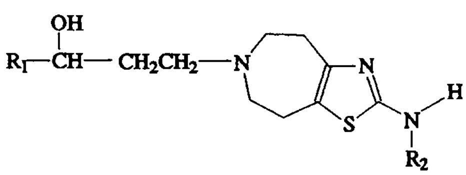

(XVI)

wherein
$R_1$ and $R_2$ are defined as in at least one of claims 1 to 7, is dehydrated, or
l) in order to prepare compounds of general formula II wherein $R_2$ represents an acetyl or propionyl group optionally substituted in the omega-position by a phenyl or 4-methoxyphenyl group:
an amine of general formula XVII

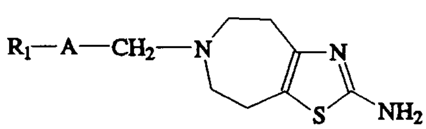

(XVII)

wherein A and $R_1$ are defined as in at least one of claims 1 to 7,
is acylated with a carboxylic acid of general formula XVIII

$$HO - \overset{\overset{O}{\|}}{C} - (CH_2)_m - R_6 \qquad (XVIII)$$

wherein
m represents the number 1 or 2 and
$R_6$ represents a hydrogen atom or a phenyl or 4-methoxyphenyl group,
or with the reactive derivatives thereof optionally prepared in the reaction mixture, or
m) in order to prepare compounds of general formula II wherein $R_2$ represents a hydrogen atom:
a compound of general formula XIX

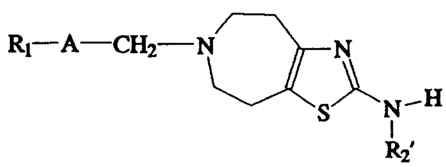

(XIX)

wherein
A and $R_1$ are defined as in at least one of claims 1 to 7 and
$R_2$' represents a hydrolytically cleavable group such as an acyl or carbonic acid ester group, is deacylated, or
n) in order to prepare compounds of general formula II wherein A has the meanings given for A in at least one of claims 1 to 7, with the exception of the -CH($OR_5$)-$CH_2$-group and $R_1$ represents a hydroxy-substituted phenyl, methylphenyl, methoxyphenyl or pyridyl group or a phenyl group substituted by two or three hydroxy groups and $R_2$ represents a hydrogen atom:
a compound of general formula XX

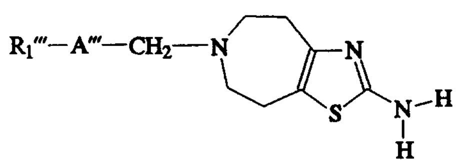

(XX)

wherein
A''' has the meanings given for A hereinbefore with the exception of the -CH($OR_5$)-$CH_2$- group and
$R_1$''' represents a phenyl, methylphenyl, methoxyphenyl or pyridyl group substituted by a benzyloxy or methoxy group, or a phenyl group substituted by two or three benzyloxy or methoxy groups, is split, and
subsequently, if desired, a compound of general formula II thus obtained wherein $R_1$ represents a nitrophenyl group, is converted by reduction into a corresponding compound wherein $R_1$ represents an aminophenyl group, or
a compound of general formula II obtained wherein $R_1$ represents a cyanophenyl group, is converted by hydration into a corresponding compound wherein $R_1$ represents an aminocarbonylphenyl group, or
a compound of general formula II obtained wherein $R_1$ represents a cyanophenyl group is converted by alcoholysis into a compound wherein $R_1$ represents a methoxycarbonylphenyl or ethoxycarbonylphenyl group, or
a compound of general formula II wherein $R_1$ represents a cyanophenyl group may be converted by hydrolysis into a compound wherein $R_1$ represents a carboxyphenyl group, or
a compound of general formula II wherein $R_1$ represents a hydroxyphenyl group may be converted

by benzyl alcohol or a pyridylmethanol in the presence of an azodicarboxylic acid diester into a compound wherein $R_1$ represents a benzyloxyphenyl or pyridylmethoxyphenyl group, or
a compound of general formula II thus obtained is converted into an acid addition salt thereof, more particularly, for pharmaceutical use, a physiologically acceptable acid addition salt thereof with an organic or inorganic acid.

## Revendications

1. 4,5,7,8-tétrahydro-6H-thiazolo[5,4-d]azépines de formule générale II

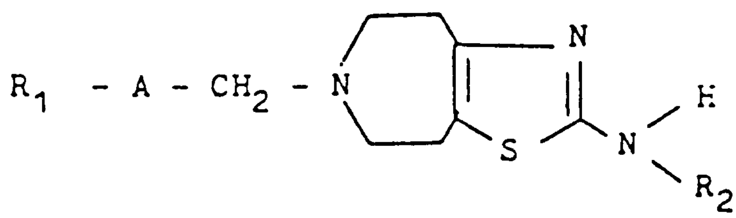

(II)

dans laquelle
A représente un groupe selon les formules

$-\underset{*}{C}(R_3)=CH-$, $-\underset{*}{C}H=C(R_4)-$, 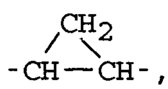 , $-C{\equiv}C-$, $-\underset{*}{C}H=CH-CH_2-$,

$-\underset{*}{C}H(OR_5)-CH_2-$ ou $-(CH_2)_n-$

dans lesquelles
n représente les nombres 2, 3 ou 4,
$R_3$ représente un atome d'hydrogène ou un groupe méthyle,
$R_4$ représente un groupe alkyle de 1 à 3 atomes de carbone ou un groupe phényle et
$R_5$ représente un atome d'hydrogène ou un groupe méthyle ou éthyle et l'atome de carbone caractérisé par * est lié au reste $R_1$, et
$R_1$ représente un reste phényle éventuellement monosubstitué par un atome d'halogène, un groupe alcoxy de 1 à 4 atomes de carbone, un groupe méthyle, trifluorométhyle, phényle, nitro, amino, diméthylamino, pipéridino, acétylamino, méthylthio, méthylsulfinyle, méthylsulfonyle, cyano, aminocarbonyle, carboxy, méthoxycarbonyle, éthoxycarbonyle, benzyloxy, pyridylméthoxy ou hydroxy, un reste phényle disubstitué par un groupe méthoxy, benzyloxy, hydroxy ou méthyle, les substituants pouvant être identiques ou différents, ou un reste phényle trisubstitué par trois groupes méthoxy, par trois groupes hydroxy ou par un groupe hydroxy ou amino et par deux atomes de chlore ou de brome, un reste pyridyle éventuellement substitué par un atome de chlore, un groupe méthyle, méthoxy, benzyloxy ou hydroxy, un reste naphtyle, quinolyle, isoquinolyle, indolyle, furyle, thiényle, (2-indolinon)-yle, carbostyryle ou 3,4-dihydrocarbostyryle,
un reste thiazolyle éventuellement substitué en position 2 par un groupe méthyle ou amino,
un reste benzothiophényle ou benzofuranyle,
un reste benzothiazolyle, benzoxazolyle ou benzimidazolyle éventuellement substitué en position 2 par un groupe méthyle, phényle ou amino, ou bien
A représente une liaison carbone-carbone et
$R_1$ représente un groupe 1H-indén-2-yle ou 1,2-dihydronaphtalén-3-yle ou un groupe 2H-1-benzopyran-3-yle ou 2H-1-benzothiopyran-3-yle éventuellement substitué par un ou deux groupes méthyle et $R_2$ représente un atome d'hydrogène ou un groupe acétyle ou propionyle éventuellement substitué en position oméga par un reste phényle ou 4-méthoxyphényle, et leurs sels d'addition d'acide.

**2.** 4,5,7,8-tétrahydro-6H-thiazolo[5,4-d]azépines de formule générale II selon la revendication 1, dans laquelle
$R_2$ est défini comme dans la revendication 1,
A représente un groupe selon les formules

$$-\underset{*}{C}(R_3)=CH-,\quad -\underset{*}{C}H=C(R_4)-,\quad -\overset{CH_2}{\underset{*}{C}H-CH}-,\quad -C\equiv C-,\quad -\underset{*}{C}H=CH-CH_2-,$$

$$-\underset{*}{C}H(OR_5)-CH_2- \text{ ou } -(CH_2)_n-$$

dans lesquelles
n représente les nombres 2, 3 ou 4,
$R_3$ représente un atome d'hydrogène ou un groupe méthyle,
$R_4$ représente un groupe alkyle de 1 à 3 atomes de carbone ou un groupe phényle et
$R_5$ représente un atome d'hydrogène ou un groupe méthyle ou éthyle et l'atome de carbone caractérisé par * est lié au reste $R_1$, et
$R_1$ représente un reste phényle éventuellement substitué par un atome de fluor, de chlore ou de brome, un groupe alcoxy de 1 à 4 atomes de carbone, un groupe méthyle, trifluorométhyle, phényle, hydroxy, benzyloxy, nitro, amino, diméthylamino, pipéridino, cyano, aminocarbonyle, méthoxycarbonyle, éthoxycarbonyle, méthylmercapto, méthylsulfinyle, méthylsulfonyle ou pyridylméthoxy, un groupe diméthoxyphényle, dihydroxyphényle, 4-hydroxy-3,5-dichlorophényle, 4-hydroxy-3,5-dibromophényle, 4-amino-3,5-dichlorophényle, 4-amino-3,5-dibromophényle, 3,4,5-triméthoxyphényle, naphtyle, 6-chloro-2-pyridyle, thiényle, furyle, quinolyle, isoquinolyle, benzothiophényle, indolyle ou indolin-2-on-4-yle ou un groupe pyridyle éventuellement substitué par un groupe méthyle, ou bien
A représente une liaison carbone-carbone et
$R_1$ représente un groupe 1H-indén-2-yle, 1,2-dihydronaphtalén-3-yle ou 2H-1-benzopyran-3-yle, et leurs sels d'addition d'acide.

**3.** 4,5,7,8-tétrahydro-6H-thiazolo[5,4-d]azépines de formule générale II selon la revendication 2, dans laquelle
$R_1$ et A sont définis comme dans la revendication 2 et $R_2$ représente un atome d'hydrogène, et leurs sels d'addition d'acide.

**4.** 4,5,7,8-tétrahydro-6H-thiazolo[5,4-d]azépines de formule générale II selon la revendication 2, dans laquelle
A représente un groupe vinylène, éthynylène, cyclopropylène ou éthylène,
$R_1$ représente un groupe phényle éventuellement substitué par un atome de chlore, un groupe hydroxy, méthoxy, benzyloxy, isobutoxy, phényle, nitro, amino, cyano ou pipéridino, un groupe pyridyle éventuellement substitué par un groupe méthyle, un groupe diméthoxyphényle, naphtyle, isoquinolyle, 2-méthylthiazolyle, furyle ou thiényle et
$R_2$ représente un atome d'hydrogène, et leurs sels d'addition d'acide.

**5.** Composé selon la revendication 1, qui est la 2-amino-6-(3-(4-cyanophényl)allyl-4,5,7,8-tétrahydro-6H-thiazolo[5,4-d]azépine et ses sels d'addition d'acide.

**6.** Composé selon la revendication 1, qui est la 2-amino-6-(3-(3-benzyloxyphényl)allyl-4,5,7,8-tétrahydro-6H-thiazolo[5,4-d]azépine et ses sels d'addition d'acide.

**7.** Composé selon la revendication 1, qui est la 2-amino-6-(3-(1-naphtyl)allyl-4,5,7,8-tétrahydro-6H-thiazolo[5,4-d]azépine et ses sels d'addition d'acide.

**8.** Sels d'addition d'acide acceptables du point de vue physiologique des composés selon l'une au moins des revendications 1 à 7 avec des acides inorganiques ou organiques.

**9.** Médicament contenant comme principe actif un composé selon l'une au moins des revendications 1 à 7 ou un sel d'addition d'acide acceptable du point de vue physiologique selon la revendication 8 et éventuellement un ou plusieurs véhicules et/ou diluants inertes.

**10.** Médicament selon la revendication 9 pour le traitement des maladies du système nerveux central comme la maladie de Parkinson, l'hyperprolactinémie et la schizophrénie.

**11.** Médicament selon la revendication 9 pour le traitement des maladies cardiovasculaires.

**12.** Utilisation d'un composé selon les revendications 1 à 8 pour la préparation d'un médicament selon les revendications 9 à 11.

**13.** Procédé de préparation d'un médicament selon les revendications 9, 10 ou 11, caractérisé en ce qu'un composé selon l'une au moins des revendications 1 à 7 ou un sel d'addition d'acide acceptable du point de vue physiologique selon la revendication 8 est incorporé dans un ou plusieurs véhicules et/ou diluants inertes.

**14.** Procédé de préparation des 4,5,7,8-tétrahydro-6H-thiazolo[5,4-d]azépines de formule générale II selon l'une au moins des revendications 1 à 8, caractérisé en ce que

a) un composé de formule générale III

$R_1$ - A - $CH_2$ - X (III)

dans laquelle
A et $R_1$ sont définis comme dans l'une au moins des revendications 1 à 7 et
X représente un groupe partant nucléophile,
est mis à réagir avec un composé de formule générale IV

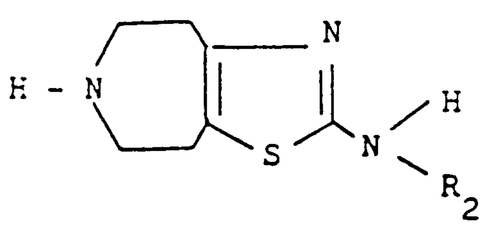

(IV)

dans laquelle
$R_2$ est défini comme dans l'une au moins des revendications 1 à 7, ou bien

b) pour la préparation de composés de formule générale II dans laquelle $R_1$ possède les significations indiquées dans l'une au moins des revendications 1 à 7 pour $R_1$ à l'exception d'un reste 2-pyridyle éventuellement substitué par un atome de chlore, un groupe méthyle, méthoxy, benzyloxy ou hydroxy, d'un reste 2-quinolyle, 1-isoquinolyle, 3-isoquinolyle, 2-thiazolyle, 2-benzoxazolyle ou 2-benzimidazolyle, un aldéhyde de formule générale V

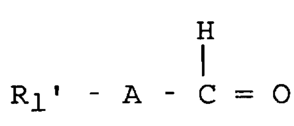

(V)

dans laquelle
A est défini comme dans l'une au moins des revendications 1 à 7 et
$R_1$' possède les significations indiquées dans l'une au moins des revendications 1 à 7 pour $R_1$ à l'exception d'un reste 2-pyridyle éventuellement substitué par un atome de chlore, un groupe méthyle, méthoxy, benzyloxy ou hydroxy, d'un reste 2-quinolyle, 1-isoquinolyle, 3-isoquinolyle, 2-thiazolyle, 2-benzoxazolyle ou 2-benzimidazolyle, est aminé de manière réductrice avec un composé de formule générale IV

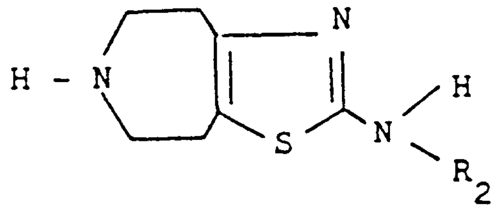

(IV)

dans laquelle
$R_2$ est défini comme dans l'une au moins des revendications 1 à 7, ou bien
c) une 5-halogéno-azépin-4-one de formule générale VI

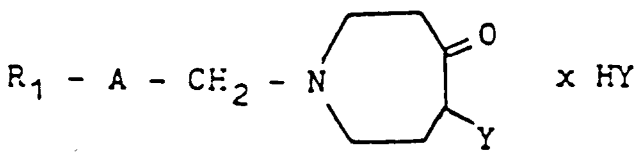

(VI)

dans laquelle
A et $R_1$ sont définis comme dans l'une au moins des revendications 1 à 7 et
Y représente un atome de brome ou de chlore,
est mise à réagir avec une thiourée de formule générale VII

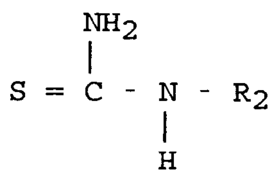

(VII)

dans laquelle
$R_2$ est défini comme dans l'une au moins des revendications 1 à 7, ou bien
d) pour la préparation de composés de formule générale II dans laquelle $R_2$ représente un atome d'hydrogène, une azépin-4-one de formule générale VIII

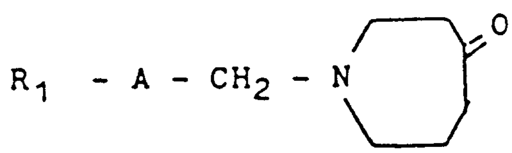

(VIII)

dans laquelle
A et $R_1$ sont définis comme dans l'une au moins des revendications 1 à 7,
est mise à réagir avec un sel de disulfure de formamidine de formule générale IX

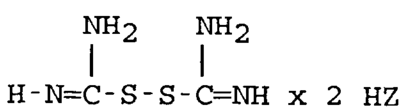

(IX)

dans laquelle
Z représente un reste d'un acide inorganique ou organique, ou bien

e) pour la préparation de composés de formule générale II dans laquelle $R_1$ représente un reste phényle, naphtyle, pyridyle, quinolinyle, isoquinolinyle, furyle, thiényle, benzofuryle, benzothiényle, (2-indolinon)yle, carbostyryle ou 3,4-dihydrocarbostyryle et A représente un groupe éthynylène, un composé de formule générale X

$R_1''$ - Hal (X)

dans laquelle
$R_1''$ représente un reste phényle, naphtyle, pyridyle, quinolinyle, isoquinolinyle, furyle, thiényle, benzofuryle, benzothiényle, (2-indolinon)yle, carbostyryle ou 3,4-dihydrocarbostyryle et
Hal représente un atome de brome ou d'iode,
est mis à réagir avec un composé propargylique de formule générale XI

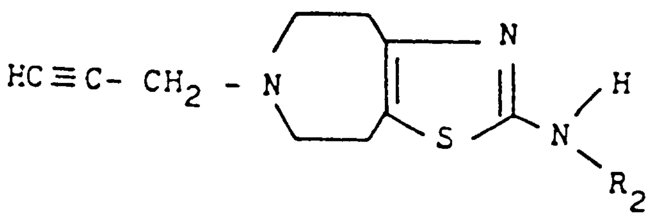

(XI)

dans laquelle
$R_2$ est défini comme dans l'une au moins des revendications 1 à 7, ou bien
f) pour la préparation de composés de formule générale II dans laquelle A représente un groupe vinylène, un composé propargylique de formule générale XII

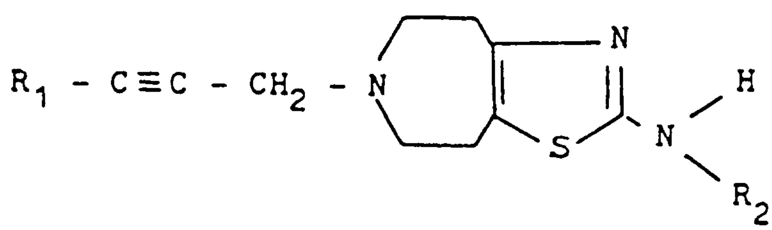

(XII)

dans laquelle
$R_1$ et $R_2$ sont définis comme dans l'une au moins des revendications 1 à 7, est réduit, ou bien
g) pour la préparation de composés de formule générale II dans laquelle A représente un groupe éthylène, un composé de formule générale XIII

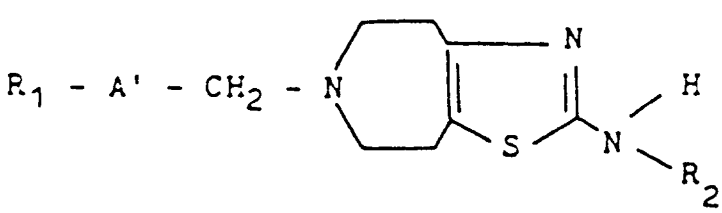

(XIII)

dans laquelle
$R_1$ et $R_2$ sont définis comme dans l'une au moins des revendications 1 à 7 et
A' représente un groupe vinylène ou éthynylène, est réduit, ou bien
h) pour la préparation de composés de formule générale II dans laquelle A représente un groupe cyclopropylène ou un groupe n-alkylène de deux à quatre atomes de carbone et $R_2$ représente un atome d'hydrogène, un amide de formule générale XIV

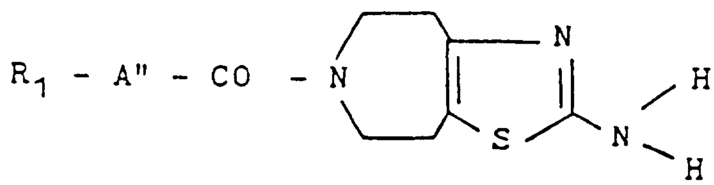

(XIV)

dans laquelle
$R_1$ est défini comme dans l'une au moins des revendications 1 à 7 et
A" représente un groupe cyclopropylène ou un groupe n-alkylène de deux à quatre atomes de carbone, est réduit, ou bien
i) pour la préparation de composés de formule générale II dans laquelle A représente un groupe de formule $-CH(OR_5)-CH_2-$, une cétone de formule générale XV

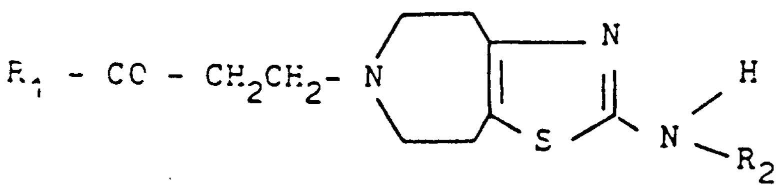

(XV)

dans laquelle
$R_1$ et $R_2$ sont définis comme dans l'une au moins des revendications 1 à 7, est réduite, ou bien
k) pour la préparation de composés de formule générale II dans laquelle A représente un groupe vinylène, un alcool de formule générale XVI

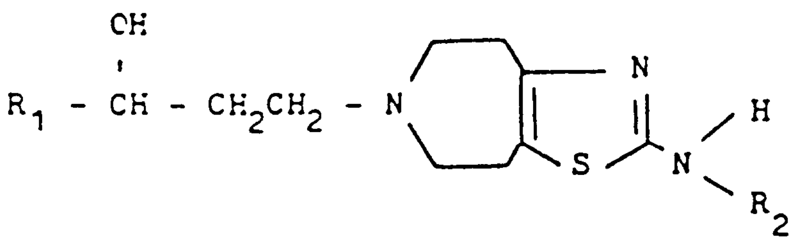

(XVI)

dans laquelle
$R_1$ et $R_2$ sont définis comme dans l'une au moins des revendications 1 à 7, est déshydraté, ou bien
l) pour la préparation de composés de formule générale II dans laquelle $R_2$ représente un groupe acétyle ou propionyle éventuellement substitué en position oméga par un reste phényle ou 4-méthoxyphényle, une amine de formule générale XVII

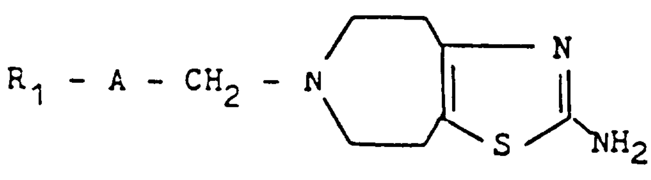

(XVII)

dans laquelle
A et $R_1$ sont définis comme dans l'une au moins des revendications 1 à 7,
est acylée avec un acide carboxylique de formule générale XVIII

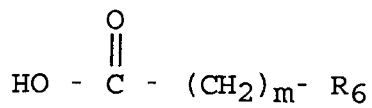 (XVIII)

dans laquelle
m représente le nombre 1 ou 2 et
$R_6$ représente un atome d'hydrogène ou un reste phényle ou 4-méthoxyphényle,
ou avec ses dérivés réactifs éventuellement formés dans le mélange réactionnel, ou bien
m) pour la préparation de composés de formule générale II dans laquelle $R_2$ représente un atome d'hydrogène, un composé de formule générale XIX

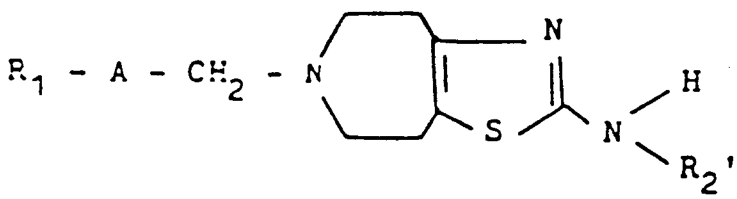 (XIX)

dans laquelle
A et $R_1$ sont définis comme dans l'une au moins des revendications 1 à 7 et
$R_2$' représente un reste clivable par hydrolyse tel qu'un reste acyle ou un reste d'ester d'acide carbonique, est désacylé, ou bien
n) pour la préparation de composés de formule générale II dans laquelle A possède les significations indiquées pour A dans l'une au moins des revendications 1 à 7 à l'exception du groupe -CH($OR_5$)-$CH_2$- et $R_1$ représente un reste phényle, méthylphényle, méthoxyphényle ou pyridyle substitué par un groupe hydroxy ou un reste phényle substitué par deux ou trois groupes hydroxy et $R_2$ représente un atome d'hydrogène, un composé de formule générale XX

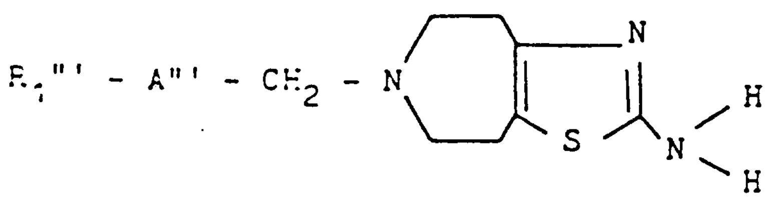 (XX)

dans laquelle
A''' possède les significations indiquées au début pour A à l'exception du groupe -CH($OR_5$)-$CH_2$- et
$R_1$''' représente un reste phényle, méthylphényle, méthoxyphényle ou pyridyle substitué par un groupe benzyloxy ou méthoxy ou un reste phényle substitué par deux ou trois groupes benzyloxy ou méthoxy, est clivé et éventuellement, si on le souhaite,
un composé ainsi obtenu de formule générale II dans laquelle $R_1$ représente un reste nitrophényle est ensuite converti par réduction en un composé correspondant dans lequel $R_1$ représente un reste aminophényle, ou bien
un composé ainsi obtenu de formule générale II dans laquelle $R_1$ représente un reste cyanophényle est converti par hydratation en un composé correspondant dans lequel $R_1$ représente un reste aminocarbonylphényle, ou bien
un composé ainsi obtenu de formule générale II dans laquelle $R_1$ représente un reste cyanophényle est converti par alcoolyse en un composé dans lequel $R_1$ représente un reste méthoxycarbonylphényle ou éthoxycarbonylphényle, ou bien
un composé ainsi obtenu de formule générale II dans laquelle $R_1$ représente un reste cyanophényle est converti par hydrolyse en un composé dans lequel $R_1$ représente un reste carboxyphényle, ou bien

un composé ainsi obtenu de formule générale II dans laquelle $R_1$ représente un reste hydroxyphényle est converti au moyen de l'alcool benzylique ou d'un pyridylméthanol en présence d'un diester d'acide azodicarboxylique en un composé dans lequel $R_1$ représente un reste benzyloxyphényle ou pyridylméthoxyphényle, ou bien
un composé ainsi obtenu de formule générale II est converti en son sel d'addition d'acide, en particulier, pour l'utilisation pharmaceutique, en son sel d'addition d'acide acceptable du point de vue physiologique avec un acide inorganique ou organique.